(19) Europäisches Patentamt
European Patent Office
Office européen des brevets

(11) EP 4 024 484 B1

(12) **EUROPEAN PATENT SPECIFICATION**

(45) Date of publication and mention of the grant of the patent:
**25.10.2023 Bulletin 2023/43**

(21) Application number: **22150131.5**

(22) Date of filing: **04.01.2022**

(51) International Patent Classification (IPC):
***H10K 85/60*** *(2023.01)* *H10K 30/30* *(2023.01)*
*H10K 39/32* *(2023.01)*

(52) Cooperative Patent Classification (CPC):
**H10K 85/657;** H10K 30/30; H10K 39/32

(54) **COMPOSITION FOR PHOTOELECTRIC DEVICE, AND PHOTOELECTRIC DEVICE, IMAGE SENSOR, AND ELECTRONIC DEVICE INCLUDING THE SAME**

ZUSAMMENSETZUNG FÜR FOTOELEKTRISCHE VORRICHTUNG SOWIE FOTOELEKTRISCHE VORRICHTUNG, BILDSENSOR UND ELEKTRONISCHE VORRICHTUNG DAMIT

COMPOSITION POUR DISPOSITIF PHOTOÉLECTRIQUE, ET DISPOSITIF PHOTOÉLECTRIQUE, CAPTEUR D'IMAGES ET DISPOSITIF ÉLECTRONIQUE LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.01.2021 KR 20210000470**

(43) Date of publication of application:
**06.07.2022 Bulletin 2022/27**

(73) Proprietor: **Samsung Electronics Co., Ltd.**
**Gyeonggi-do 16677 (KR)**

(72) Inventors:
- **LIM, Youn Hee**
  **Suwon-si (KR)**
- **PARK, Jeong Il**
  **Seongnam-si (KR)**
- **KIM, Hyeongju**
  **Changwon-si (KR)**
- **PARK, Kyung Bae**
  **Hwaseong-si (KR)**
- **FANG, Feifei**
  **Suwon-si (KR)**
- **YUN, Sung Young**
  **Suwon-si (KR)**
- **LIM, Seon-Jeong**
  **Yongin-si (KR)**
- **CHOI, Taejin**
  **Suwon-si (KR)**

(74) Representative: **Elkington and Fife LLP**
**Prospect House**
**8 Pembroke Road**
**Sevenoaks, Kent TN13 1XR (GB)**

(56) References cited:
**EP-A1- 3 243 822** **EP-A1- 3 252 051**
**WO-A1-2019/230562** **US-A1- 2016 211 465**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

Example embodiments relate to compositions for photoelectric devices and photoelectric devices, image sensors, and electronic devices including the same are disclosed.

### BACKGROUND OF THE INVENTION

A photoelectric device may convert light into an electrical signal using photoelectric effects. A photoelectric device may include a photodiode, a phototransistor, etc., and may be applied to an image sensor, etc.

An image sensor including a photodiode may require high resolution and thus a small pixel. At present, a silicon photodiode is widely used. In some cases, a silicon photodiode exhibits a problem of deteriorated sensitivity because of a relatively small absorption area due to relatively small pixels.

EP 3 252 051 A1 relates to specific heterocyclic compounds, to a photoelectric device having two electrodes and an organic layer comprising said specific heterocyclic compounds, to an image sensor comprising said photoelectric device, and to an electronic device comprising said image sensor or photoelectric device. $

EP 3 243 822 A1 relates to selenophene derivatives useful for photoelectric devices, to an image sensor, and to an electronic device including said selenophene derivatives. WO2019/230562 A1 refers to heterocyclic compounds for organic solar cells. $

US 2016/211465 A1 refers to substituted thieno(2,3-b)indole compounds useful in organic green photoelectric devices and image sensors.

### SUMMARY OF THE INVENTION

Example embodiments provide a composition for a photoelectric device capable of replacing silicon photodiodes and capable of selectively absorbing light in a green wavelength region and having improved thermal stability. Such a composition may be or may be included in an organic material of a photoelectric device that may have a relatively high extinction coefficient and may selectively absorb light in a particular wavelength region depending on a molecular structure, and thus may simultaneously replace a photodiode and a color filter and resultantly improve sensitivity and contribute to relatively high integration.

Some example embodiments also provide a photoelectric device capable of selectively absorbing light in a green wavelength region and maintaining improved efficiency in a process at a high temperature condition.

Some example embodiments also provide an image sensor including the photoelectric device.

Some example embodiments also provide an electronic device including the image sensor.

A composition for a photoelectric device includes a p-type semiconductor compound represented by Chemical Formula 1 and an n-type semiconductor compound.

[Chemical Formula 1]

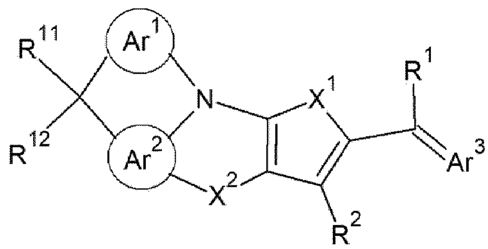

In Chemical Formula 1,

$Ar^1$ and $Ar^2$ may each independently be a substituted or unsubstituted C6 to C30 arene group, a substituted or unsubstituted C3 to C30 heteroarene group, or a condensed ring thereof,
$X^1$ may be -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$,

$R^{dd}$ and $R^{ee}$, or $R^{ff}$ and $R^{gg}$ may be linked to each other to provide a ring structure,

$X^2$ may be -O-, -S-, -Se-, -Te-, -S(=O)-, -S(=O)$_2$-, -$NR^{a1}$-, -$BR^{a2}$-, -$SiR^bR^c$-, - $SiR^{bb}R^{cc}$-, -$GeR^dR^e$-, -$GeR^{dd}R^{ee}$-, -$(CR^fR^g)_{n1}$-, -$(CR^{ff}R^{gg})$-, -$(C(R^m)=C(R^n))$-, - $(C(R^{mm})=C(R^{nn}))$-, or -$(C(R^p)=N)$-, wherein $R^{a1}$, $R^{a2}$, $R^b$, $R^c$, $R^d$, $R^e$, $R^f$, $R^g$, $R^m$, $R^n$, and $R^p$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and $R^{ee}$, $R^{ff}$ and $R^{gg}$, or $R^{mm}$ and $R^{nn}$ may be linked to each other to provide a ring structure, and n1 of -$(CR^fR^g)_{n1}$- may be 1 or 2,

$R^{11}$ and $R^{12}$ may each independently be hydrogen, deuterium, a halogen, a cyano group, a substituted or unsubstituted C1 to C20 alkyl group, a substituted or unsubstituted C6 to C20 aryl group, or a substituted or unsubstituted C6 to C20 aryloxy group, wherein $R^{11}$ and $R^{12}$ may each independently be present or may be linked to each other to provide a ring structure or -$CR^{11}R^{12}$- may be linked with $Ar^1$ or $Ar^2$ to provide a ring structure,

$Ar^3$ may be a substituted or unsubstituted C6 to C30 hydrocarbon cyclic group having at least one functional group selected from C=O, C=S, C=Se, and C=Te, a substituted or unsubstituted C2 to C30 heterocyclic group having at least one functional group selected from C=O, C=S, C=Se, and C=Te, or a fused ring thereof, and

$R^1$ and $R^2$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group (-$SF_5$), a hydroxyl group, an amine group, a hydrazine group, a hydrazone group, a carboxyl group or a salt thereof, a sulfonic acid group or a salt thereof, a phosphoric acid group or a salt thereof, -$SiR^aR^bR^c$, wherein $R^a$, $R^b$, and $R^c$ may each independently be hydrogen or a substituted or unsubstituted C1 to C10 alkyl group, or any combination thereof.

In some example embodiments, the compound of Chemical Formula 1 may be represented by Chemical Formula 2A.

[Chemical Formula 2A]

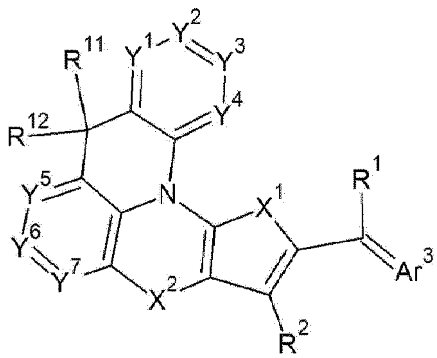

In Chemical Formula 2A,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$Y^1$ to $Y^7$ may each independently be N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof.

In some example embodiments, in Chemical Formula 2A, $Y^4$ may be N or $CR^k$, wherein $R^k$ is a halogen, a cyano group, a C1 to C10 haloalkyl group, or a C1 to C10 cyanoalkyl group, or

in Chemical Formula 2A, $Y^7$ may be N or $CR^k$, wherein $R^k$ may be a halogen, a cyano group, a C1 to C10 haloalkyl group, or a C1 to C10 cyanoalkyl group, and $X^2$ may be -O-, -S-, -Se-, -Te-, -S(=O)-, -S(=O)$_2$-, -$NR^{a1}$-, -$BR^{a2}$-, -$SiR^bR^c$-, -$GeR^dR^e$-, -$(CR^fR^g)_{n1}$-, -$(C(R^m)=C(R^n))$-, or -$(C(R^p)=N)$-, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group and n1 of -$(CR^fR^g)_{n1}$- may be 1 or 2.

In some example embodiments, in Chemical Formula 2A, when $Y^1$ and $Y^5$ are $CR^k$, at least one of $Y^1$ or $Y^5$ and carbon (C) (i.e., C in $-CR^{11}R^{12}-$, the same below) may be linked to each other to provide a fused ring. A structure in which $Y^5$ and carbon (C) are linked to each other may be represented by Chemical Formula 2A-1 and a structure in which $Y^1$ and carbon (C) are linked to each other may be represented by Chemical Formula 2A-2.

[Chemical Formula 2A-1]

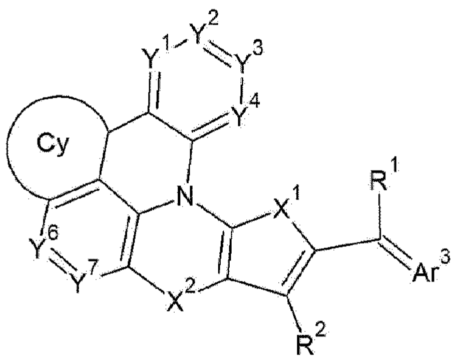

In Chemical Formula 2A-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^1$ to $Y^4$, $Y^6$, and $Y^7$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

[Chemical Formula 2A-2]

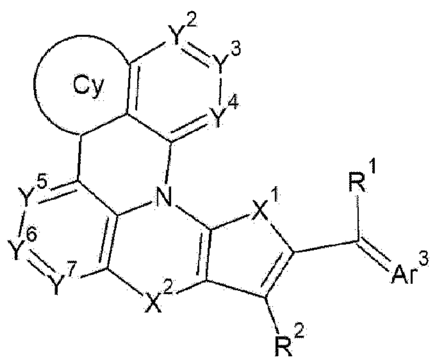

In Chemical Formula 2A-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^2$ to $Y^7$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

In some example embodiments, the compound of Chemical Formula 1 may be represented by Chemical Formula 2B.

[Chemical Formula 2B]

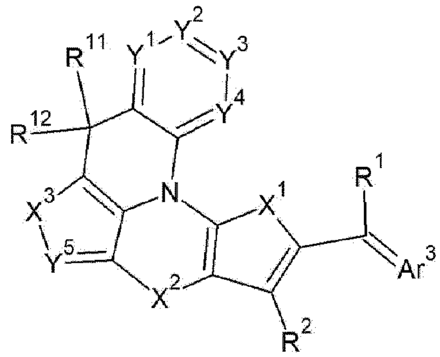

In Chemical Formula 2B,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, $Y^1$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

$X^3$ may be -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ may be linked to each other to provide a ring structure.

In some example embodiments, in Chemical Formula 2B, $Y^4$ may be N or $CR^k$, wherein $R^k$ may be a halogen, a cyano group, a C1 to C10 haloalkyl group, or a C1 to C10 cyanoalkyl group, or
in Chemical Formula 2B, $Y^5$ may be N or $CR^k$, wherein $R^k$ may be a halogen, a cyano group, a C1 to C10 haloalkyl group, or a C1 to C10 cyanoalkyl group and $X^2$ may be -O-, -S-, -Se-, -Te-, -S(=O)-, $-S(=O)_2-$, $-NR^{a1}-$ , $-BR^{a2}-$, $-SiR^bR^c-$, $-GeR^dR^e-$, - $(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group and n1 of $-(CR^fR^g)_{n1}-$ may be 1 or 2.

In some example embodiments, in Chemical Formula 2B, $X^3$ ($-NR^{a1}-$, $-BR^{a2}-$, $-SiR^bR^c-$, $-GeR^dR^e-$, or $-CR^fR^g-$) and carbon (C) may be linked to each other to provide a fused ring. This structure may be represented by Chemical Formula 2B-1.

[Chemical Formula 2B-1]

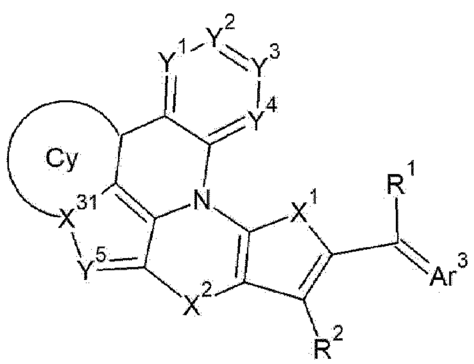

In Chemical Formula 2B-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^{31}$ may be N, B, $SiR^b$, $GeR^d$, $CR^f$, Si, Ge, or C, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,
$Y^1$ to $Y^5$ may each independently be N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

In some example embodiments, in Chemical Formula 2B, when $Y^1$ is $CR^k$, $Y^1$ and carbon (C) may be linked to each other to provide a fused ring. This structure may be represented by Chemical Formula 2B-2.

[Chemical Formula 2B-2]

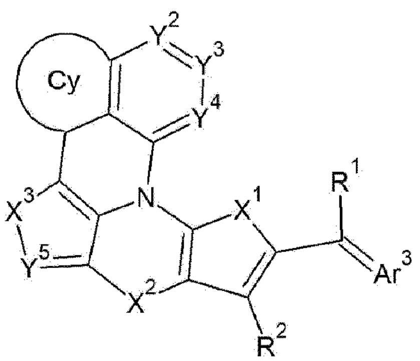

In Chemical Formula 2B-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ and $Y^5$ are the same as $X^3$ and $Y^5$, respectively, in Chemical Formula 2B,
$Y^2$ to $Y^4$ may each independently be N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

In some example embodiments, the compound of Chemical Formula 1 may be represented by Chemical Formula 2C.

[Chemical Formula 2C]

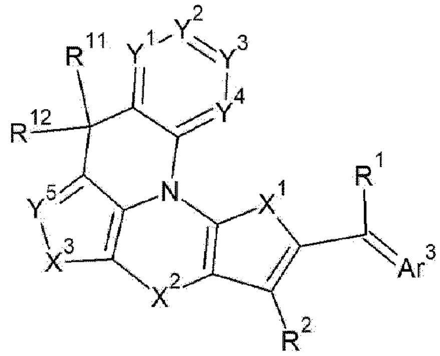

In Chemical Formula 2C,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1,
$Y^1$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
$X^3$ may be -O-, -S-, -Se-, -Te-, -S(=O)-, $-S(=O)_2-$, $-NR^{a1}-$, $-BR^{a2}-$, $-SiR^bR^c-$, $- SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ may be linked to each other to provide a ring structure.

In some example embodiments, in Chemical Formula 2C, $Y^4$ may be N or $CR^k$, wherein $R^k$ may be a halogen, a cyano group, a C1 to C10 haloalkyl group, or a C1 to C10 cyanoalkyl group, or
in Chemical Formula 2C, $X^3$ may be -O-, -S-, -Se-, -Te-, -S(=O)-, $-S(=O)_2-$, $- NR^{a1}-$, $-BR^{a2}-$, $-SiR^bR^c-$, $-GeR^dR^e-$, or $-CR^fR^g-$, wherein $R^{a1}$, $R^{a2}$, $R^b$, $R^c$, $R^d$, $R^e$, $R^f$, and $R^g$ may each independently be a halogen, a C1 to C20 haloalkyl group, or a C1 to C20 cyanoalkyl group, and $X^2$ may be -O-, -S-, -Se-, -Te-, -S(=O)-, $-S(=O)_2-$, $- NR^{a1}-$, $-BR^{a2}-$, $-SiR^bR^c-$, $-GeR^dR^e-$, $-(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group, and n1 of $- (CR^fR^g)_{n1}-$ may be 1 or 2.

In Chemical Formula 2C, when $Y^5$ is $CR^k$, $Y^5$ and carbon (C) may be linked to each other to provide a fused ring. This structure may be represented by Chemical Formula 2C-1.

[Chemical Formula 2C-1]

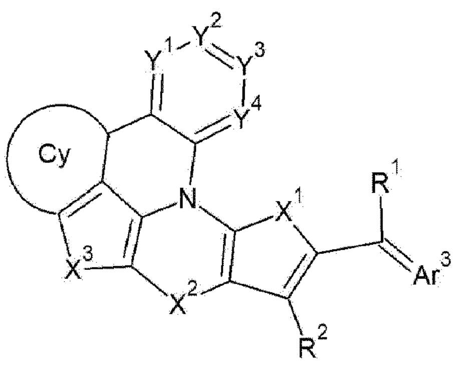

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2C,

$Y^1$ to $Y^4$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

In some example embodiments, in Chemical Formula 2C, when $Y^1$ is $CR^k$, $Y^1$ and carbon (C) may be linked to each other to provide a fused ring. This structure may be represented by Chemical Formula 2C-2.

[Chemical Formula 2C-2]

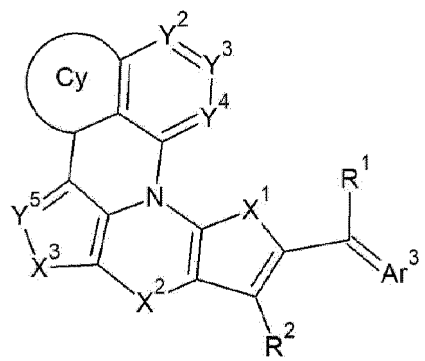

In Chemical Formula 2C-2,

$X^1$, $X^2$, $Ar^3$, $R^1$, and $R^2$ are the same as $X^1$, $X^2$, $Ar^3$, $R^1$, and $R^2$, respectively, in Chemical Formula 1,

$X^3$ and $Y^5$ are the same as $X^3$ and $Y^5$, respectively, in Chemical Formula 2C,

$Y^2$ to $Y^4$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

In some example embodiments, the compound of Chemical Formula 1 may be represented by Chemical Formula 2D.

[Chemical Formula 2D]

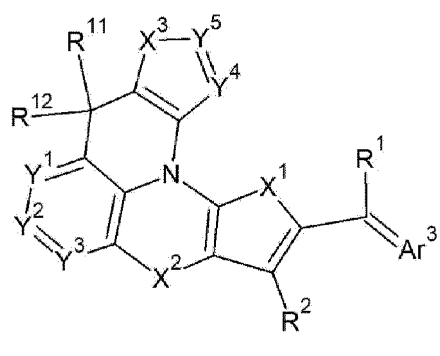

In Chemical Formula 2D,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, $Y^1$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

$X^3$ may be -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ may be linked to each other to provide a ring structure.

In some example embodiments, in Chemical Formula 2D, $Y^4$ may be N or $CR^k$, wherein $R^k$ is a halogen, a cyano group, a C1 to C10 haloalkyl group, or a C1 to C10 cyanoalkyl group, or

In Chemical Formula 2D, $Y^3$ may be N or $CR^k$, wherein $R^k$ may be a halogen, a cyano group, a C1 to C10 haloalkyl group, or a C1 to C10 cyanoalkyl group, and $X^2$ may be -O-, -S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group and n1 of $-(CR^fR^g)_{n1}-$ may be 1 or 2.

In Chemical Formula 2D, when $Y^1$ is $CR^k$, $Y^1$ and carbon (C) may be linked to each other to provide a fused ring. This structure may be represented by Chemical Formula 2D-1.

[Chemical Formula 2D-1]

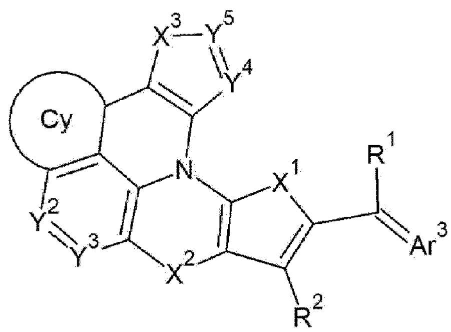

In Chemical Formula 2D-1,

$X^1$, $X^2$, $Ar^3$, $R^1$, and $R^2$ are the same as $X^1$, $X^2$, $Ar^3$, $R^1$, and $R^2$, respectively, in Chemical Formula 1,

$X^3$ is the same as $X^3$ in Chemical Formula 2D,

$Y^2$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

In Chemical Formula 2D, when $X^3$ is $-NR^{a1}-$, $-BR^{a2}-$, $-SiR^bR^c-$, $-GeR^dR^e-$, or - $CR^fR^g-$, $X^3$ and carbon (C) may be linked to each other to provide a fused ring. This structure may be represented by Chemical Formula 2D-2.

[Chemical Formula 2D-2]

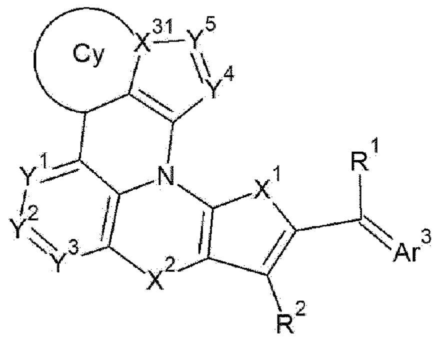

In Chemical Formula 2D-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^{31}$ may be N, B, $SiR^b$, $GeR^d$, $CR^f$, Si, Ge, or C, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,
$Y^1$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

In some example embodiments, the compound of Chemical Formula 1 may be represented by Chemical Formula 2E.

[Chemical Formula 2E]

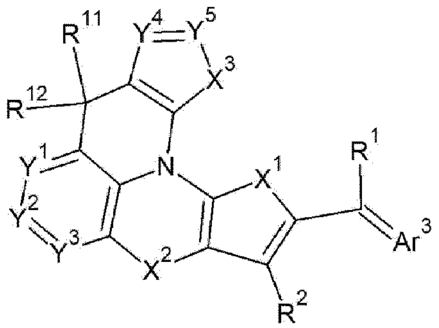

In Chemical Formula 2E,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and
$X^3$ may be -O-, -S-, -Se-, -Te-, -S(=O)-, -S(=O)$_2$-, -$NR^{a1}$-, -$BR^{a2}$-, -$SiR^bR^c$-, - $SiR^{bb}R^{cc}$-, -$GeR^dR^e$-, -$GeR^{dd}R^{ee}$-, -$CR^fR^g$-, or -$CR^{ff}R^{gg}$-, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ may be linked to each other to provide a ring structure, and
$Y^1$ to $Y^5$ may each independently be N or $CR^k$, wherein $R^k$ may be hydrogen, deuterium, a halogen, a cyano group,

a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof.

In some example embodiments, in Chemical Formula 2E, $X^3$ may be -O-, -S-, -Se-, -Te-, -S(=O)-, $-S(=O)_2-$, $-NR^{a1}-$, $-BR^{a2}-$, $-SiR^bR^c-$, $-GeR^dR^e-$, or $-CR^fR^g-$, wherein $R^{a1}$, $R^{a2}$, $R^b$, $R^c$, $R^d$, $R^e$, $R^f$, and $R^g$ may each independently be a halogen, a C1 to C20 haloalkyl group, or a C1 to C20 cyanoalkyl group, or
in Chemical Formula 2E, $Y^3$ may be N or $CR^k$, wherein $R^k$ may be a halogen, a cyano group, a C1 to C10 haloalkyl group, or a C1 to C10 cyanoalkyl group and $X^2$ may be -O-, -S-, -Se-, -Te-, -S(=O)-, $-S(=O)_2-$, $-NR^{a1}-$ , $-BR^{a2}-$, $-SiR^bR^c-$, $-GeR^dR^e-$, $-(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group and n1 of $-(CR^fR^g)_{n1}-$ may be 1 or 2.

In some example embodiments, in Chemical Formula 2E, when $Y^1$ and $Y^4$ are $CR^k$, one of $Y^1$ or $Y^4$ and carbon (C) may be linked to each other to provide a fused ring. This structure may be represented by Chemical Formula 2E-1 or Chemical Formula 2E-2.

[Chemical Formula 2E-1]

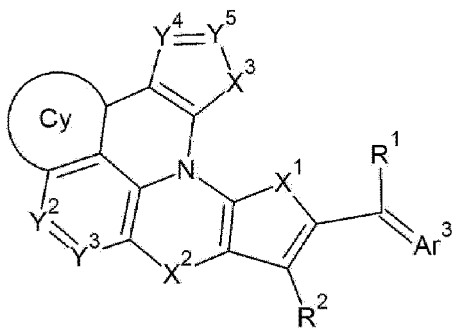

In Chemical Formula 2E-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2E,
$Y^2$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

[Chemical Formula 2E-2]

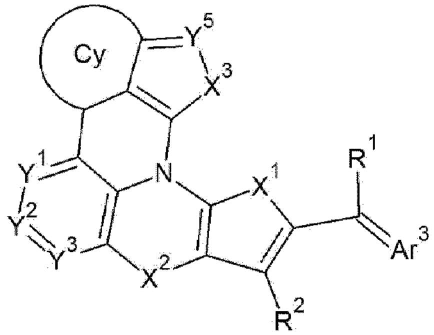

In Chemical Formula 2E-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2E,
$Y^1$ to $Y^3$ and $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

In some example embodiments, in $X^1$, $X^2$, and $-CR^{11}R^{12}-$ of Chemical Formula 1, each ring structure of the ring structure of $X^1$, the ring structure of $X^2$, and the ring structure of $-CR^{11}R^{12}-$ is a spiro structure or a fused ring structure.

In some example embodiments, the spiro structure may include a moiety represented by Chemical Formula 3.

[Chemical Formula 3]

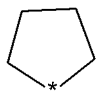
(1)
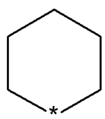
(2)
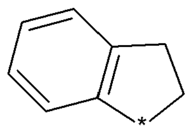
(3)
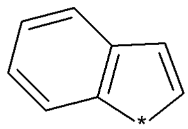
(4)
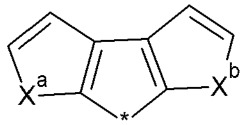
(5)
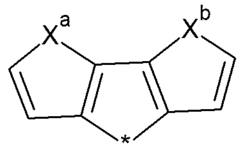
(6)
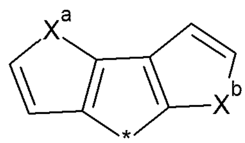
(7)
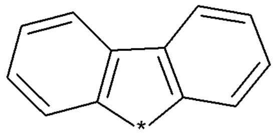
(8)
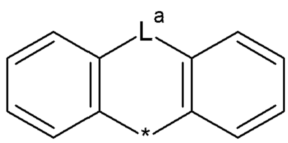
(9)
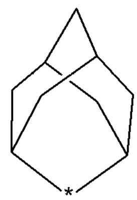
(10)

In Chemical Formula 3,

$X^a$ and $X^b$ may each independently be -O-, -S-, -Se-, -Te-, -S(=O)-, $-S(=O)_2-$, $-NR^{a1}-$ , $-BR^{a2}-$, $-SiR^bR^c-$, $-SiR^{bb}R^{cc}-$, $-GeR^dR^e-$, or $-GeR^{dd}R^{ee}-$, wherein $R^{a1}$, $R^{a2}$, $R^b$, $R^c$, $R^d$, and $R^e$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$ or $R^{dd}$ and $R^{ee}$ may be linked to each other to provide a ring structure,
$L^a$ may be -O-, -S-, -Se-, -Te-, $-NR^{a1}-$, $-BR^{a2}-$, $-SiR^bR^c-$, $-GeR^dR^e-$, $-(CR^fR^g)_{n1}-$, $-(C(R^p)=N)-$, or a single bond, wherein $R^{a1}$, $R^{a2}$, $R^b$, $R^c$, $R^d$, $R^e$, $R^f$, $R^g$, and $R^p$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and n1 of $-(CR^fR^g)_{n1}-$ may be 1 or 2, and
at least one hydrogen (e.g., some or all hydrogens) of each ring of one or more moieties of Chemical Formula 3

may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 3, one or more CH present in the aromatic ring of the moieties (3), (4), (5), (6), (7), (8), or (9) of Chemical Formula 3 may be replaced by N.

In some example embodiments, in Chemical Formula 1, $Ar^3$ may be a cyclic group represented by Chemical Formula 4.

[Chemical Formula 4]

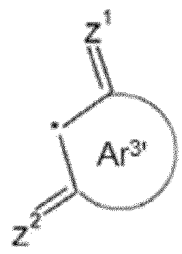

In Chemical Formula 4,

$Ar^{3'}$ may be a substituted or unsubstituted C6 to C30 aryl group or a substituted or unsubstituted C3 to C30 heteroaryl group,
$Z^1$ may be O, S, Se, or Te, and
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group.

In some example embodiments, in Chemical Formula 1, $Ar^3$ may be a cyclic group represented by one of Chemical Formula 5A to Chemical Formula 5G:

[Chemical Formula 5A]

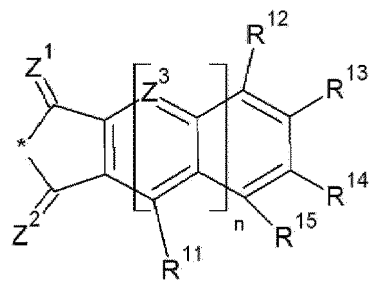

In Chemical Formula 5A,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ may be N or $CR^c$, wherein $R^c$ is hydrogen, deuterium, or a substituted or unsubstituted C1 to C10 alkyl group,
$R^{11}$, $R^{12}$, $R^{13}$, $R^{14}$, and $R^{15}$ may each independently be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ may each independently be present or may be linked to each other to provide a fused aromatic ring,

n may be 0 or 1, and
* may be a linking point.

[Chemical Formula 5B]

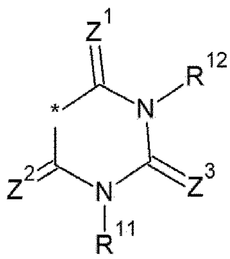

In Chemical Formula 5B,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ may be O, S, Se, Te, or $C(R^a)(CN)$, wherein $R^a$ is hydrogen, a cyano group (-CN), or a C1 to C10 alkyl group,
$R^{11}$ and $R^{12}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* may be a linking point.

[Chemical Formula 5C]

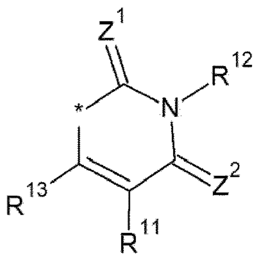

In Chemical Formula 5C,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* may be a linking point.

[Chemical Formula 5D]

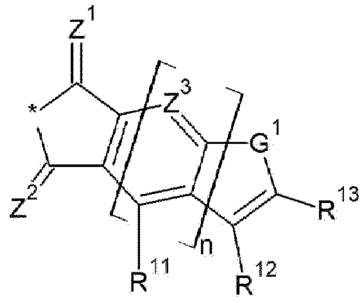

In Chemical Formula 5D,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ may be N or $CR^c$, wherein $R^c$ may be hydrogen or a substituted or unsubstituted C1 to C10 alkyl group,
$G^1$ may be O, S, Se, Te, $SiR^xR^y$, or $GeR^zR^w$, wherein $R^x$, $R^y$, $R^z$, and $R^w$ may each independently be hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, wherein $R^{12}$ and $R^{13}$ may each independently be present or may be linked to each other to provide a fused aromatic ring,
n may be 0 or 1, and
* may be a linking point.

[Chemical Formula 5E]

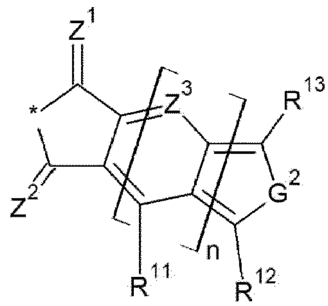

In Chemical Formula 5E,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ may be N or $CR^c$, wherein $R^c$ may be hydrogen or a substituted or unsubstituted C1 to C10 alkyl group,
$G^2$ may be O, S, Se, Te, $SiR^xR^y$, or $GeR^zR^w$, wherein $R^x$, $R^y$, $R^z$, and $R^w$ may each independently be hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or

any combination thereof,
n may be 0 or 1, and
* may be a linking point.

[Chemical Formula 5F]

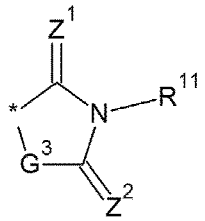

In Chemical Formula 5F,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$R^{11}$ may be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, and
$G^3$ may be O, S, Se, Te, $SiR^xR^y$, or $GeR^zR^w$, wherein $R^x$, $R^y$, $R^z$, and $R^w$ may each independently be hydrogen, deuterium, a halogen, a substituted or unsubstituted C1 to C20 alkyl group, or a substituted or unsubstituted C6 to C20 aryl group.

[Chemical Formula 5G]

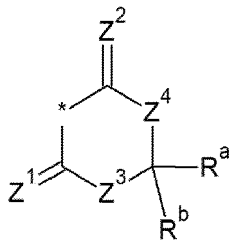

In Chemical Formula 5G,

$Z^1$ may be O, S, Se, or Te,
$R^a$ and $R^b$ may each independently be hydrogen, a substituted or unsubstituted C1 to C10 alkyl group, a cyano group, or a cyano-containing group, and
$Z^2$ to $Z^4$ may each independently be O, S, Se, Te, or $CR^cR^d$, wherein $R^c$ and $R^d$ are each independently hydrogen, a substituted or unsubstituted C1 to C10 alkyl group, a cyano group, or a cyano-containing group, provided that when $Z^2$ is $CR^cR^d$, at least one of $R^c$ or $R^d$ is a cyano group or a cyano-containing group.

In some example embodiments, the composition for a photoelectric device may have a maximum absorption wavelength ($\lambda_{max}$) in a wavelength range of greater than or equal to about 500 nm and less than or equal to about 600 nm in a thin film state.

In some example embodiments, the composition for a photoelectric device may be configured to exhibit a light absorption curve having a full width at half maximum (FWHM) in a thin film state of about 50 nm to about 110 nm.

According to some example embodiments, a photoelectric device (e.g., organic photoelectric device) includes a first electrode and a second electrode facing each other, and an active layer between the first electrode and the second electrode and including the composition for a photoelectric device including the p type semiconductor compound represented by Chemical Formula 1 and n-type semiconductor compound.

According to some example embodiments, an image sensor including the photoelectric device is provided.

In some example embodiments, the image sensor may include a semiconductor substrate integrated with a plurality of first photo-sensing devices configured to sense light in a blue wavelength region and a plurality of second photo-sensing devices configured to sense light in a red wavelength region, and the photoelectric device may be on the semiconductor substrate and configured to selectively sense light in a green wavelength region.

In some example embodiments, the plurality of first photo-sensing devices and the plurality of second photo-sensing devices may be stacked in a vertical direction in a semiconductor substrate.

In some example embodiments, the image sensor may further include a color filter layer including a blue filter configured to selectively transmit light in a blue wavelength region and a red filter configured to selectively transmit light in a red wavelength region.

In some example embodiments, the photoelectric device is a green photoelectric device configured to selectively absorb light in a green wavelength region, and the image sensor may include the green photoelectric device, a blue photoelectric device configured to selectively sense light in a blue wavelength region, and a red photoelectric device configured to selectively sense light in a red wavelength region, which may be stacked.

According to some example embodiments, an electronic device including the image sensor is provided.

The composition for a photoelectric device is capable of selectively absorbing light in a green wavelength region and excellent in thermal stability and charge mobility. The wavelength selectivity in the green wavelength region by the composition for a photoelectric device may be increased to improve efficiency of the device, and a photoelectric device, an image sensor, and an electronic device in which performance is not degraded even in a high-temperature process are provided.

## BRIEF DESCRIPTION OF THE DRAWINGS

FIG. 1 is a cross-sectional view showing a photoelectric device according to some example embodiments,
FIG. 2 is a cross-sectional view showing a photoelectric device according to some example embodiments,
FIG. 3 is a schematic plan view showing an organic CMOS image sensor according to some example embodiments,
FIG. 4 is a cross-sectional view of the organic CMOS image sensor of FIG. 3,
FIG. 5 is a schematic cross-sectional view of an organic CMOS image sensor according to some example embodiments,
FIG. 6 is a schematic cross-sectional view of an organic CMOS image sensor according to some example embodiments,
FIG. 7 is a cross-sectional view of an organic CMOS image sensor according to some example embodiments,
FIG. 8 is a schematic view showing an organic CMOS image sensor according to some example embodiments,
FIG. 9 is a block diagram of a digital camera including an image sensor according to some example embodiments, and
FIG. 10 is a schematic diagram showing an electronic device according to some example embodiments.

## DETAILED DESCRIPTION OF THE EMBODIMENTS

Hereinafter, some example embodiments are described in detail so that those of ordinary skill in the art can easily implement them. However, a structure that is actually applied may be implemented in various different forms, and is not limited to the example embodiments described herein.

In the drawings, the thickness of layers, films, panels, regions, etc., are exaggerated for clarity. Like reference numerals designate like elements throughout the specification. It will be understood that when an element such as a layer, film, region, or substrate is referred to as being "on" another element, it can be directly on the other element or intervening elements may also be present. In contrast, when an element is referred to as being "directly on" another element, there are no intervening elements present.

In the drawings, parts having no relationship with the description are omitted for clarity of some example embodiments, and the same or similar constituent elements are indicated by the same reference numeral throughout the specification.

As used herein, "at least one of A, B, or C," "one of A, B, C, or any combination thereof" and "one of A, B, C, and any combination thereof' refer to each constituent element, and any combination thereof (e.g., A; B; C; A and B; A and C; B and C; or A, B and C).

As used herein, when specific definition is not otherwise provided, "substituted" refers to replacement of a hydrogen of a compound or a functional group by a halogen atom (F, Br, Cl, or I), a hydroxy group, a nitro group, a cyano group, an azido group, an amidino group, an amine group (-NR'R", wherein R' and R" are each independently a hydrogen atom, a C1 to C20 alkyl group or a C6 to C30 aryl group), a hydrazino group, a hydrazono group, a carbonyl

group, a carbamyl group, a thiol group, an ester group, a carboxyl group or a salt thereof, sulfonic acid group or a salt thereof, a phosphoric acid group or a salt thereof, a C1 to C20 alkyl group, a C1 to C20 alkoxy group, a C2 to C20 alkenyl group, a C2 to C20 alkynyl group, a C6 to C30 aryl group, a C7 to C30 arylalkyl group, a C2 to C20 heteroaryl group, a C3 to C20 heteroarylalkyl group, a C3 to C30 cycloalkyl group, a C3 to C15 cycloalkenyl group, a C6 to C15 cycloalkynyl group, a C2 to C20 heterocycloalkyl group, or any combination thereof.

The "arene group" refers to a hydrocarbon cyclic group having an aromatic ring, and includes monocyclic and multicyclic hydrocarbon cyclic groups, and additional rings of the multicyclic hydrocarbon cyclic group may be an aromatic ring or a non-aromatic ring. The arene group may be a C6 to C30 arene group, a C6 to C20 arene group, or a C6 to C10 arene group.

"Heteroarene group" refers to an arene group including 1 to 3 heteroatoms selected from N, O, S, P, and Si in the ring. The heteroarene group may be a C3 to C30 heteroarene group, a C3 to C20 heteroarene group, or a C3 to C10 heteroarene group.

As used herein, "hydrocarbon cyclic group" may be a C3 to C30 hydrocarbon cyclic group. The hydrocarbon cyclic group may be an aromatic hydrocarbon cyclic group (e.g., C6 to C30 arene group, C6 to C20 arene group, or C6 to C10 arene group or C6 to C30 aryl group, C6 to C20 aryl group, or C6 to C10 aryl group), an alicyclic hydrocarbon cyclic group (e.g., C3 to C30 cycloalkyl group, C5 to C30 cycloalkyl group, C3 to C20 cycloalkyl group, or C3 to C10 cycloalkyl group), or a fused ring group thereof. For example, the fused ring group may refer to a fused ring of an aromatic ring (arene ring) and a non-aromatic ring (alicyclic ring), for example a fused ring in which at least one aromatic ring (arene ring) such as a C6 to C30 arene group, a C6 to C20 arene group, or a C6 to C10 arene group or a C6 to C30 aryl group, a C6 to C20 aryl group, or a C6 to C10 aryl group and at least one non-aromatic ring (alicyclic ring) such as a C3 to C30 cycloalkyl group, a C3 to C20 cycloalkyl group, or a C3 to C10 cycloalkyl group are fused to each other.

As used herein, the "heterocyclic group" may be a C2 to C30 heterocyclic group. The heterocyclic group may be a cyclic group in which at least one, for example 1 to 3 carbons of an aromatic hydrocarbon cyclic group (e.g., a C6 to C30 arene group, a C6 to C20 arene group, or a C6 to C10 arene group or a C6 to C30 aryl group, a C6 to C20 aryl group, or a C6 to C10 aryl group), an alicyclic hydrocarbon cyclic group (e.g., a C3 to C30 cycloalkyl group, a C3 to C20 cycloalkyl group, or a C3 to C10 cycloalkyl group), and a fused ring group thereof are replaced by a heteroatom selected from N, O, S, P, and Si. In addition, at least one carbon atom of the heterocyclic group may be replaced by a thiocarbonyl group (C=S).

As used herein, when specific definition is not otherwise provided, "'hetero" refers to one including 1 to 3 heteroatoms selected from N, O, S, P, and Si.

As used herein, "alkyl group" refers to a monovalent linear or branched saturated hydrocarbon group, for example a methyl group, an ethyl group, a propyl group, an isopropyl group, an n-butyl group, an isobutyl group, a t-butyl group, a pentyl group, a hexyl group, and the like.

As used herein, "cycloalkyl group" refers to a monovalent saturated hydrocarbon cyclic group in which the atoms of the cycle are carbon, for example a cyclopropyl group, a cyclobutyl group, a cyclopentyl group, or a cyclohexyl group.

As used herein, "aryl group" refers to a cyclic functional group wherein all elements of the cycle have p-orbitals which form conjugation, and may be a monocyclic, polycyclic or fused ring polycyclic (e.g., rings sharing adjacent pairs of carbon atoms) functional group.

As used herein, when a definition is not otherwise provided, "cyano-containing group" refers to a monovalent group such as a C1 to C30 alkyl group, a C2 to C30 alkenyl group, or a C2 to C30 alkynyl group where at least one hydrogen is substituted with a cyano group. The cyano-containing group also refers to a divalent group such as $=CR^{x'}-(CR^xR^y)_p-CR^{y'}(CN)_2$ wherein $R^x$, $R^y$, $R^{x'}$, and $R^{y'}$ may each independently be hydrogen or a C1 to C10 alkyl group and p is an integer of 0 to 10 (or 1 to 10). Specific examples of the cyano-containing group may be a dicyanomethyl group, a dicyanovinyl group, a cyanoethynyl group, and the like. As used herein, the cyano-containing group does not include a functional group including a cyano group (-CN) alone.

As used herein, when a definition is not otherwise provided, "aromatic hydrocarbon group" may include a C6 to C30 arene group such as a phenyl group and a naphthyl group, a C6 to C30 aryl group, and a C6 to C30 arylene group, but is not limited thereto.

As used herein, when a definition is not otherwise provided, "aliphatic hydrocarbon group" may include a C1 to C15 alkyl group such as a methyl group, an ethyl group, a propyl group, and the like, a C1 to C15 alkylene group, a C2 to C15 alkenyl group such as an ethenyl group or a propenyl group, a C2 to C15 alkynyl group such as an ethynyl group or a propynyl group, but is not limited thereto.

As used herein, when a definition is not otherwise provided, "aromatic ring" refers to a C6 to C10 hydrocarbon cyclic group (e.g., a C6 to C10 aryl group) providing a conjugated structure or a C2 to C10 heterocyclic group (e.g., a C2 to C10 heteroaryl group) providing a conjugated structure.

As used herein, when a definition is not otherwise provided, "spiro structure" may be a substituted or unsubstituted C5 to C30 hydrocarbon cyclic group, a substituted or unsubstituted C2 to C30 heterocyclic group, or a fused

ring thereof. The substituted or unsubstituted C5 to C30 hydrocarbon cyclic group may be for example a substituted or unsubstituted C5 to C30 cycloalkyl group (e.g., a substituted or unsubstituted C5 to C20 cycloalkyl group or a substituted or unsubstituted C5 to C10 cycloalkyl group) or a substituted or unsubstituted C6 to C30 aryl group (e.g., a substituted or unsubstituted C6 to C20 aryl group, or a substituted or unsubstituted C6 to C10 aryl group) and the substituted or unsubstituted C2 to C30 heterocyclic group may be for example a substituted or unsubstituted C2 to C20 heterocycloalkyl group (e.g., a substituted or unsubstituted C2 to C10 heterocycloalkyl group) or a substituted or unsubstituted C2 to C20 heteroaryl group (e.g., a substituted or unsubstituted C2 to C10 heteroaryl group).

As used herein, when a definition is not otherwise provided, "fused ring" is a fused ring of two or more substituted or unsubstituted C5 to C30 hydrocarbon cyclic groups, a fused ring of two or more substituted or unsubstituted C2 to C30 heterocyclic groups, or a fused ring of a substituted or unsubstituted C5 to C30 hydrocarbon cyclic group and a substituted or unsubstituted C2 to C30 heterocyclic group (e.g., a fluorenyl group). Herein, the hydrocarbon cyclic group and the hetero cyclic group are as defined above.

As used herein, when a definition is not otherwise provided, "combination" refers to a mixture of two or more, substitution in which one substituent is substituted with another substituent, fusion with each other, or a linkage to each other by a single bond or a C1 to C10 alkylene group.

It will further be understood that when an element is referred to as being "on" another element, it may be above or beneath or adjacent (e.g., horizontally adjacent) to the other element.

It will be understood that elements and/or properties thereof (e.g., structures, surfaces, directions, or the like), which may be referred to as being "perpendicular," "parallel," "coplanar," or the like with regard to other elements and/or properties thereof (e.g., structures, surfaces, directions, or the like) may be "perpendicular," "parallel," "coplanar," or the like or may be "substantially perpendicular," "substantially parallel," "substantially coplanar," respectively, with regard to the other elements and/or properties thereof.

Elements and/or properties thereof (e.g., structures, surfaces, directions, or the like) that are "substantially perpendicular" with regard to other elements and/or properties thereof will be understood to be "perpendicular" with regard to the other elements and/or properties thereof within manufacturing tolerances and/or material tolerances and/or have a deviation in magnitude and/or angle from "perpendicular," or the like with regard to the other elements and/or properties thereof that is equal to or less than 10% (e.g., a. tolerance of ±10%).

Elements and/or properties thereof (e.g., structures, surfaces, directions, or the like) that are "substantially parallel" with regard to other elements and/or properties thereof will be understood to be "parallel" with regard to the other elements and/or properties thereof within manufacturing tolerances and/or material tolerances and/or have a deviation in magnitude and/or angle from "parallel," or the like with regard to the other elements and/or properties thereof that is equal to or less than 10% (e.g., a. tolerance of ±10%).

Elements and/or properties thereof (e.g., structures, surfaces, directions, or the like) that are "substantially coplanar" with regard to other elements and/or properties thereof will be understood to be "coplanar" with regard to the other elements and/or properties thereof within manufacturing tolerances and/or material tolerances and/or have a deviation in magnitude and/or angle from "coplanar," or the like with regard to the other elements and/or properties thereof that is equal to or less than 10% (e.g., a. tolerance of ±10%).

It will be understood that elements and/or properties thereof may be recited herein as being "the same" or "equal" as other elements, and it will be further understood that elements and/or properties thereof recited herein as being "identical" to, "the same" as, or "equal" to other elements may be "identical" to, "the same" as, or "equal" to or "substantially identical" to, "substantially the same" as or "substantially equal" to the other elements and/or properties thereof. Elements and/or properties thereof that are "substantially identical" to, "substantially the same" as or "substantially equal" to other elements and/or properties thereof will be understood to include elements and/or properties thereof that are identical to, the same as, or equal to the other elements and/or properties thereof within manufacturing tolerances and/or material tolerances. Elements and/or properties thereof that are identical or substantially identical to and/or the same or substantially the same as other elements and/or properties thereof may be structurally the same or substantially the same, functionally the same or substantially the same, and/or compositionally the same or substantially the same.

It will be understood that elements and/or properties thereof described herein as being the "substantially" the same and/or identical encompasses elements and/or properties thereof that have a relative difference in magnitude that is equal to or less than 10%. Further, regardless of whether elements and/or properties thereof are modified as "substantially," it will be understood that these elements and/or properties thereof should be construed as including a manufacturing or operational tolerance (e.g., ±10%) around the stated elements and/or properties thereof.

When the term "about" is used in this specification in connection with a numerical value, it is intended that the associated numerical value includes a manufacturing or operational tolerance (e.g., ±10%) around the stated numerical value.

Hereinafter, a composition for a photoelectric device according to some example embodiments is described. The composition for a photoelectric device includes a p-type semiconductor compound represented by Chemical Formula 1 and an n-type semiconductor compound.

[Chemical Formula 1]

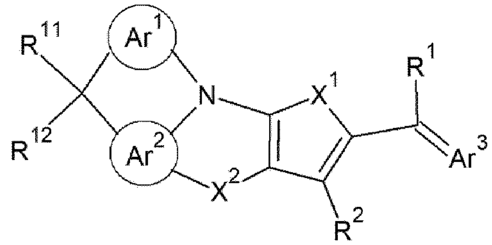

In Chemical Formula 1,

$Ar^1$ and $Ar^2$ may each independently be a substituted or unsubstituted C6 to C30 arene group, a substituted or unsubstituted C3 to C30 heteroarene group, or a condensed ring thereof,
$X^1$ may be -S-, -Se-, -Te-, -S(=O)-, -S(=O)$_2$-, -NR$^{a1}$-, -BR$^{a2}$-, -SiR$^b$R$^c$-, - SiR$^{bb}$R$^{cc}$-, -GeR$^d$R$^e$-, -GeR$^{dd}$R$^{ee}$-, -CR$^f$R$^g$-, or -CR$^{ff}$R$^{gg}$-, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and $R^{bb}$, $R^{cc}$, $R^{dd}$, $R^{ee}$, $R^{ff}$, and $R^{gg}$ may each independently be $(CH_2)_w$, wherein w is a positive integer (e.g., any integer having a value of 1 or greater, 1 to 10, 1 to 8, or 1 to 6), or a heteroatom of O, NR$^w$, wherein $R^w$ is hydrogen or a C1 to C10 alkyl group, S, Se, or Te, and/or at least one pair of $R^{bb}$ and $R^{cc}$, $R^{dd}$ and $R^{ee}$, or $R^{ff}$ and $R^{gg}$ is linked to each other to provide a ring structure,
$X^2$ may be -O-, -S-, -Se-, -Te-, -S(=O)-, -S(=O)$_2$-, -NR$^{a1}$-, -BR$^{a2}$-, -SiR$^b$R$^c$-, - SiR$^{bb}$R$^{cc}$-, -GeR$^d$R$^e$-, -GeR$^{dd}$R$^{ee}$-, -(CR$^f$R$^g$)$_{n1}$-, -(CR$^{ff}$R$^{gg}$)-, -(C(R$^m$)=C(R$^n$))-, - (C(R$^{mm}$)=C(R$^{nn}$))-, or -(C(R$^p$)=N)-, wherein $R^{a1}$, $R^{a2}$, $R^b$, $R^c$, $R^d$, $R^e$, $R^f$, $R^g$, $R^m$, $R^n$, and $R^p$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and $R^{bb}$, $R^{cc}$, $R^{dd}$, $R^{ee}$, $R^{ff}$, $R^{gg}$, $R^{mm}$, and $R^{nn}$ may each independently be $(CH_2)_v$, wherein v is a positive integer (e.g., any integer having a value of 1 or greater, 1 to 10, 1 to 8, or 1 to 6), or a heteroatom of O, NR$^v$, wherein $R^v$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{mm}$ and $R^{nn}$ may be linked to each other to provide a ring structure, and n1 of -(CR$^f$R$^g$)$_{n1}$- may be 1 or 2,
$R^{11}$ and $R^{12}$ may each independently be hydrogen, deuterium, a halogen, a cyano group, a substituted or unsubstituted C1 to C20 alkyl group, a substituted or unsubstituted C6 to C20 aryl group, or a substituted or unsubstituted C6 to C20 aryloxy group, wherein $R^{11}$ and $R^{12}$ may each independently be present or may be linked to each other to provide a ring structure, or -CR$^{11}$R$^{12}$- may be linked with $Ar^1$ or $Ar^2$ to provide a ring structure,
$Ar^3$ may be a substituted or unsubstituted C6 to C30 hydrocarbon cyclic group having at least one functional group selected from C=O, C=S, C=Se, and C=Te, a substituted or unsubstituted C2 to C30 heterocyclic group having at least one functional group selected from C=O, C=S, C=Se, and C=Te, or a fused ring thereof, and
$R^1$ and $R^2$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group ($-SF_5$), a hydroxyl group, an amine group, a hydrazine group, a hydrazone group, a carboxyl group or a salt thereof, a sulfonic acid group or a salt thereof, a phosphoric acid group or a salt thereof, -SiR$^a$R$^b$R$^c$, wherein $R^a$, $R^b$, and $R^c$ may each independently be hydrogen or a substituted or unsubstituted C1 to C10 alkyl group, or any combination thereof.

The compound represented by Chemical Formula 1 includes an electron donor moiety including a first ring moiety including nitrogen (N) and carbon (C) and an $X^1$-containing second ring moiety; and an electron acceptor moiety represented by $Ar^3$; wherein the first ring moiety including the nitrogen (N) and carbon (C) and the $X^1$-containing second ring moiety are fused to each other by a third ring moiety including nitrogen (N) and $X^2$ to provide a fused ring. In Chemical Formula 1, by fusing the first ring moiety including the nitrogen (N) and carbon (C) and the $X^1$-containing second ring moiety, by the third ring moiety including nitrogen (N) and $X^2$ to provide a fused ring, stability of the molecular structure of the compound may be improved, decomposition of the compound in the deposition process may be limited and/or prevented, so that the reliability of the device may be improved. In addition, in the first ring moiety including nitrogen (N) and carbon (C), $Ar^1$ and $Ar^2$ are linked by carbon (C), so that stability of the molecular structure may be further improved.

The compound represented by Chemical Formula 1 includes an electron donor moiety including a first ring moiety including nitrogen (N) and carbon (C) and an $X^1$-containing second ring moiety; and an electron acceptor moiety represented by $Ar^3$; wherein the first ring moiety including the nitrogen (N) and carbon (C) and the $X^1$-containing second ring moiety are fused to each other by a third ring moiety including nitrogen (N) and $X^2$ to provide a fused ring.

In some example embodiments, the p-type semiconductor compound of Chemical Formula 1 may be represented by Chemical Formula 2A.

[Chemical Formula 2A]

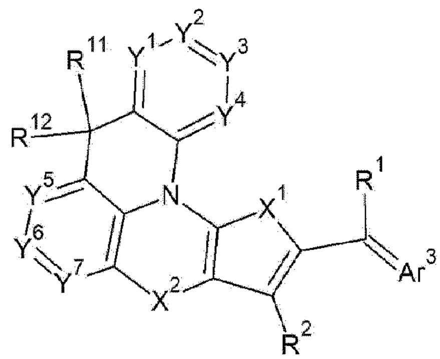

In Chemical Formula 2A,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$Y^1$ to $Y^7$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof.

In some example embodiments, in Chemical Formula 2A, $Y^4$ may be N or $CR^k$, wherein $R^k$ is a halogen, a cyano group, a C1 to C10 haloalkyl group, or a C1 to C10 cyanoalkyl group. In this case, $Y^4$, N, $X^1$, and functional groups (C=O, C=S, C=Se, or C=Te) of $Ar^3$ increase an intramolecular interaction, thereby increasing the absorption intensity at a specific wavelength.

In some example embodiments, in Chemical Formula 2A, $Y^7$ may be N or $CR^k$, wherein $R^k$ is a halogen, a cyano group, a C1 to C10 haloalkyl group, or a C1 to C10 cyanoalkyl group and $X^2$ may be -O-, -S-, -Se-, -Te-, -S(=O)-, $-S(=O)_2-$, $-NR^{a1}-$, - $BR^{a2}-$, $-SiR^bR^c-$, $-GeR^dR^e-$, $-(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group, and n1 of $-(CR^fR^g)_{n1}-$may be 1 or 2. In this case, $Y^7$ and $X^2$ may increase an intramolecular interaction, thereby improving the absorption intensity at a specific wavelength.

In some example embodiments, in Chemical Formula 2A, when $Y^1$ and $Y^5$ are $CR^k$, at least one of Y' or $Y^5$ and carbon (C) may be linked to each other to provide a fused ring. This structure may be represented by Chemical Formula 2A-1 or Chemical Formula 2A-2.

[Chemical Formula 2A-1]

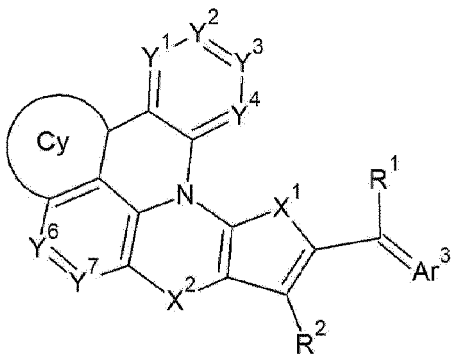

In Chemical Formula 2A-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^1$ to $Y^4$, $Y^6$ and $Y^7$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C3 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

[Chemical Formula 2A-2]

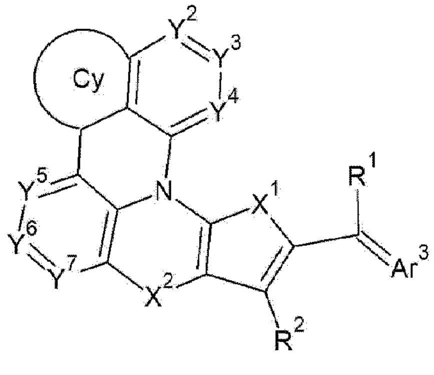

In Chemical Formula 2A-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^2$ to $Y^7$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or

unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C5 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

In some example embodiments, Cy of Chemical Formula 2A-1 and Chemical Formula 2A-2 may be an arene group, a heteroarene group, a cycloalkene group, or a heterocycloalkene group, and they may have a 5-membered to 10-membered ring structure. The heteroarene group or heterocycloalkene group may include N in the ring.

When Cy of Chemical Formula 2A-1 has a 6-membered ring structure, the compound of Chemical Formula 2A-1 may be represented by Chemical Formula 2A-11a.

[Chemical Formula 2A-11a]

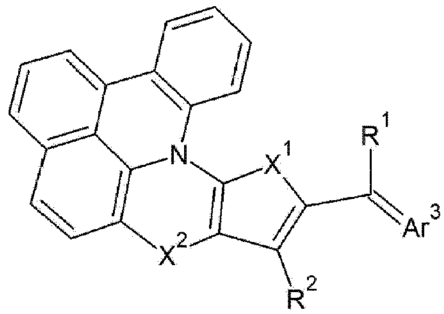

In Chemical Formula 2A-11a,

$X^1$, $X^2$, $Ar^3$, $R^1$, and $R^2$ are the same as $X^1$, $X^2$, $Ar^3$, $R^1$, and $R^2$, respectively, in Chemical Formula 1, and hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2A-11a may be replaced by N, and one or more (e.g., 1, 2, or 3) N may be included in one 6-member ring structure.

When Cy of Chemical Formula 2A-2 has a 6-membered ring structure, the compound of Chemical Formula 2A-2 may be represented by Chemical Formula 2A-21a.

[Chemical Formula 2A-21a]

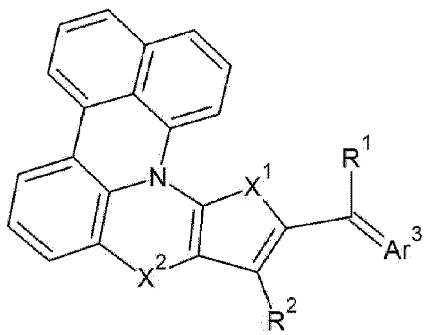

In Chemical Formula 2A-21a,

$X^1$, $X^2$, $Ar^3$, $R^1$, and $R^2$ are the same as $X^1$, $X^2$, $Ar^3$, $R^1$, and $R^2$, respectively, in Chemical Formula 1, and hydrogen of each aromatic ring may be replaced by at least one substituent selected from deuterium, a halogen, a

substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2A-21a may be replaced by N, and one or more (e.g., 1, 2, or 3) N may be included in one 6-member ring structure.

In some example embodiments, in Chemical Formula 2A, $Y^1$ ($CR^k$) and carbon (C) may be linked to each other to provide a first fused ring and $Y^5$ ($CR^k$) and carbon (C) may be linked to each other to provide a second fused ring. When the first fused ring and the second fused ring each have a 6-membered ring structure, it may be represented by Chemical Formula 2A-3a.

[Chemical Formula 2A-3a]

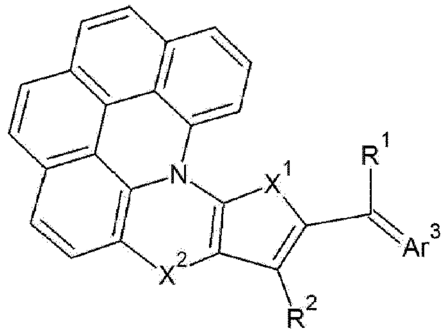

In Chemical Formula 2A-3a,

$X^1$, $X^2$, $Ar^3$, $R^1$, and $R^2$ are the same as $X^1$, $X^2$, $Ar^3$, $R^1$, and $R^2$, respectively, in Chemical Formula 1, and hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2A-3a may be replaced by N, and one or more (e.g., 1, 2, or 3) N may be included in one 6-member ring structure.

In some example embodiments, in Chemical Formula 2A, when $Y^1$ to $Y^4$ are $CR^k$ and adjacent $R^k$'s are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2A-41 to Chemical Formula 2A-44.

[Chemical Formula 2A-41]

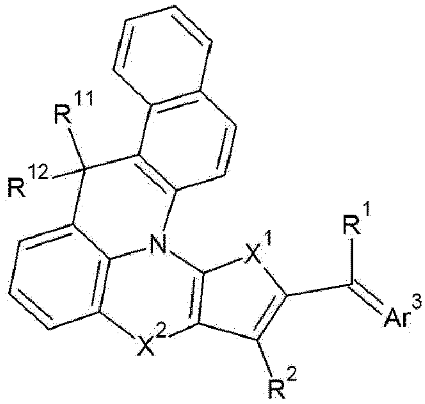

[Chemical Formula 2A-42]

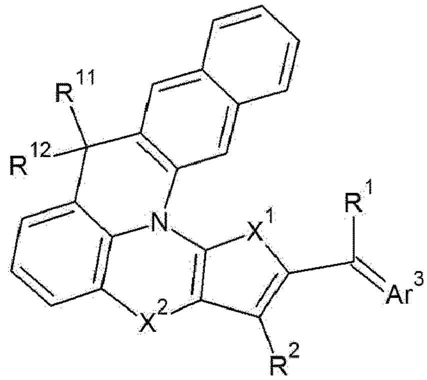

[Chemical Formula 2A-43]

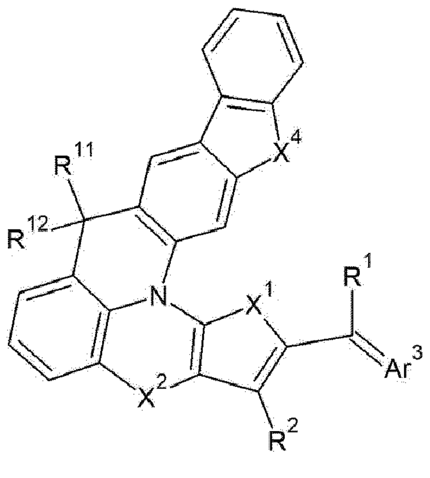

[Chemical Formula 2A-44]

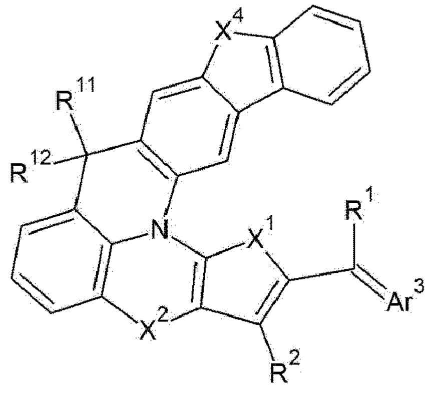

In Chemical Formula 2A-41 to Chemical Formula 2A-44,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, $X^4$ may be -O-, -S-, -Se-, -Te-, -S(=O)-, -S(=O)$_2$-, -NR$^{a1}$-, -BR$^{a2}$-, -SiR$^b$R$^c$-, - SiR$^{bb}$R$^{cc}$-, -GeR$^d$R$^e$-, -GeR$^{dd}$R$^{ee}$-, -CR$^f$R$^g$-, -CR$^{ff}$R$^{gg}$-, -CR$^h$=CR$^i$-, or -CR$^{hh}$=CR$^{ii}$-, wherein $R^{a1}$, $R^{a2}$, $R^b$, $R^c$, $R^d$, $R^e$, $R^f$, $R^g$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and $R^{bb}$, $R^{cc}$, $R^{dd}$, $R^{ee}$, $R^{ff}$, $R^{gg}$, $R^{hh}$, and $R^{ii}$ may each independently be $(CH_2)_t$, wherein t is a positive integer (e.g., any integer having a value of 1 or greater, 1 to 10, 1 to 8, or 1 to 6), or a heteroatom of O, NR$^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{hh}$ and $R^{ii}$ may be linked to each other to provide a ring structure,

hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2A-41 to Chemical Formula 2A-44, CH of the aromatic ring may be replaced by N and one or more (e.g., 1, 2, or 3) N may be included in one aromatic ring.

In Chemical Formula 2A, when $Y^5$ to $Y^7$ are CR$^k$ and adjacent R$^k$'s are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2A-45 to Chemical Formula 2A-48.

[Chemical Formula 2A-45]

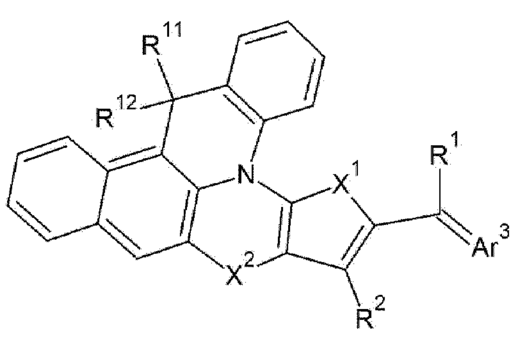

[Chemical Formula 2A-46]

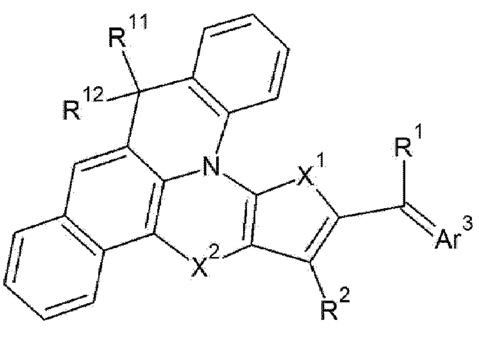

[Chemical Formula 2A-47]

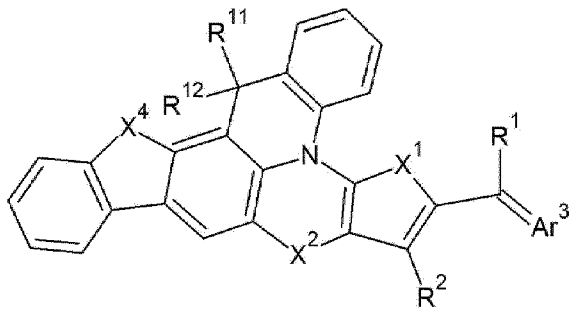

[Chemical Formula 2A-48]

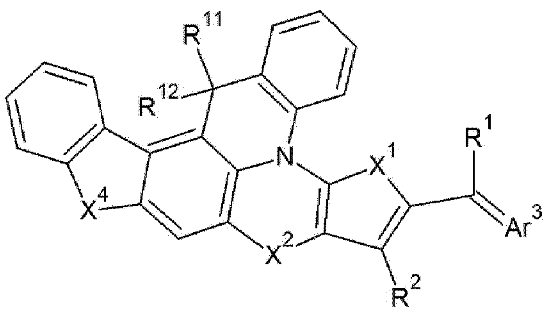

In Chemical Formula 2A-45 to Chemical Formula 2A-48,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, $X^4$ may be -O-, -S-, -Se-, -Te-, -S(=O)-, -S(=O)$_2$-, -NR$^{a1}$-, -BR$^{a2}$-, -SiR$^b$R$^c$-, - SiR$^{bb}$R$^{cc}$-, -GeR$^d$R$^e$-, -GeR$^{dd}$R$^{ee}$-, -CR$^f$R$^g$-, -CR$^{ff}$R$^{gg}$-, -CR$^h$=CR$^i$-, or -CR$^{hh}$=CR$^{ii}$-, wherein $R^{a1}$, $R^{a2}$, $R^b$, $R^c$, $R^d$, $R^e$, $R^f$, $R^g$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and $R^{bb}$, $R^{cc}$, $R^{dd}$, $R^{ee}$, $R^{ff}$, $R^{gg}$, $R^{hh}$, and $R^{ii}$ may each independently be $(CH_2)_t$, wherein t is a positive integer (e.g., any integer having a value of 1 or greater, 1 to 10, 1 to 8, or 1 to 6), or a heteroatom of O, NR$^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{hh}$ and $R^{ii}$ may be linked to each other to provide a ring structure, and
hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2A-45 to Chemical Formula 2A-48, CH of the aromatic ring may be replaced by N and one or more (e.g., 1, 2, or 3) N may be included in one aromatic ring.
In some example embodiments, the compound of Chemical Formula 1 may be represented by Chemical Formula 2B.

[Chemical Formula 2B]

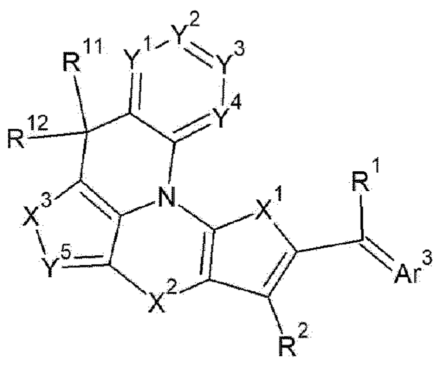

In Chemical Formula 2B,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, $Y^1$ to $Y^5$ may each independently be N or CR$^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

$X^3$ may be -O-, -S-, -Se-, -Te-, -S(=O)-, -S(=O)$_2$-, -NR$^{a1}$-, -BR$^{a2}$-, -SiR$^bR^c$-, - SiR$^{bb}R^{cc}$-, -GeR$^dR^e$-, -GeR$^{dd}R^{ee}$-, -CR$^fR^g$-, or -CR$^{ff}R^{gg}$-, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and $R^{bb}$, $R^{cc}$, $R^{dd}$, $R^{ee}$, $R^{ff}$, and $R^{gg}$ may each independently be $(CH_2)_u$, wherein u is a positive integer (e.g., any integer having a value of 1 or greater, 1 to 10, 1 to 8, or 1 to 6) or a heteroatom of O, NR$^u$, wherein $R^u$ is hydrogen or a C1 to C10 alkyl group, S, Se, or Te, and/or at least one pair of $R^{bb}$ and $R^{cc}$, $R^{dd}$ and $R^{ee}$, or $R^{ff}$ and $R^{gg}$ may be linked to each other to provide a ring structure.

In some example embodiments, in Chemical Formula 2B, $Y^4$ may be N or CR$^k$, wherein $R^k$ is a halogen, a cyano group, a C1 to C10 haloalkyl group, or a C1 to C10 cyanoalkyl group. In this case, $Y^4$, N, $X^1$, and functional groups (C=O, C=S, C=Se, or C=Te) of $Ar^3$ increase an intramolecular interaction, thereby increasing the absorption intensity at a specific wavelength.

In some example embodiments, in Chemical Formula 2B, $Y^5$ may be N or CR$^k$, wherein $R^k$ is a halogen, a cyano group, a C1 to C10 haloalkyl group, or a C1 to C10 cyanoalkyl group and $X^2$ may be -O-, -S-, -Se-, -Te-, -S(=O)-, -S(=O)$_2$-, -NR$^{a1}$-, - BR$^{a2}$-, -SiR$^bR^c$-, -GeR$^dR^e$-, -(CR$^fR^g$)$_{n1}$-, -(C(R$^m$)=C(R$^n$))-, or -(C(R$^p$)=N)-, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group, and n1 of -(CR$^fR^g$)$_{n1}$-may be 1 or 2. In this case, $Y^5$ and $X^2$ may increase an intramolecular interaction, thereby improving the absorption intensity at a specific wavelength.

In some example embodiments, in Chemical Formula 2B, $X^3$ (-NR$^{a1}$-, -BR$^{a2}$-, -SiR$^bR^c$-, -GeR$^dR^e$-, or -CR$^fR^g$-) and carbon (C) may be linked to each other to provide a fused ring. This structure may be represented by Chemical Formula 2B-1.

[Chemical Formula 2B-1]

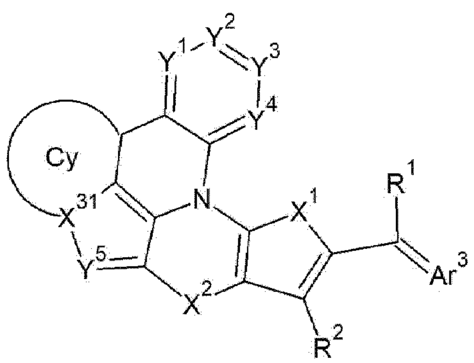

In Chemical Formula 2B-1,

$X^1$, $X^2$, $Ar^3$, $R^1$, and $R^2$ are the same as $X^1$, $X^2$, $Ar^3$, $R^1$, and $R^2$, respectively, in Chemical Formula 1,

$X^{31}$ may be N, B, SiR$^b$, GeR$^d$, CR$^f$, Si, Ge, or C, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,

$Y^1$ to $Y^5$ may each independently be N or CR$^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C3 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

In some example embodiments, in Chemical Formula 2B, $Y^1$ ($CR^k$) and carbon (C) may be linked to each other to provide a fused ring. This structure may be represented by Chemical Formula 2B-2.

[Chemical Formula 2B-2]

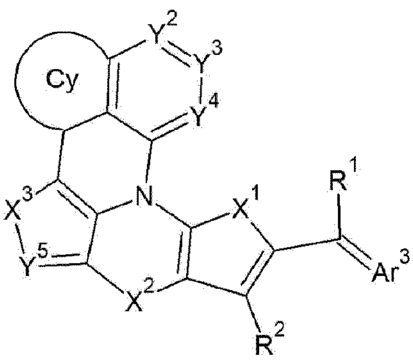

In Chemical Formula 2B-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ and $Y^5$ are the same as $X^3$ and $Y^5$, respectively, in Chemical Formula 2B,
$Y^2$ to $Y^4$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C3 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

In some example embodiments, Cy of Chemical Formula 2B-1 and Chemical Formula 2B-2 may be an arene group, a heteroarene group, a cycloalkene group, or a heterocycloalkene group, and they may have a 5-membered to 10-membered ring structure. The heteroarene group or heterocycloalkene group may include N in the ring.

When Cy of Chemical Formula 2B-1 has a 6-membered ring structure, the compound of Chemical Formula 2B-1 may be represented by Chemical Formula 2B-11a.

[Chemical Formula 2B-11a]

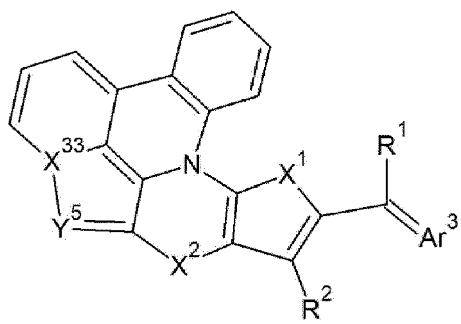

In Chemical Formula 2B-11a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^5$ is the same as $Y^5$ in Chemical Formula 2B,
$X^{33}$ may be N, B, $SiR^b$, $GeR^d$, or $CR^f$, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and
hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2B-11a may be replaced by N, and one or more (e.g., 1, 2, or 3) N may be included in one 6-member ring structure.

When Cy of Chemical Formula 2B-2 has a 6-membered ring structure, the compound of Chemical Formula 2B-2 may be represented by Chemical Formula 2B-21a.

[Chemical Formula 2B-21a]

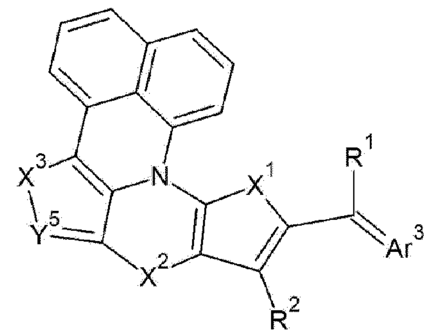

In Chemical Formula 2B-21a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ and $Y^5$ are the same as $X^3$ and $Y^5$, respectively, in Chemical Formula 2B, and
hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2B-21a may be replaced by N, and one or more (e.g., 1, 2, or 3) N may be included in one 6-member ring structure.

In some example embodiments, in Chemical Formula 2B, $Y^1$ ($CR^k$) and carbon (C) may be linked to each other to provide a first fused ring and $X^3$ ($-NR^{a1}-$, $-BR^{a2}-$, $-SiR^bR^c-$, $-GeR^dR^e-$, or $-CR^fR^g-$) and carbon (C) may be linked to each other to provide a second fused ring. When the first fused ring and the second fused ring each have a 6-membered ring structure, it may be represented by Chemical Formula 2B-3a.

[Chemical Formula 2B-3a]

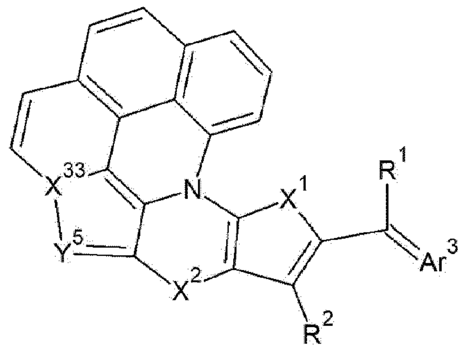

In Chemical Formula 2B-3a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^5$ is the same as $Y^5$ in Chemical Formula 2B,
$X^{33}$ may be N, B, $SiR^b$, $GeR^d$, or $CR^f$, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and
hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2B-3a may be replaced by N, and one or more (e.g., 1, 2, or 3) N may be included in one 6-member ring structure.

In some example embodiments, in Chemical Formula 2B, when $Y^1$ to $Y^4$ are $CR^k$ and adjacent $R^k$'s are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2B-41 to Chemical Formula 2B-44.

[Chemical Formula 2B-41]

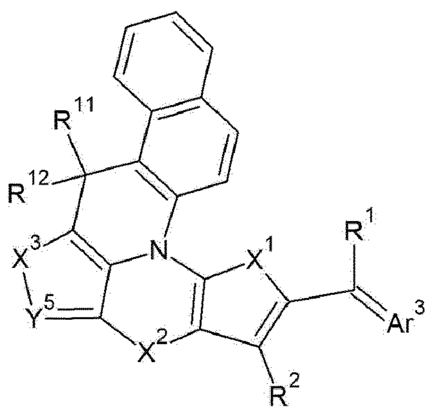

[Chemical Formula 2B-42]

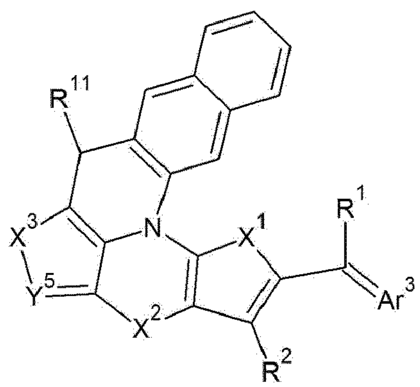

[Chemical Formula 2B-43]

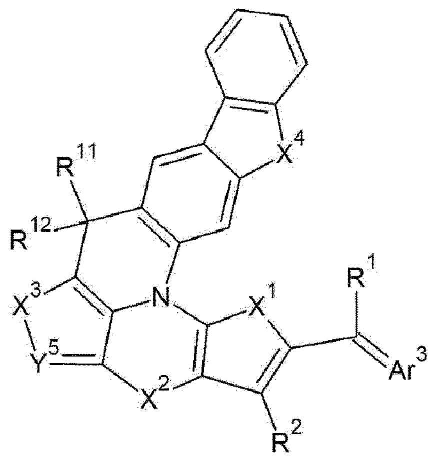

[Chemical Formula 2B-44]

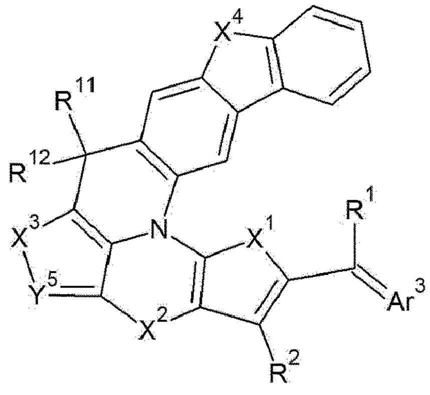

In Chemical Formula 2B-41 to Chemical Formula 2B-44,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$X^3$ and $Y^5$ are the same as $X^3$ and $Y^5$, respectively, in Chemical Formula 2B,

$X^4$ may be -O-, -S-, -Se-, -Te-, -S(=O)-, -S(=O)$_2$-, -NR$^{a1}$-, -BR$^{a2}$-, -SiR$^b$R$^c$-, - SiR$^{bb}$R$^{cc}$-, -GeR$^d$R$^e$-, -GeR$^{dd}$R$^{ee}$-, -CR$^f$R$^g$-, -CR$^{ff}$R$^{gg}$-, -CR$^h$=CR$^i$-, or -CR$^{hh}$=CR$^{ii}$-, wherein $R^{a1}$, $R^{a2}$, $R^b$, $R^c$, $R^d$, $R^e$, $R^f$, $R^g$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, $R^{gg}$, $R^{hh}$, and $R^{ii}$ may each independently be $(CH_2)_t$, wherein t is a positive integer (e.g., any integer having a value of 1 or greater, 1 to 10, 1 to 8, or 1 to 6), or a heteroatom of O, NR$^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{hh}$ and $R^{ii}$ may be linked to each other to provide a ring structure, and

hydrogen of each aromatic ring may be replaced by at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2B-41 to Chemical Formula 2B-44, CH of the aromatic ring may be replaced by N and one or more (e.g., 1, 2, or 3) N may be included in one aromatic ring.

In some example embodiments, in Chemical Formula 2B, when $X^3$ and $Y^5$ are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2B-45 to Chemical Formula 2B-50.

[Chemical Formula 2B-45]

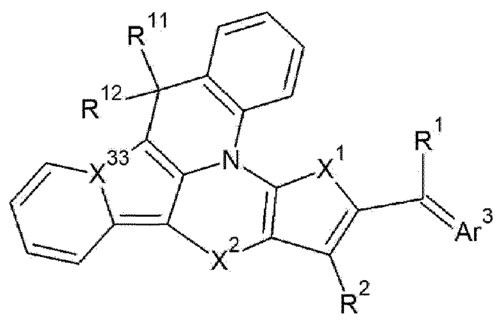

[Chemical Formula 2B-46]

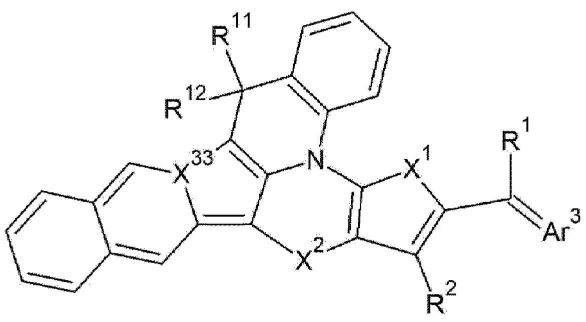

[Chemical Formula 2B-47]

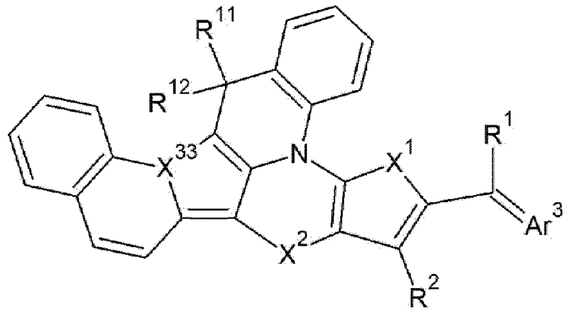

[Chemical Formula 2B-48]

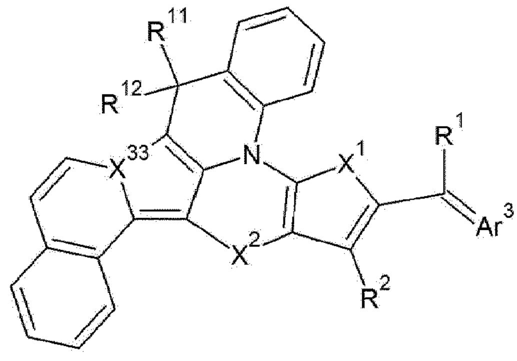

[Chemical Formula 2B-49]

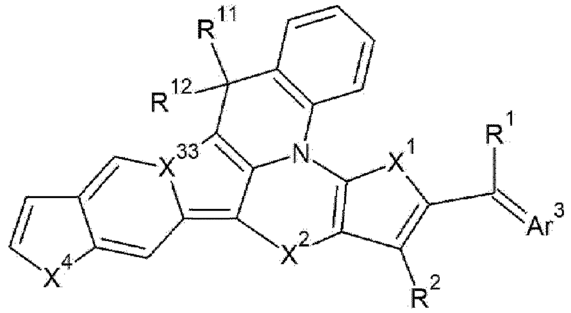

[Chemical Formula 2B-50]

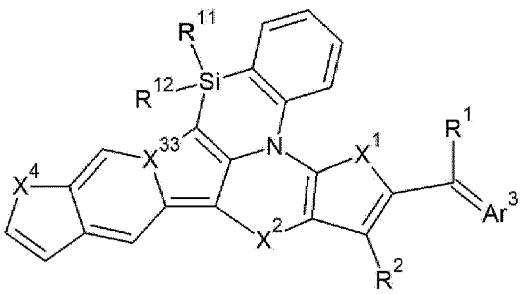

In Chemical Formula 2B-45 to Chemical Formula 2B-50,

$X^1$, $X^2$, $Ar^3$, $R^{11}$, $R^{12}$, $R^1$, and $R^2$ are the same as $X^1$, $X^2$, $Ar^3$, $R^{11}$, $R^{12}$, $R^1$, and $R^2$, respectively, in Chemical Formula 1,
$X^{33}$ may be N, B, $SiR^b$, $GeR^d$, or $CR^f$, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,
$X^4$ may be -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$, $-CR^{ff}R^{gg}-$, $-CR^h=CR^i-$, or $-CR^{hh}=CR^{ii}-$, wherein $R^{a1}$, $R^{a2}$, $R^b$, $R^c$, $R^d$, $R^e$, $R^f$, $R^g$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, $R^{gg}$, $R^{hh}$, and $R^{ii}$ may each independently be $(CH_2)_t$, wherein t is a positive integer (e.g., any integer having a value of 1 or greater, 1 to 10, 1 to 8, or 1 to 6), or a heteroatom of O, $NR^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{hh}$ and $R^{ii}$ may be linked to each other to provide a ring structure, and
hydrogen of each aromatic ring may be replaced by at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2B-45 to Chemical Formula 2B-50, CH of the aromatic ring may be replaced by N and one or more (e.g., 1, 2, or 3) N may be included in one aromatic ring.

In some example embodiments, the p-type semiconductor compound of Chemical Formula 1 may be represented by Chemical Formula 2C.

[Chemical Formula 2C]

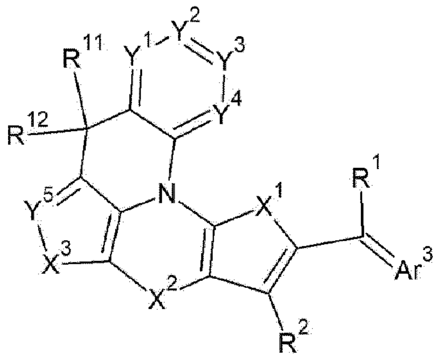

In Chemical Formula 2C,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$Y^1$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof,

$X^3$ may be -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, and $R^{gg}$ may each independently be $(CH_2)_u$, wherein u is a positive integer (e.g., any integer having a value of 1 or greater, 1 to 10, 1 to 8, or 1 to 6) or a heteroatom of O, $NR^u$, wherein $R^u$ is hydrogen or a C1 to C10 alkyl group, S, Se, or Te, and/or at least one pair of $R^{bb}$ and $R^{cc}$, $R^{dd}$ and $R^{ee}$, or $R^{ff}$ and $R^{gg}$ may be linked to each other to provide a ring structure.

In some example embodiments, in Chemical Formula 2C, $Y^4$ may be N or $CR^k$, wherein $R^k$ is a halogen, a cyano group, a C1 to C10 haloalkyl group, or a C1 to C10 cyanoalkyl group. In this case, $Y^4$, N, $X^1$, and functional groups (C=O, C=S, C=Se, or C=Te) of $Ar^3$ increase an intramolecular interaction, thereby increasing the absorption intensity at a specific wavelength.

In some example embodiments, in Chemical Formula 2C, $X^3$ may be -O-, -S-, -Se-, -Te-, -S(=O)-, $-S(=O)_2-$, $-NR^{a1}-$, $-BR^{a2}-$, $-SiR^bR^c-$, $-GeR^dR^e-$, or $-CR^fR^g-$, wherein $R^{a1}$, $R^{a2}$, $R^b$, $R^c$, $R^d$, $R^e$, $R^f$, and $R^g$ may each independently be a halogen, a C1 to C20 haloalkyl group, or a C1 to C20 cyanoalkyl group, and $X^2$ may be -O-, -S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, - $(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group and n1 of $-(CR^fR^g)_{n1}-$ may be 1 or 2. In this case, $X^3$ and $X^2$ may increase an intramolecular interaction, thereby improving the absorption intensity at a specific wavelength.

In some example embodiments, in Chemical Formula 2C, when $Y^5$ is $CR^k$, $Y^5$ and carbon (C) may be linked to each other to provide a fused ring. This structure may be represented by Chemical Formula 2C-1.

[Chemical Formula 2C-1]

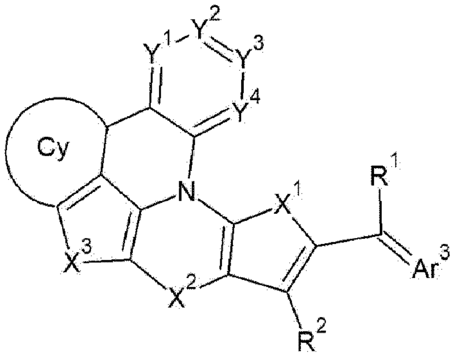

In Chemical Formula 2C-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2C,
$Y^1$ to $Y^4$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C3 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

In some example embodiments, in Chemical Formula 2C, when $Y^1$ is $CR^k$, $Y^1$ and carbon (C) may be linked to each other to provide a fused ring. This structure may be represented by Chemical Formula 2C-2.

[Chemical Formula 2C-2]

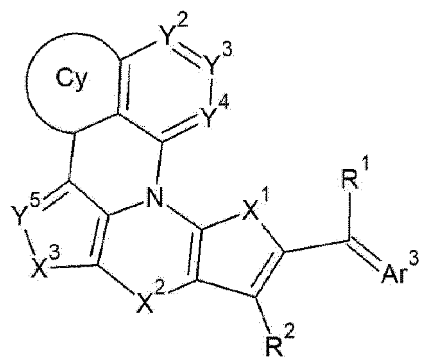

In Chemical Formula 2C-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ and $Y^5$ are the same as $X^3$ and $Y^5$, respectively, in Chemical Formula 2C,
$Y^2$ to $Y^4$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or

unsubstituted C6 to C30 arene group, a substituted or unsubstituted C3 to C30 heteroarene group, or a condensed ring thereof, and

Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; or a condensed ring thereof.

In some example embodiments, Cy of Chemical Formula 2C-1 and Chemical Formula 2C-2 may be an arene group, a heteroarene group, a cycloalkene group, or a heterocycloalkene group, and they may have a 5-membered to 10-membered ring structure. The heteroarene group or heterocycloalkene group may include N in the ring.

When Cy of Chemical Formula 2C-1 has a 6-membered ring structure, the p-type semiconductor compound of Chemical Formula 2C-1 may be represented by Chemical Formula 2C-11a.

[Chemical Formula 2C-11a]

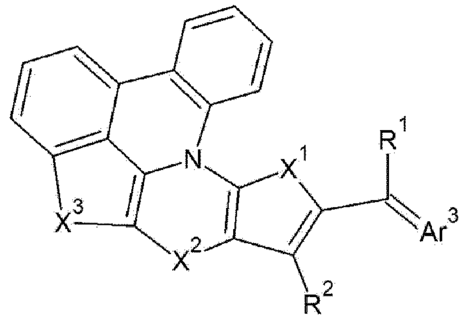

In Chemical Formula 2C-11a,

$X^1$, $X^2$, $Ar^3$, $R^1$, and $R^2$ are the same as $X^1$, $X^2$, $Ar^3$, $R^1$, and $R^2$, respectively, in Chemical Formula 1,

$X^3$ is the same as $X^3$ in Chemical Formula 2C, and

hydrogen of each aromatic ring may be replaced by at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2C-11a may be replaced by N, and one or more (e.g., 1, 2, or 3) N may be included in one 6-member ring structure.

When Cy of Chemical Formula 2C-2 has a 6-membered ring structure, the p-type semiconductor compound of Chemical Formula 2C-2 may be represented by Chemical Formula 2C-21a.

[Chemical Formula 2C-21a]

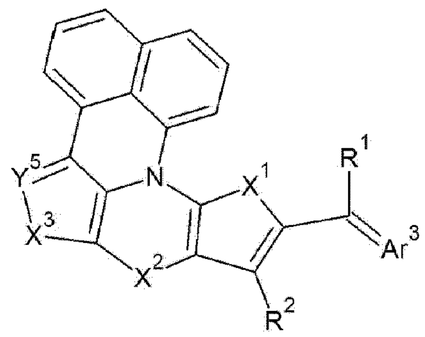

In Chemical Formula 2C-21a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ and $Y^5$ are the same as $X^3$ and $Y^5$, respectively, in Chemical Formula 2C, and
hydrogen of each aromatic ring may be replaced by at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2C-21a may be replaced by N, and one or more (e.g., 1, 2, or 3) N may be included in one 6-member ring structure.

In some example embodiments, in Chemical Formula 2C, $Y^5$ ($CR^k$) and carbon (C) may be linked to each other to provide a first fused ring and $Y^1$ ($CR^k$) and carbon (C) may be linked to each other to provide a second fused ring. When the first fused ring and the second fused ring each have a 6-membered ring structure, it may be represented by Chemical Formula 2C-3a.

[Chemical Formula 2C-3a]

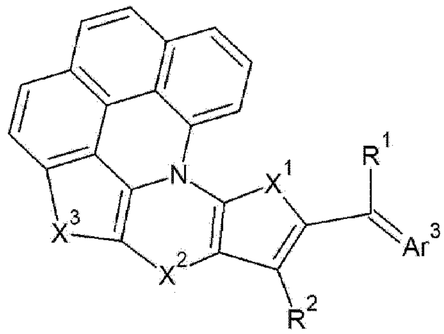

In Chemical Formula 2C-3a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2C, and
hydrogen of each aromatic ring may be replaced by at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2C-3a may be replaced by N, and one or more (e.g., 1, 2, or 3) N may be included in one 6-member ring structure.

In Chemical Formula 2C, when $Y^1$ to $Y^4$ are $CR^k$ and adjacent $R^k$'s are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2C-41 to Chemical Formula 2C-44.

[Chemical Formula 2C-41]

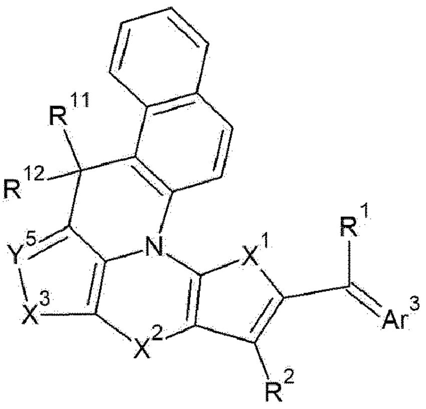

[Chemical Formula 2C-42]

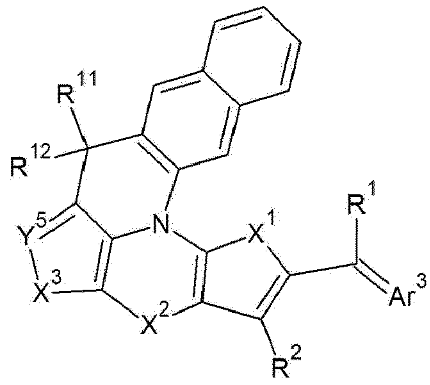

[Chemical Formula 2C-43]

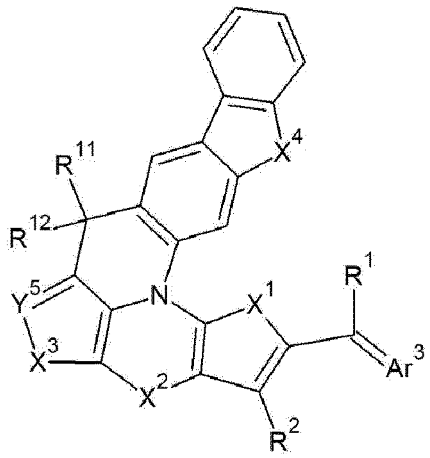

[Chemical Formula 2C-44]

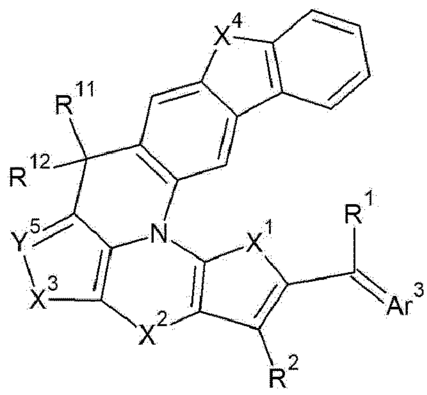

In Chemical Formula 2C-41 to Chemical Formula 2C-44,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$X^3$ and $Y^5$ are the same as $X^3$ and $Y^5$, respectively, in Chemical Formula 2C,

$X^4$ may be -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, $-CR^{ff}R^{gg}-$, $-CR^h=CR^i-$, or $-CR^{hh}=CR^{ii}-$, wherein $R^{a1}$, $R^{a2}$, $R^b$, $R^c$, $R^d$, $R^e$, $R^f$, $R^g$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, $R^{gg}$, $R^{hh}$, and $R^{ii}$ may each independently be $(CH_2)_t$, wherein t is a positive integer (e.g., any integer having a value of 1 or greater, 1 to 10, 1 to 8, or 1 to 6) or a heteroatom of O, $NR^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{hh}$ and $R^{ii}$ may be linked to each other to provide a ring structure,

hydrogen of each aromatic ring may be replaced by at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2C-41 to Chemical Formula 2C-44, CH of the aromatic ring may be replaced by N and one or more (e.g., 1, 2, or 3) N may be included in one aromatic ring.

In Chemical Formula 2C, when $X^3$ and $Y^5$ are linked to each other to provide a fused ring (a substituted or unsubstituted C6 to C30 arene group, a substituted or unsubstituted C3 to C30 heteroarene group, a substituted or

unsubstituted C5 to C30 cycloalkene group, a substituted or unsubstituted C3 to C30 heterocycloalkene group, or any combination thereof), it may be represented by one of Chemical Formula 2C-45 to Chemical Formula 2C-50.

[Chemical Formula 2C-45]

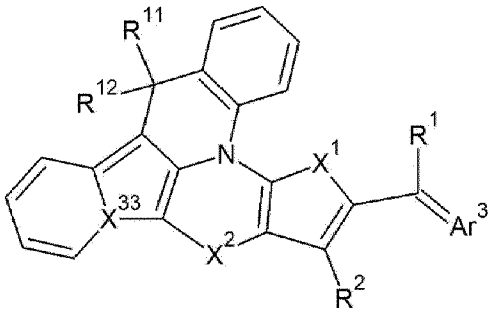

[Chemical Formula 2C-46]

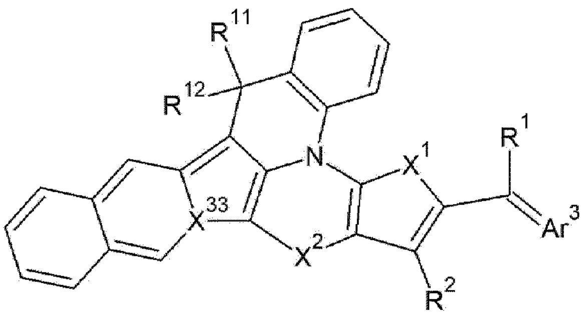

[Chemical Formula 2C-47]

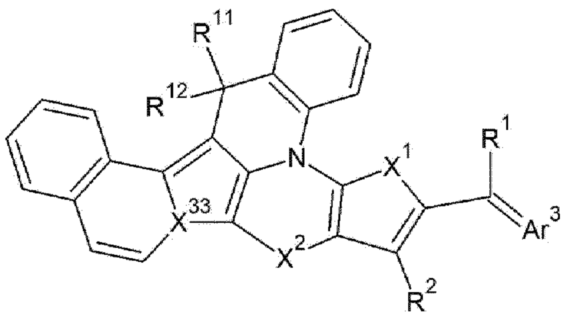

[Chemical Formula 2C-48]

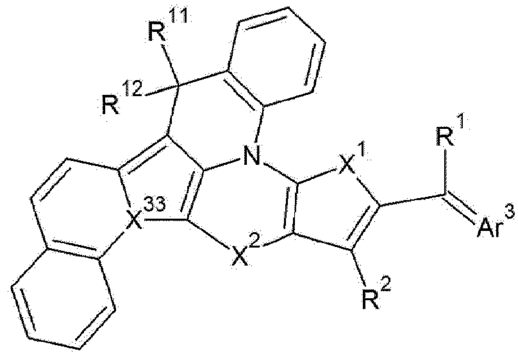

[Chemical Formula 2C-49]

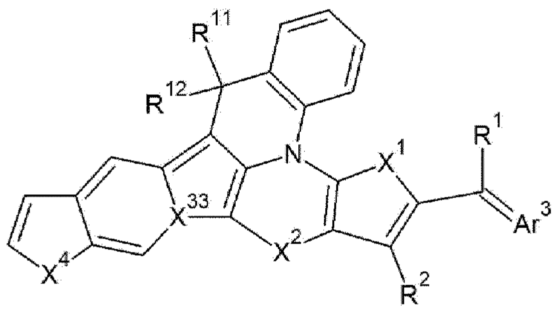

[Chemical Formula 2C-50]

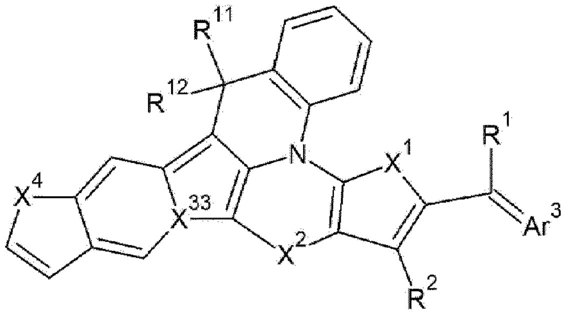

In Chemical Formula 2C-45 to Chemical Formula 2C-50,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$X^{33}$ may be N, B, $SiR^b$, $GeR^d$, or $CR^f$, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,

$X^4$ may be -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, $-CR^{ff}R^{gg}-$, $-CR^h=CR^i-$, or $-CR^{hh}=CR^{ii}-$, wherein $R^{a1}$, $R^{a2}$, $R^b$, $R^c$, $R^d$, $R^e$, $R^f$, $R^g$, $R^h$, and $R^i$ may each independently be hydrogen, deuterium, a halogen, a cyano group, a substituted or unsubstituted C1 to C20 alkyl

group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, $R^{gg}$, $R^{hh}$, and $R^{ii}$ may each independently be $(CH_2)_t$, wherein t is a positive integer (e.g., any integer having a value of 1 or greater, 1 to 10, 1 to 8, or 1 to 6), or a heteroatom of O, $NR^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{hh}$ and $R^{ii}$ may be linked to each other to provide a ring structure, and

hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2C-45 to Chemical Formula 2C-50, CH of the aromatic ring may be replaced by N and one or more (e.g., 1, 2, or 3) N may be included in one aromatic ring.

In some example embodiments, the p-type semiconductor compound of Chemical Formula 1 may be represented by Chemical Formula 2D.

[Chemical Formula 2D]

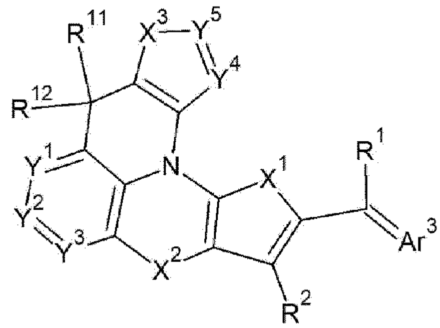

In Chemical Formula 2D,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1,

$Y^1$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

$X^3$ may be -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, and $R^{gg}$, may each independently be $(CH_2)_u$, wherein u is a positive integer (e.g., any integer having a value of 1 or greater, 1 to 10, 1 to 8, or 1 to 6) or a heteroatom of O, $NR^u$, wherein $R^u$ is hydrogen or a C1 to C10 alkyl group, S, Se, or Te, and/or at least one pair of $R^{bb}$ and $R^{cc}$, $R^{dd}$ and $R^{ee}$, or $R^{ff}$ and $R^{gg}$ may be linked to each other to provide a ring structure.

In some example embodiments, in Chemical Formula 2D, $Y^4$ may be N or $CR^k$, wherein $R^k$ may be a halogen, a cyano group, a C1 to C10 haloalkyl group, or a C1 to C10 cyanoalkyl group. In this case, $Y^4$, N, $X^1$, and functional groups (C=O, C=S, C=Se, or C=Te) of $Ar^3$ increase an intramolecular interaction, thereby increasing the absorption intensity at a specific wavelength.

In some example embodiments, in Chemical Formula 2D, $Y^3$ may be N or $CR^k$, wherein $R^k$ may be a halogen, a cyano group, a C1 to C10 haloalkyl group, or a C1 to C10 cyanoalkyl group, and $X^2$ may be -O-, -S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group, and n1 of $-(CR^fR^g)_{n1}-$ may be 1 or 2. In this case, $Y^3$ and $X^2$ may increase an intramolecular interaction, thereby improving the absorption intensity at a specific wavelength.

In some example embodiments, in Chemical Formula 2D, when $Y^1$ is $CR^k$, $Y^1$ and carbon (C) may be linked

to each other to provide a fused ring. This structure may be represented by Chemical Formula 2D-1.

[Chemical Formula 2D-1]

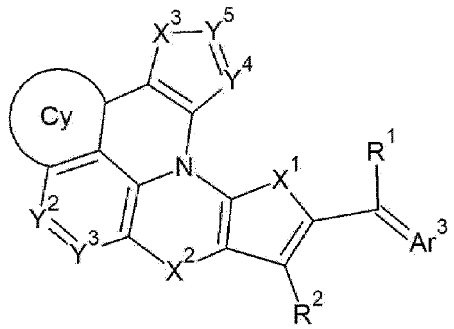

In Chemical Formula 2D-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2D,
$Y^2$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C3 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

In some example embodiments, in Chemical Formula 2D, $X^3$ ($-NR^{a1}-$, $-BR^{a2}-$, $-SiR^bR^c-$, $-GeR^dR^e-$, or $-CR^fR^g-$) and carbon (C) may be linked to each other to provide a fused ring. This structure may be represented by Chemical Formula 2D-2.

[Chemical Formula 2D-2]

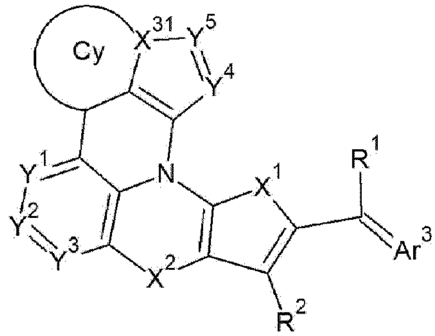

In Chemical Formula 2D-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^{31}$ may be N, B, $SiR^b$, $GeR^d$, $CR^f$, Si, Ge, or C, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted

C6 to C20 aryloxy group,

$Y^1$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C3 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

In some example embodiments, Cy of Chemical Formula 2D-1 and Chemical Formula 2D-2 may be an arene group, a heteroarene group, a cycloalkene group, or a heterocycloalkene group, and they may have a 5-membered to 10-membered ring structure. The heteroarene group or heterocycloalkene group may include N in the ring.

When Cy of Chemical Formula 2D-1 has a 6-membered ring structure, the compound of Chemical Formula 2D-1 may be represented by Chemical Formula 2D-11a.

[Chemical Formula 2D-11a]

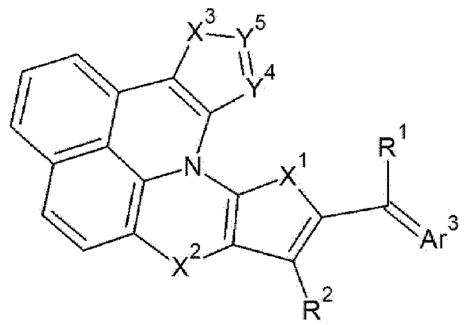

In Chemical Formula 2D-11a,

$X^1$, $X^2$, $Ar^3$, $R^1$, and $R^2$ are the same as $X^1$, $X^2$, $Ar^3$, $R^1$, and $R^2$, respectively, in Chemical Formula 1,

$X^3$ is the same as $X^3$ in Chemical Formula 2D,

$Y^4$ and $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2D-11a may be replaced by N, and one or more (e.g., 1, 2, or 3) N may be included in one 6-member ring structure.

When Cy of Chemical Formula 2D-2 has a 6-membered ring structure, the compound of Chemical Formula 2D-2 may be represented by Chemical Formula 2D-21a.

[Chemical Formula 2D-21a]

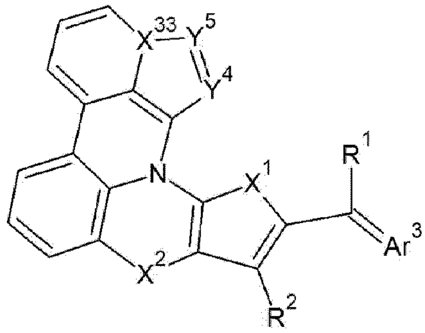

In Chemical Formula 2D-21a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^4$ and $Y^5$ are the same as $Y^4$ and $Y^5$, respectively, in Chemical Formula 2D,
$X^{33}$ may be N, B, $SiR^b$, $GeR^d$, or $CR^f$, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and
hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2D-21a may be replaced by N, and one or more (e.g., 1, 2, or 3) N may be included in one 6-member ring structure.

In some example embodiments, in Chemical Formula 2D, $Y^1$ ($CR^k$) and carbon (C) may be linked to each other to provide a first fused ring and $X^3$ ($-NR^{a1}-$, $-BR^{a2}-$, $-SiR^bR^c-$, $-GeR^dR^e-$, or $-CR^fR^g-$) and carbon (C) may be linked to each other to provide a second fused ring. When the first fused ring and the second fused ring each have a 6-membered ring structure, it may be represented by Chemical Formula 2D-3a.

[Chemical Formula 2D-3a]

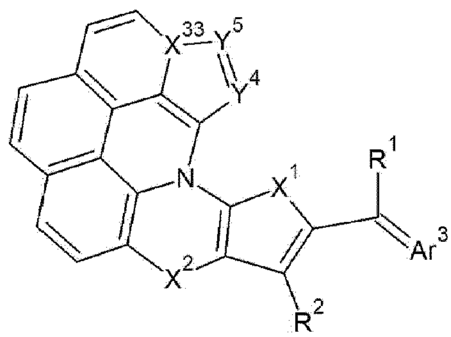

In Chemical Formula 2D-3a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^{33}$ may be N, B, $SiR^b$, $GeR^d$, or $CR^f$, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and
$Y^4$ and $Y^5$ may each independently be N or $CR^k$, wherein $R^k$ may be hydrogen, deuterium, a halogen, a cyano

group, a nitro group, a hydroxyl group, an amine group, a substituted or unsubstituted C1 to C10 alkyl group, or a substituted or unsubstituted C1 to C10 alkoxy group, or adjacent $R^{k}$'s may be linked to each other to provide a substituted or unsubstituted C6 to C30 arene group, a substituted or unsubstituted C3 to C30 heteroarene group, or a condensed ring thereof.

In some example embodiments, CH of a 6-membered ring structure (for example, a benzene ring and/or a cyclohexadiene ring) in Chemical Formula 2D-3a may be replaced by N, and one or more (e.g., 1, 2, or 3) N may be included in one 6-member ring structure.

In Chemical Formula 2D, when $X^3$ and $Y^5$ or $Y^4$ and $Y^5$ are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2D-41 to Chemical Formula 2D-46.

[Chemical Formula 2D-41]

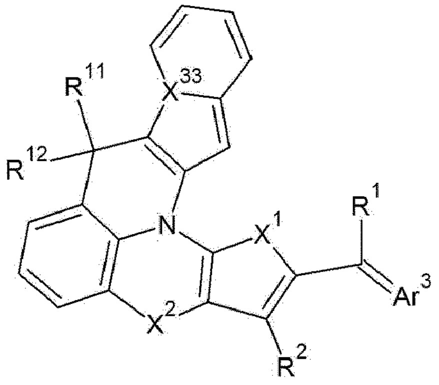

[Chemical Formula 2D-42]

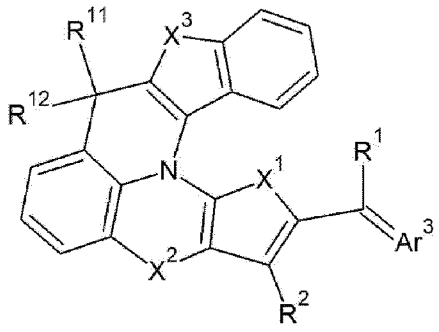

[Chemical Formula 2D-43]

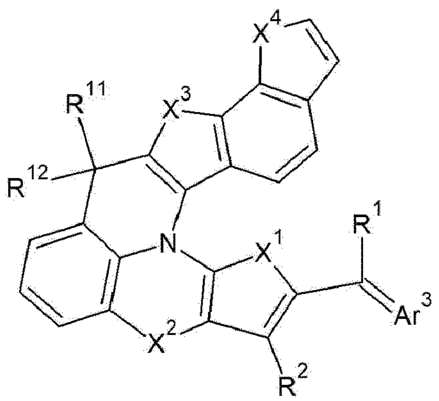

[Chemical Formula 2D-44]

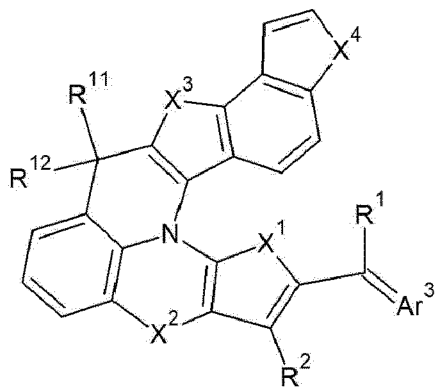

[Chemical Formula 2D-45]

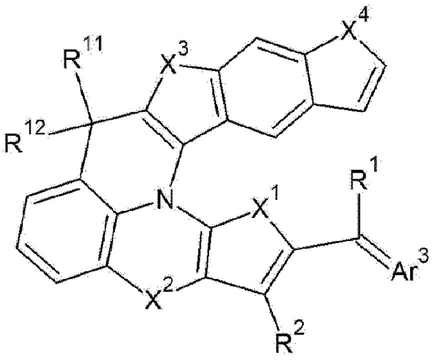

[Chemical Formula 2D-46]

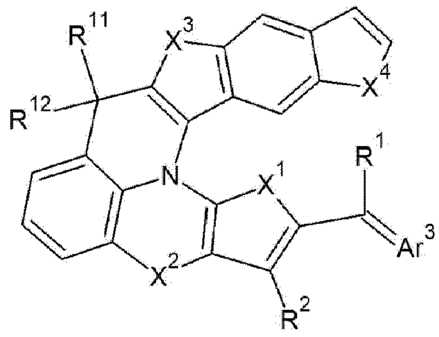

In Chemical Formula 2D-41 to Chemical Formula 2D-46,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, $X^{33}$ may be N, B, $SiR^b$, $GeR^d$, or $CR^f$, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,

$X^3$ may be the same as $X^3$ in Chemical Formula 2D,

$X^4$ may be -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, $-CR^{ff}R^{gg}-$, $-CR^h=CR^i-$, or $-CR^{hh}=CR^{ii}-$, wherein $R^{a1}$, $R^{a2}$, $R^b$, $R^c$, $R^d$, $R^e$, $R^f$, $R^9$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, $R^{gg}$ $R^{hh}$, and $R^{ii}$ may each independently be $(CH_2)t$, wherein t is a positive integer (e.g., any integer having a value of 1 or greater, 1 to 10, 1 to 8, or 1 to 6) or a heteroatom of O, $NR^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{hh}$ and $R^{ii}$ may be linked to each other to provide a ring structure, and

hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2D-41 to Chemical Formula 2D-46, CH of the aromatic ring may be replaced by N and one or more (e.g., 1, 2, or 3) N may be included in one aromatic ring.

In Chemical Formula 2D, when adjacent two of $Y^1$ to $Y^3$ are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2D-47 to Chemical Formula 2D-50.

[Chemical Formula 2D-47]

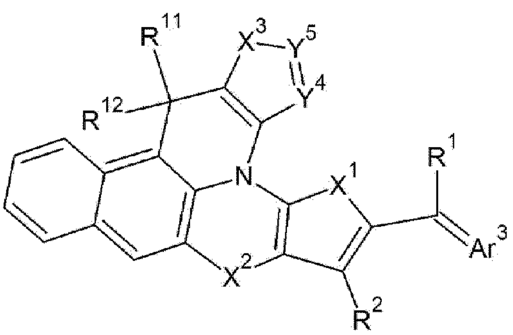

[Chemical Formula 2D-48]

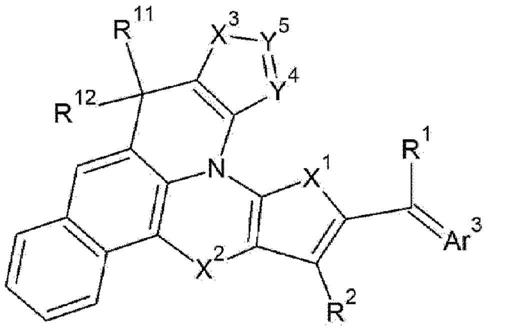

[Chemical Formula 2D-49]

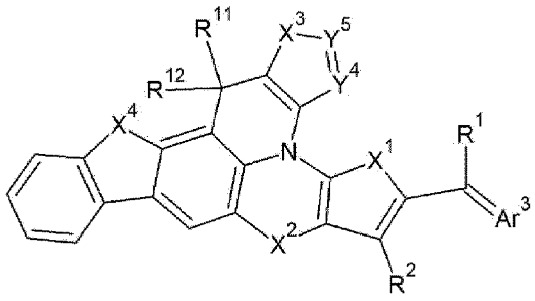

[Chemical Formula 2D-50]

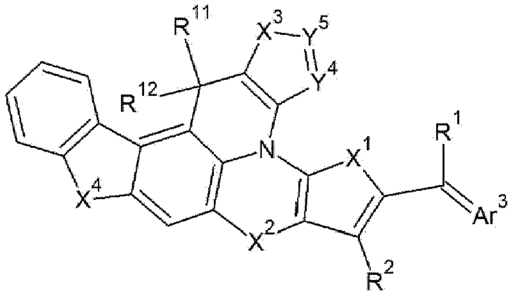

In Chemical Formula 2D-47 to Chemical Formula 2C-50,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$X^3$, $Y^4$, and $Y^5$ are the same as $X^3$, $Y^4$, and $Y^5$, respectively, in Chemical Formula 2D,

$X^4$ may be -O-, -S-, -Se-, -Te-, -S(=O)-, -S(=O)$_2$-, -NR$^{a1}$-, -BR$^{a2}$-, -SiR$^b$R$^c$-, - SiR$^{bb}$R$^{cc}$-, -GeR$^d$R$^e$-, -GeR$^{dd}$R$^{ee}$-, -CR$^f$R$^g$-, -CR$^{ff}$R$^{gg}$-, -CR$^h$=CR$^i$-, or -CR$^{hh}$=CR$^{ii}$-, wherein $R^{a1}$, $R^{a2}$, $R^b$, $R^c$, $R^d$, $R^e$, $R^f$, $R^9$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, $R^{gg}$ $R^{hh}$, and $R^{ii}$ may each independently be $(CH_2)t$, wherein t is a positive integer (e.g., any integer having a value of 1 or greater, 1 to 10, 1 to 8, or 1 to 6) or a heteroatom of O, $NR^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$, or $R^{hh}$ and $R^{ii}$ may be linked to each other to provide a ring structure, and

hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2D-47 to Chemical Formula 2D-50, CH of the aromatic ring may be replaced by N and one or more (e.g., 1, 2, or 3) N may be included in one aromatic ring.

In some example embodiments, the p-type semiconductor compound of Chemical Formula 1 may be represented by Chemical Formula 2E.

[Chemical Formula 2E]

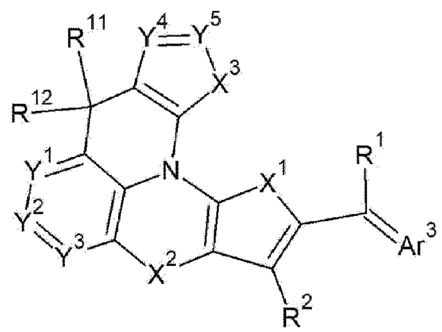

In Chemical Formula 2E,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$Y^1$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed

ring thereof, and

$X^3$ may be -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, and $R^{gg}$, may each independently be $(CH_2)_u$, wherein u is a positive integer (e.g., any integer having a value of 1 or greater, 1 to 10, 1 to 8, or 1 to 6) or a heteroatom of O, $NR^u$, wherein $R^u$ is hydrogen or a C1 to C10 alkyl group, S, Se, or Te, and/or at least one pair of $R^{bb}$ and $R^{cc}$, $R^{dd}$ and $R^{ee}$, or $R^{ff}$ and $R^{gg}$ may be linked to each other to provide a ring structure.

In some example embodiments, in Chemical Formula 2E, $X^3$ may be -O-, -S-, -Se-, -Te-, -S(=O)-, $-S(=O)_2-$, $-NR^{a1}-$, $-BR^{a2}-$, $-SiR^bR^c-$, $-GeR^dR^e-$, or $-CR^fR^g-$, wherein $R^{a1}$, $R^{a2}$, $R^b$, $R^c$, $R^d$, $R^e$, $R^f$, and $R^g$ may each independently be a halogen, a C1 to C20 haloalkyl group, or a C1 to C20 cyanoalkyl group. In this case, $X^3$, N, $X^1$, and functional groups (C=O, C=S, C=Se, or C=Te) of $Ar^3$ increase an intramolecular interaction, thereby increasing the absorption intensity at a specific wavelength.

In some example embodiments, in Chemical Formula 2E, $Y^3$ may be N or $CR^k$, wherein $R^k$ may be a halogen, a cyano group, a C1 to C10 haloalkyl group, or a C1 to C10 cyanoalkyl group and $X^2$ may be -O-, -S-, -Se-, -Te-, -S(=O)-, $-S(=O)_2-$, - $NR^{a1}-$, $-BR^{a2}-$, $-SiR^bR^c-$, $-GeR^dR^e-$, $-(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group, and n1 of - $(CR^fR^g)_{n1}$- may be 1 or 2. In this case, $Y^3$ and $X^2$ may increase an intramolecular interaction, thereby improving the absorption intensity at a specific wavelength.

In some example embodiments, in Chemical Formula 2E, $Y^1$ ($CR^k$) and carbon (C) may be linked to each other to provide a fused ring. This structure may be represented by Chemical Formula 2E-1.

[Chemical Formula 2E-1]

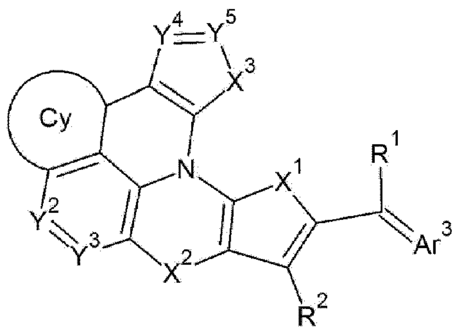

In Chemical Formula 2E-1,

$X^1$, $X^2$, $Ar^3$, $R^1$, and $R^2$ are the same as $X^1$, $X^2$, $Ar^3$, $R^1$, and $R^2$, respectively, in Chemical Formula 1,

$X^3$ is the same as $X^3$ in Chemical Formula 2E,

$Y^2$ to $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and

Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C3 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

In some example embodiments, in Chemical Formula 2E, $Y^4$ ($CR^k$) and carbon (C) may be linked to each other

to provide a fused ring. This structure may be represented by Chemical Formula 2E-2.

[Chemical Formula 2E-2]

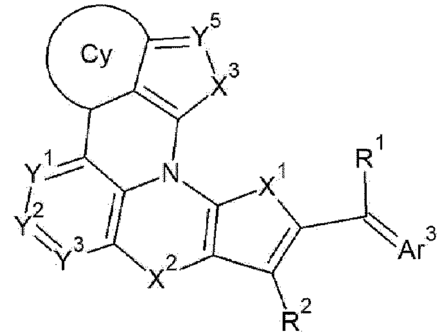

In Chemical Formula 2E-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2E,
$Y^1$ to $Y^3$ and $Y^5$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and
Cy may be a substituted or unsubstituted C6 to C30 arene group, for example a substituted or unsubstituted C6 to C20 arene group, or a substituted or unsubstituted C6 to C10 arene group; a substituted or unsubstituted C3 to C30 heteroarene group, for example a substituted or unsubstituted C3 to C20 heteroarene group, or a substituted or unsubstituted C3 to C10 heteroarene group; a substituted or unsubstituted C5 to C30 cycloalkene group, for example a substituted or unsubstituted C5 to C20 cycloalkene group, or a substituted or unsubstituted C5 to C10 cycloalkene group; a substituted or unsubstituted C3 to C30 heterocycloalkene group, for example a substituted or unsubstituted C5 to C30 heterocycloalkene group, a substituted or unsubstituted C5 to C20 heterocycloalkene group, or a substituted or unsubstituted C5 to C10 heterocycloalkene group; or a condensed ring thereof.

In some example embodiments, Cy of Chemical Formula 2E-1 and Chemical Formula 2E-2 may be an arene group, a heteroarene group, a cycloalkene group, or a heterocycloalkene group, and they may have a 5-membered to 10-membered ring structure. The heteroarene group or heterocycloalkene group may include N in the ring.

When Cy of Chemical Formula 2E-1 has a 6-membered ring structure, the compound of Chemical Formula 2E-1 may be represented by Chemical Formula 2E-11a.

[Chemical Formula 2E-11a]

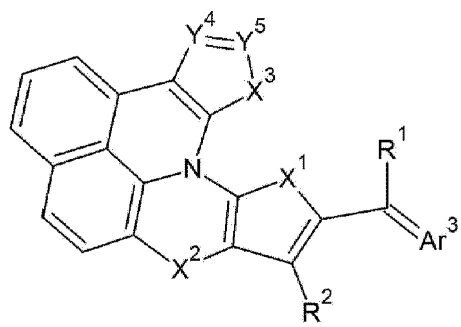

In Chemical Formula 2E-11a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$, $Y^4$, and $Y^5$ are the same as $X^3$, $Y^4$, and $Y^5$, respectively, in Chemical Formula 2E,

hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2E-11a may be replaced by N, and one or more (e.g., 1, 2, or 3) N may be included in one 6-member ring structure.

When Cy of Chemical Formula 2E-2 has a 6-membered ring structure, the compound of Chemical Formula 2E-2 may be represented by Chemical Formula 2E-21a.

[Chemical Formula 2E-21a]

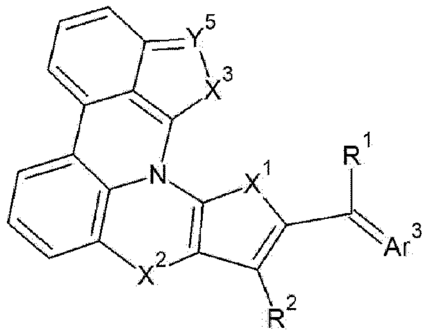

In Chemical Formula 2E-21a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ and $Y^5$ are the same as $X^3$ and $Y^5$, respectively, in Chemical Formula 2E, and
hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2E-21a may be replaced by N, and one or more (e.g., 1, 2, or 3) N may be included in one 6-member ring structure.

In some example embodiments, in Chemical Formula 2E, $Y^1$ ($CR^k$) and carbon (C) may be linked to each other to provide a first fused ring and $Y^4$ ($CR^k$) and carbon (C) may be linked to each other to provide a second fused ring. When the first fused ring and the second fused ring each have a 6-membered ring structure, it may be represented by Chemical Formula 2E-3a.

[Chemical Formula 2E-3a]

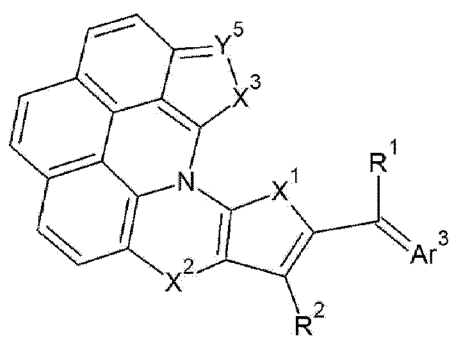

In Chemical Formula 2E-3a,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2E,
$Y^5$ may be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s may be linked to each other to provide a substituted or unsubstituted C6 to C30 arene group, a substituted or unsubstituted C3 to C30 heteroarene group, or a condensed ring thereof, and hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, CH of a 6-membered ring structure (for example, a benzene ring and/or a cyclohexadiene ring) in Chemical Formula 2E-3a may be replaced by N, and one or more (e.g., 1, 2, or 3) N may be included in one 6-member ring structure.

In some example embodiments, in Chemical Formula 2E, when $Y^4$ and $Y^5$ or $Y^5$ and $X^3$ are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2E-41 to Chemical Formula 2E-46.

[Chemical Formula 2E-41]

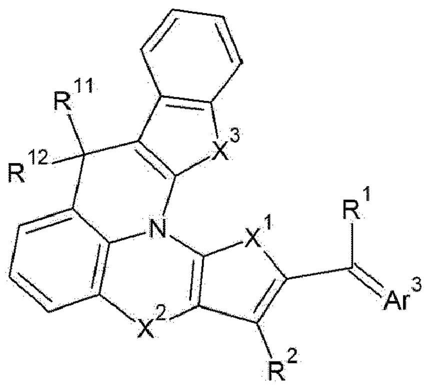

[Chemical Formula 2E-42]

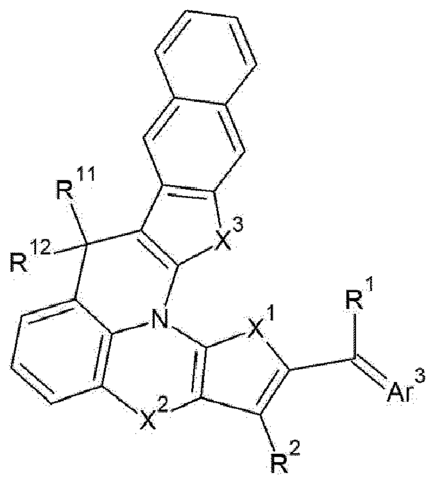

[Chemical Formula 2E-43]

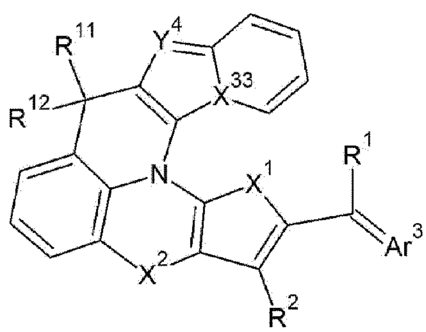

[Chemical Formula 2E-44]

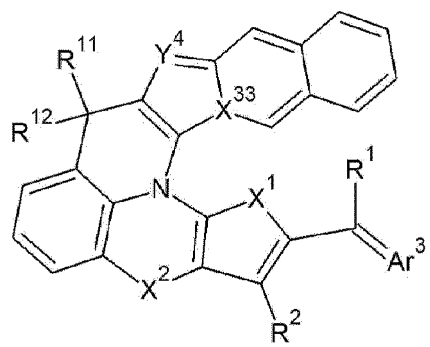

[Chemical Formula 2E-45]

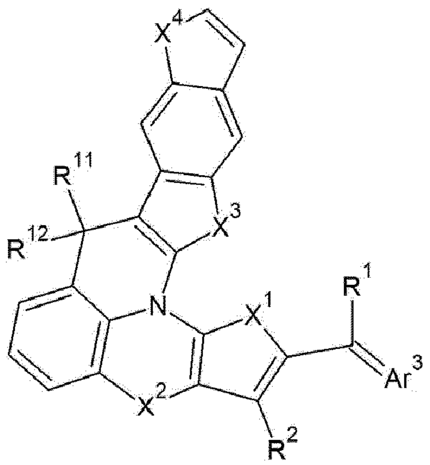

[Chemical Formula 2E-46]

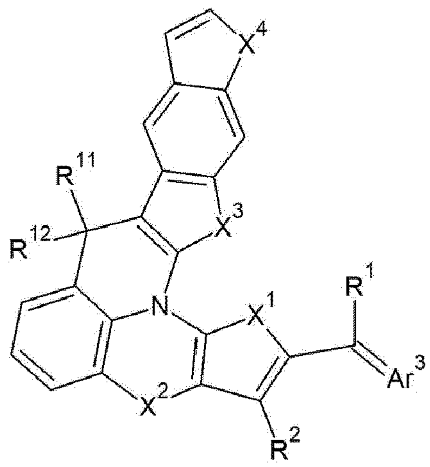

In Chemical Formula 2E-41 to Chemical Formula 2E-46,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$X^{33}$ may be N, B, $SiR^b$, $GeR^d$, or $CR^f$, wherein $R^b$, $R^d$, and $R^f$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,

$X^3$ and $Y^4$ are the same as $X^3$ and $Y^4$, respectively, in Chemical Formula 2E,

$X^4$ may be -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$, $-CR^{ff}R^{gg}-$, $-CR^h=CR^i-$, or $-CR^{hh}=CR^{ii}-$, wherein $R^{a1}$, $R^{a2}$, $R^b$, $R^c$, $R^d$, $R^e$, $R^f$, $R^g$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, $R^{gg}$ $R^{hh}$, and $R^{ii}$ may each independently be $(CH_2)t$, wherein t is a positive integer (e.g., any integer having a value of 1 or greater, 1 to 10, 1 to 8, or 1 to 6) or a heteroatom of O, $NR^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$ or $R^{hh}$ and $R^{ii}$ is linked to each other to provide a ring structure, and

hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2E-41 to Chemical Formula 2E-46, CH of the aromatic ring may be replaced by N and one or more (e.g., 1, 2, or 3) N may be included in one aromatic ring.

In some example embodiments, in Chemical Formula 2E, when $Y^1$ to $Y^3$ are $CR^k$ and adjacent $R^k$'s are linked to each other to provide a fused ring (a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or any combination thereof), it may be represented by one of Chemical Formula 2E-47 to Chemical Formula 2E-50.

[Chemical Formula 2E-47]

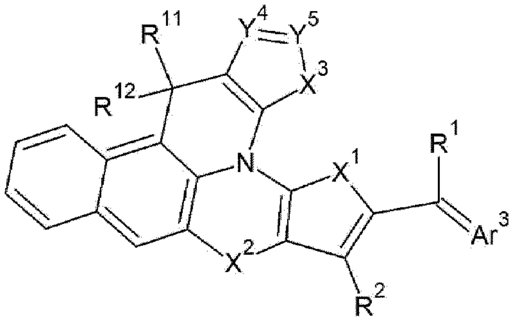

[Chemical Formula 2E-48]

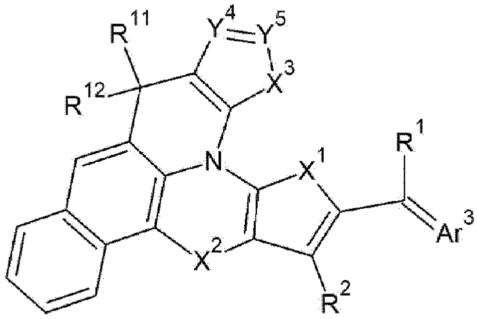

[Chemical Formula 2E-49]

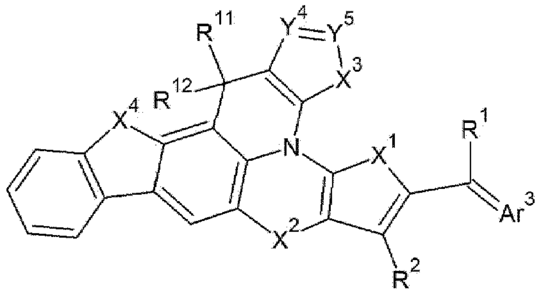

[Chemical Formula 2E-50]

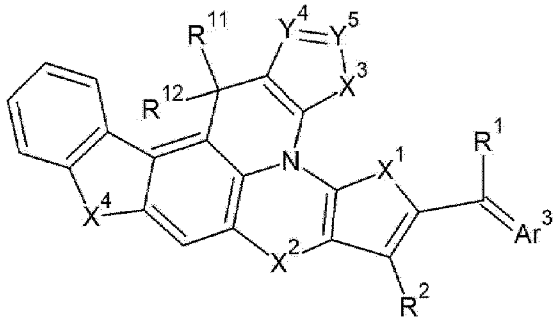

In Chemical Formula 2E-47 to Chemical Formula 2E-50,

L may $-CR^{11}R^{12}-$ of Chemical Formula 1,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$X^3$, $Y^4$, and $Y^5$ are the same as $X^3$, $Y^4$, and $Y^5$, respectively, in Chemical Formula 2E,

$X^4$ may be -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, $-CR^{ff}R^{gg}-$, $-CR^h=CR^i-$, or $-CR^{hh}=CR^{ii}-$, wherein $R^{a1}$, $R^{a2}$, $R^b$, $R^c$, $R^d$, $R^e$, $R^f$, $R^g$, $R^h$, and $R^i$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and $R^{bb}$, $R^{cc}$, $R^{dd}$, $R^{ee}$, $R^{ff}$, $R^{gg}$, $R^{hh}$, and $R^{ii}$ may each independently be $(CH_2)t$, wherein t is a positive integer (e.g., any integer having a value of 1 or greater, 1 to 10, 1 to 8, or 1 to 6) or a heteroatom of O, $NR^t$, wherein $R^t$ is hydrogen or a C1 to C10 alkyl group, S, Se, or Te, and/or at least one pair of $R^{bb}$ and $R^{cc}$, $R^{dd}$ and $R^{ee}$, $R^{ff}$ and $R^{gg}$ or $R^{hh}$ and $R^{ii}$ is linked to each other to provide a ring structure, and

hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In some example embodiments, in Chemical Formula 2E-47 to Chemical Formula 2E-50, CH of the aromatic ring may be replaced by N and one or more (e.g., 1, 2, or 3) N may be included in one aromatic ring.

In $X^1$, $X^2$, and $-CR^{11}R^{12}-$ of Chemical Formula 1 and $X^3$ of Chemical Formula 2B, Chemical Formula 2C, Chemical Formula 2D, and Chemical Formula 2E, the ring structures thereof may be a spiro structure or a fused ring structure. Restated, each ring structure of the ring structure of $X^1$, the ring structure of $X^2$, and the ring structure of $-CR^{11}R^{12}-$ may be a spiro structure or a fused ring structure. The spiro structure may be a substituted or unsubstituted C5 to C30 hydrocarbon cyclic group, a substituted or unsubstituted C2 to C30 heterocyclic group, or a fused ring thereof. The substituted or unsubstituted C5 to C30 hydrocarbon cyclic group may be for example a substituted or unsubstituted C5 to C30 cycloalkyl group (e.g., a substituted or unsubstituted C5 to C20 cycloalkyl group, or a substituted or unsubstituted C5 to C10 cycloalkyl group). Examples of the fused ring include a fluorenyl group and an indanyl group. The substituted or unsubstituted C2 to C30 heterocyclic group may be for example a substituted or unsubstituted C2 to C30

heterocycloalkyl group (e.g., a substituted or unsubstituted C2 to C20 heterocycloalkyl group or a substituted or unsubstituted C2 to C10 heterocycloalkyl group).

The fused ring structure may have a fused substituted or unsubstituted C5 to C30 hydrocarbon cyclic group, a fused substituted or unsubstituted C2 to C30 heterocyclic group, or a fused ring thereof. The substituted or unsubstituted C5 to C30 hydrocarbon cyclic group may be for example a substituted or unsubstituted C5 to C30 cycloalkyl group (e.g., a substituted or unsubstituted C5 to C20 cycloalkyl group, or a substituted or unsubstituted C5 to C10 cycloalkyl group) or a substituted or unsubstituted C6 to C30 aryl group (e.g., a substituted or unsubstituted C6 to C20 aryl group or a substituted or unsubstituted C6 to C10 aryl group) and the substituted or unsubstituted C2 to C30 heterocyclic group may be for example a substituted or unsubstituted C2 to C30 heterocycloalkyl group (e.g., a substituted or unsubstituted C2 to C20 heterocycloalkyl group or a substituted or unsubstituted C2 to C10 heterocycloalkyl group) or a substituted or unsubstituted C2 to C30 heteroaryl group (e.g., a substituted or unsubstituted C2 to C20 heteroaryl group, or a substituted or unsubstituted C2 to C10 heteroaryl group).

The spiro structure may include a moiety represented by Chemical Formula 3.

[Chemical Formula 3]

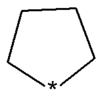
(1)

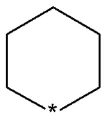
(2)

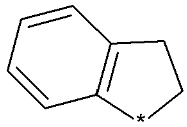
(3)

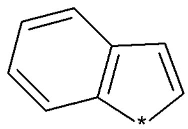
(4)

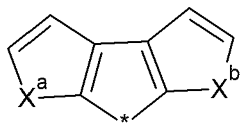
(5)

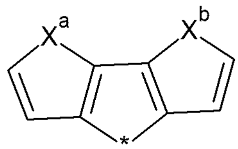
(6)

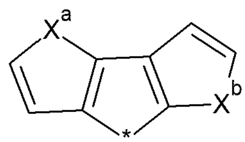
(7)

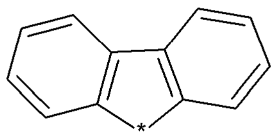
(8)

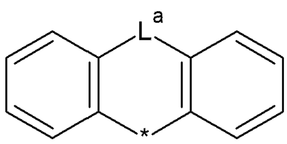
(9)

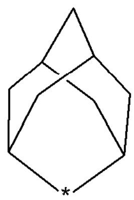
(10)

In Chemical Formula 3,

$X^a$ and $X^b$ may each independently be -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, or $-GeR^{dd}R^{ee}-$, wherein $R^{a1}$, $R^{a2}$, $R^b$, $R^c$, $R^d$, and $R^e$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$ or $R^{dd}$ and $R^{ee}$ may be linked to each other to provide a ring structure,

$L^a$ may be -O-, -S-, -Se-, -Te-, $-NR^{a1}-$, $-BR^{a2}-$, $-SiR^bR^c-$, $-GeR^dR^e-$, $-(CR^fR^g)_{n1}-$, $-(C(R^p)=N)-$, or a single bond, wherein $R^{a1}$, $R^{a2}$, $R^b$, $R^c$, $R^d$, $R^e$, $R^f$, $R^g$, and $R^p$ may each independently be hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group and n1 of $-(CR^fR^g)_{n1}-$ may be 1 or 2, and

at least one hydrogen (e.g., some or all hydrogens) of each ring of one or more of the moieties of Chemical Formula

3 may optionally be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In Chemical Formula 3, one or more CH present in the aromatic ring of the moieties (3), (4), (5), (6), (7), (8), or (9) may be replaced by N.

In Chemical Formula 1, $Ar^3$ may be represented by Chemical Formula 4.

[Chemical Formula 4]

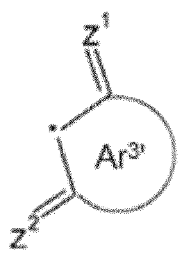

In Chemical Formula 4,

$Ar^{3'}$ may be a substituted or unsubstituted C6 to C30 aryl group or a substituted or unsubstituted C3 to C30 heteroaryl group,
$Z^1$ may be O, S, Se, or Te, and
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group.

In Chemical Formula 1, $Ar^3$ may be a cyclic group represented by one of Chemical Formula 5A to Chemical Formula 5G.

[Chemical Formula 5A]

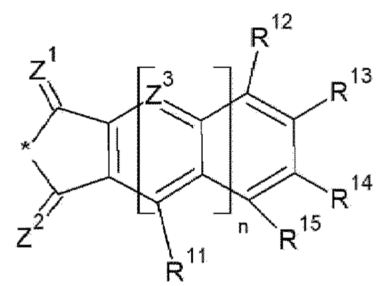

In Chemical Formula 5A,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ may be N or $CR^c$, wherein $R^c$ may be hydrogen, deuterium or a substituted or unsubstituted C1 to C10 alkyl group,
$R^{11}$, $R^{12}$, $R^{13}$, $R^{14}$, and $R^{15}$ may each independently be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ may each independently be present or may be linked to each other to provide a fused aromatic ring,
n may be 0 or 1, and

* may be a linking point.

In some example embodiments, in Chemical Formula 5A, at least one of $CR^{11}$, $CR^{12}$, $CR^{13}$, $CR^{14}$, or $CR^{15}$ may be replaced by nitrogen (N). That is, the substituted or unsubstituted benzene ring moiety of Chemical Formula 5A may include a hetero atom (N).

[Chemical Formula 5B]

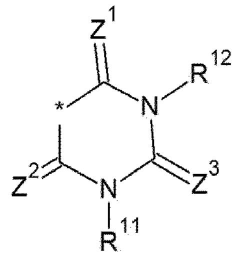

In Chemical Formula 5B,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ may be O, S, Se, Te, or $C(R^a)(CN)$, wherein $R^a$ is hydrogen, a cyano group (-CN), or a C1 to C10 alkyl group,
$R^{11}$ and $R^{12}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* may be a linking point.

[Chemical Formula 5C]

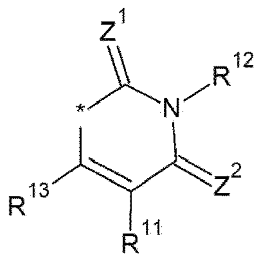

In Chemical Formula 5C,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* may be a linking point.

[Chemical Formula 5D]

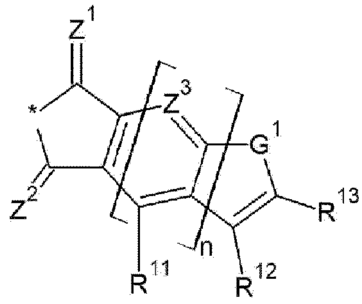

In Chemical Formula 5D,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ may be N or $CR^c$, wherein $R^c$ may be hydrogen or a substituted or unsubstituted C1 to C10 alkyl group,
$G^1$ may be O, S, Se, Te, $SiR^xR^y$, or $GeR^zR^w$, wherein $R^x$, $R^y$, $R^z$, and $R^w$ may each independently be hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, wherein $R^{12}$ and $R^{13}$ may each independently be present or may be linked to each other to provide a fused aromatic ring,
n may be 0 or 1, and
* may be a linking point.

[Chemical Formula 5E]

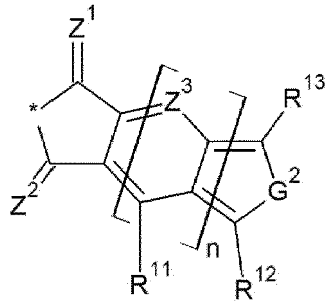

In Chemical Formula 5E,

$Z^1$ may be O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ may be N or $CR^c$, wherein $R^c$ may be hydrogen or a substituted or unsubstituted C1 to C10 alkyl group,
$G^2$ may be O, S, Se, Te, $SiR^xR^y$, or $GeR^zR^w$, wherein $R^x$, $R^y$, $R^z$, and $R^w$ may each independently be hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or

any combination thereof,
n may be 0 or 1, and
* may be a linking point.

[Chemical Formula 5F]

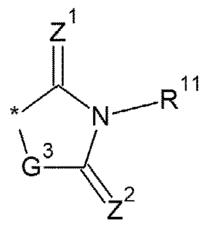

In Chemical Formula 5F,

$Z^1$ is O, S, Se, or Te,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$R^{11}$ may be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, and
$G^3$ may be O, S, Se, Te, $SiR^xR^y$, or $GeR^zR^w$, wherein $R^x$, $R^y$, $R^z$, and $R^w$ may each independently be hydrogen, deuterium, a halogen, a substituted or unsubstituted C1 to C20 alkyl group, or a substituted or unsubstituted C6 to C20 aryl group.

[Chemical Formula 5G]

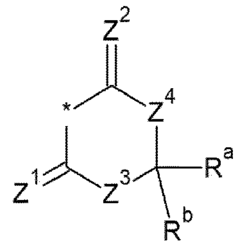

In Chemical Formula 5G,

$Z^1$ may be O, S, Se, or Te, and
$R^a$ and $R^b$ may each independently be hydrogen, a substituted or unsubstituted C1 to C10 alkyl group, a cyano group, or a cyano-containing group, and
$Z^2$ to $Z^4$ may each independently be O, S, Se, Te, or $CR^cR^d$, wherein $R^c$ and $R^d$ are each independently hydrogen, a substituted or unsubstituted C1 to C10 alkyl group, a cyano group, or a cyano-containing group, provided that when $Z^2$ is $CR^cR^d$, at least one of $R^c$ or $R^d$ is a cyano group or a cyano-containing group.

The cyclic group represented by Chemical Formula 5A may be a cyclic group represented by Chemical Formula 5A-1 or Chemical Formula 5A-2.

[Chemical Formula 5A-1]

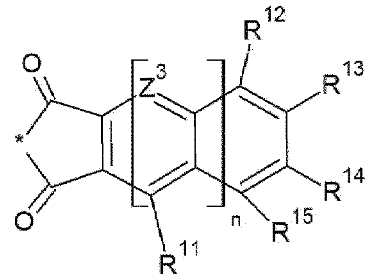

[Chemical Formula 5A-2]

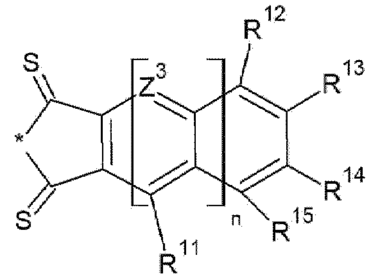

In Chemical Formula 5A-1 and Chemical Formula 5A-2,
$Z^3$, $R^{11}$, n, $R^{11}$, $R^{12}$, $R^{13}$, $R^{14}$, and $R^{15}$ are the same as $Z^3$, $R^{11}$, n, $R^{11}$, $R^{12}$, $R^{13}$, $R^{14}$, and $R^{15}$, respectively, in Chemical Formula 5A.

The cyclic group represented by Chemical Formula 5A may be a cyclic group represented by Chemical Formula 5A-3 when $R^{12}$ and $R^{13}$ and/or $R^{14}$ and $R^{15}$ may each independently be linked to form a fused aromatic ring.

[Chemical Formula 5A-3]

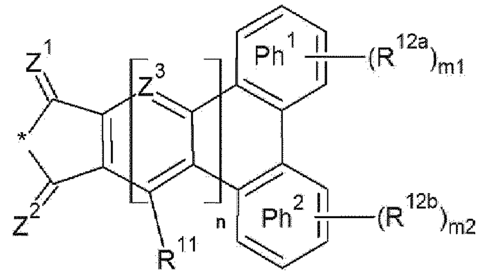

In Chemical Formula 5A-3,

$Z^1$, $Z^2$, $Z^3$, $R^{11}$, and n are the same as $Z^1$, $Z^2$, $Z^3$, $R^{11}$, and n, respectively, in Chemical Formula 5A,
$R^{12a}$ and $R^{12b}$ may each independently be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof,
m1 and m2 may each independently be an integer ranging from 0 to 4, and
$Ph^1$ and $Ph^2$ refer to a fused phenylene ring, and one of $Ph^1$ or $Ph^2$ may be optionally omitted.

The cyclic group represented by Chemical Formula 5B may be, for example, a cyclic group represented by Chemical Formula 5B-1, 5B-2, or 5B-3.

[Chemical Formula 5B-1] [Chemical Formula 5B-2] [Chemical Formula 5B-3]

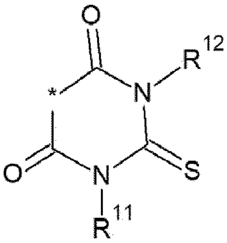
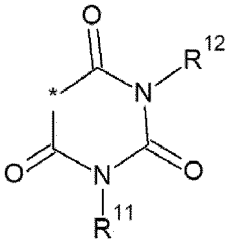
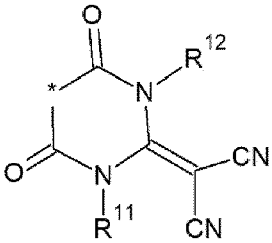

In Chemical Formulas 5B-1, 5B-2, and 5B-3,
$R^{11}$ and $R^{12}$ are the same as $R^{11}$ and $R^{12}$, respectively, in Chemical Formula 5B.

The cyclic group represented by Chemical Formula 5C may be, for example, a cyclic group represented by Chemical Formula 5C-1 or 5C-3.

[Chemical Formula 5C-1]

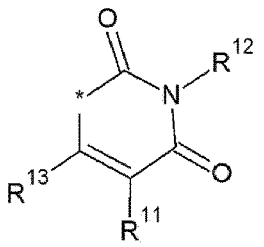

[Chemical Formula 5C-2]

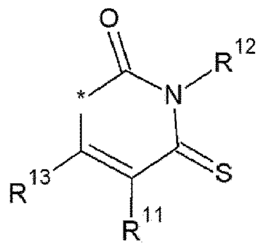

In Chemical Formulas 5C-1 and 5C-2,
$R^{11}$ to $R^{13}$ are the same as $R^{11}$ to $R^{13}$, respectively, in Chemical Formula 5C.

In Chemical Formula 1, N of the electron donor moiety, $X^1$ of the $X^1$-containing ring, the functional groups (C=O, C=S, C=Se, or C=Te) of $Ar^3$, which is the electron acceptor moiety increase intramolecular interactions to improve the absorption intensity at a specific wavelength.

Specific examples of the compound represented by Chemical Formula 2A may include compounds of Group 1, but are not limited thereto.

[Group 1]

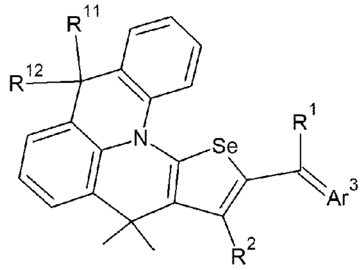
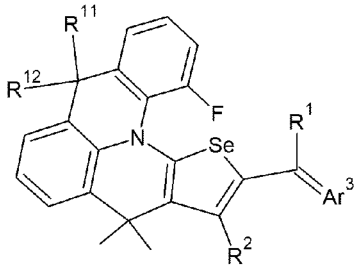
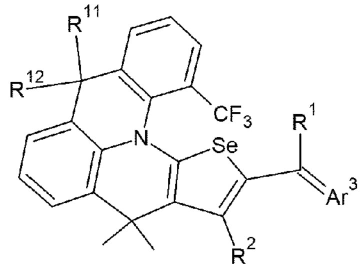

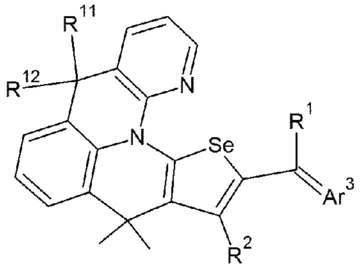
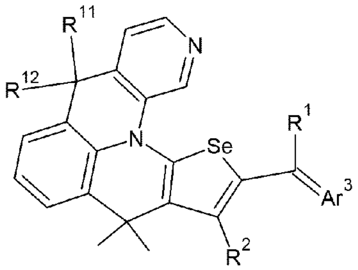
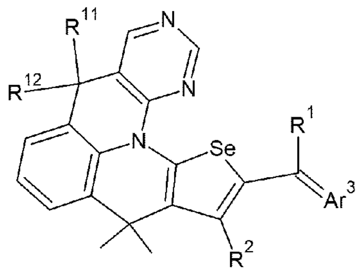

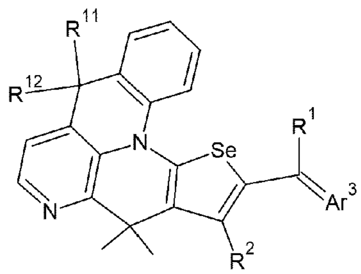
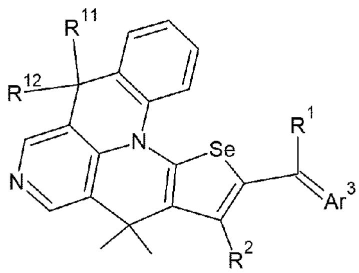
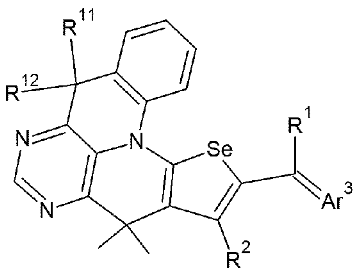

In Group 1, $Ar^3$, $R^1$, $R^2$, $R^{11}$, and $R^{12}$ are the same as $Ar^3$, $R^1$, $R^2$, $R^{11}$, and $R^{12}$, respectively, in Chemical Formula 1, and

at least one hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In Group 1, compounds in which $X^1$ is -Se- in Chemical Formula 2A are shown, but the -Se- may be replaced by another linking group of $X^1$ (-S-, -Te-, - S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$, or $-CR^{ff}R^{gg}-$).

In Group 1, compounds in which $X^2$ is $-(C(CH_3)(CH_3))-$ in Chemical Formula 2A are shown, but the $-(C(CH_3)(CH_3))$ may be replaced by another linking group of $X^2$ (-O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, - $GeR^dR^e-$, $-GeR^{dd}R^{ee}-$, $-(CR^fR^g)_{n1}-$, $-(CR^{ff}R^{gg})-$, $-(C(R^m)=C(R^n))-$, $-(C(R^{mm})=C(R^{nn}))-$, or $-(C(R^p)=N)-$).

Specific examples of the compound represented by Chemical Formula 2A-1 or Chemical Formula 2A-2 may include compounds of Group 1-1, but are not limited thereto.

[Group 1-1]

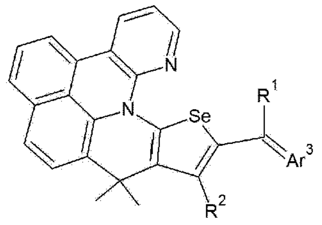
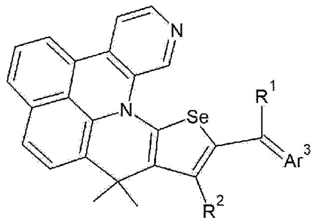
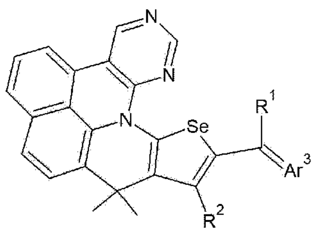
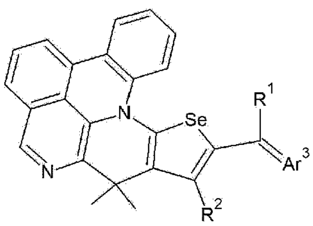
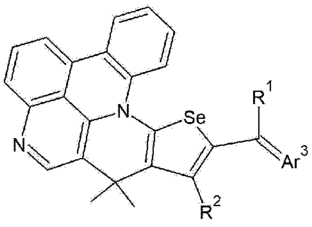
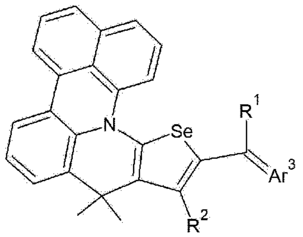
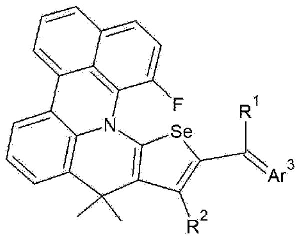
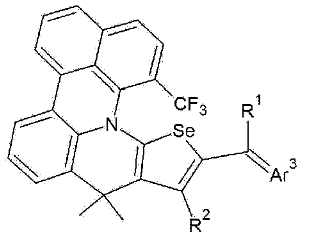
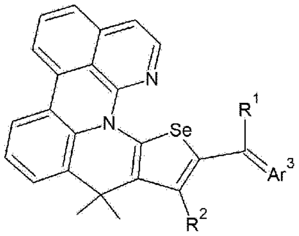
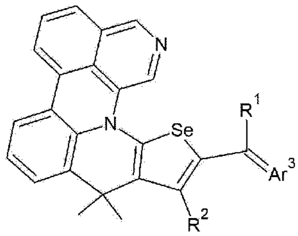
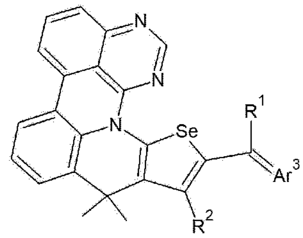
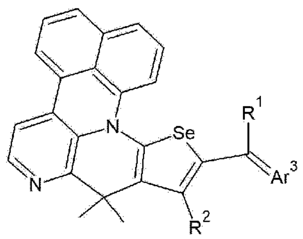
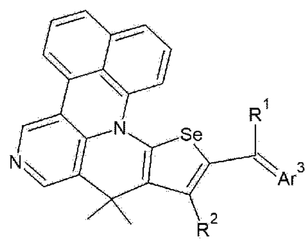
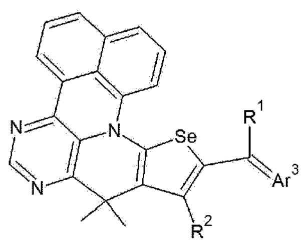

In Group 1-1,

$Ar^3$, $R^1$, and $R^2$ are the same as $Ar^3$, $R^1$, and $R^2$, respectively, in Chemical Formula 1, and at least one hydrogen of each ring (benzene ring or cyclohexadiene ring) may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

Structures in which CH present in the benzene ring in Group 1-1 is replaced by one N are illustrated, but the

CH of each ring (benzene ring or cyclohexadiene) of Group 1-1 may be replaced by N, wherein one ring may include one or more N, and a plurality of rings may contain N.

In Group 1-1, compounds in which $X^1$ is -Se- in Chemical Formula 2A-1 or Chemical Formula 2A-2 are shown, but the -Se- may be replaced by another linking group of $X^1$ (-S-, -Te-, -S(=O)-, $-S(=O)_2-$, $-NR^{a1}-$, $-BR^{a2}-$, $-SiR^bR^c-$, $-SiR^{bb}R^{cc}-$, - $GeR^dR^e-$, $-GeR^{dd}R^{ee}-$, $-CR^fR^g-$, or $-CR^{ff}R^{gg}-$).

In Group 1-1, compounds in which $X^2$ is$-(C(CH_3)(CH_3))-$ in Chemical Formula 2A-1 and Chemical Formula 2A-2 are shown, but the $-(C(CH_3)(CH_3))$ may be replaced by another linking group of $X^2$ (-O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-(CR^fR^g)_{n1}-$, $-(CR^{ff}R^{gg})-$, - $(C(R^m)=C(R^n))-$, $-(C(R^{mm})=C(R^{nn}))-$, or $-(C(R^p)=N)-$).

Group 1 and Group 1-1 may be examples of the compound represented by Chemical Formula 2A, but examples of compounds represented by Chemical Formula 2B to Chemical Formula 2E may also be provided by the same manner.

For example, a specific example of the compound represented by Chemical Formula 2E may be provided by the compound of Group 2, but is not limited thereto.

[Group 2]

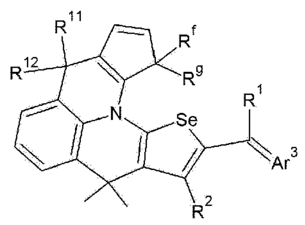
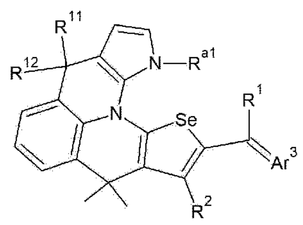
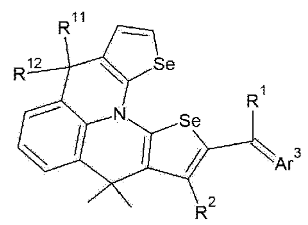
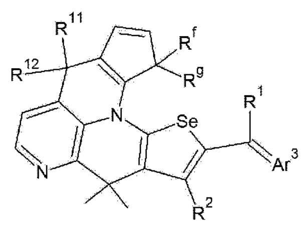
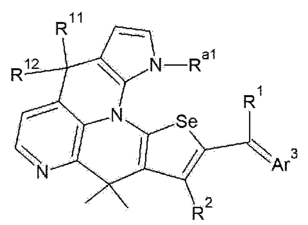
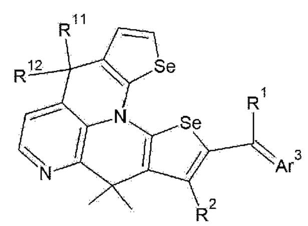
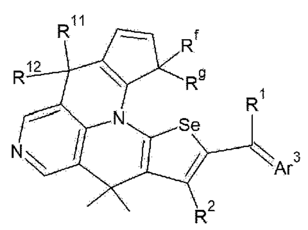
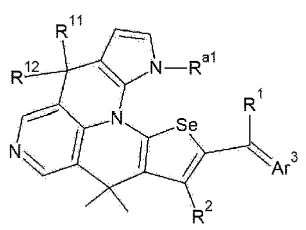
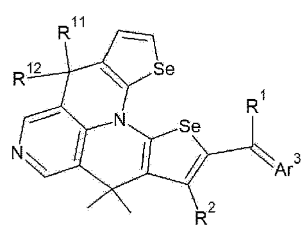
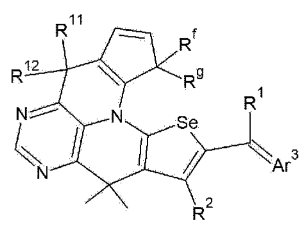
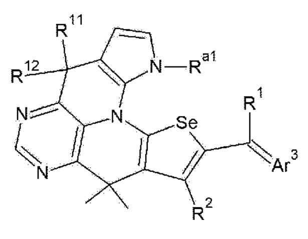
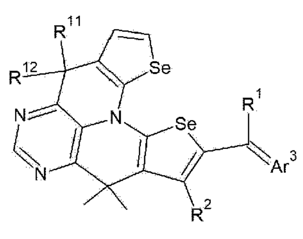

In Group 2,

$Ar^3$, $R^{11}$, $R^{12}$, $R^1$, and $R^2$ are the same as $Ar^3$, $R^{11}$, $R^{12}$, $R^1$, and $R^2$, respectively, in Chemical Formula 1, and $X^3$ is the same as in Chemical Formula 2E

$R^{a1}$, $R^f$, and $R^g$ may each independently be hydrogen, deuterium, a halogen, a cyano group, a substituted or unsubstituted C1 to C20 alkyl group, a substituted or unsubstituted C6 to C20 aryl group, or a substituted or unsubstituted C6 to C20 aryloxy group, and

at least one hydrogen of each aromatic ring may be replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In Group 2, compounds in which $X^1$ is -Se- in Chemical Formula 2E are shown, the -Se- may be replaced by another linking group of $X^1$ (-S-, -Te-, -S(=O)-, - $S(=O)_2$-, -$NR^{a1}$-, -$BR^{a2}$-, -$SiR^bR^c$-, -$SiR^{bb}R^{cc}$-, -$GeR^dR^e$-, -$GeR^{dd}R^{ee}$-, -$CR^fR^g$-, or - $CR^{ff}R^{gg}$-).

In Group 2, compounds in which $X^2$ is-($C(CH_3)(CH_3)$)- in Chemical Formula 2E are shown, but the -($C(CH_3)(CH_3)$) may be replaced by another linking group of $X^2$ (-O-, -S-, -Se-, -Te-, -S(=O)-, -$S(=O)_2$-, -$NR^{a1}$-, -$BR^{a2}$-, -$SiR^bR^c$-, -$SiR^{bb}R^{cc}$-, - $GeR^dR^e$-, -$GeR^{dd}R^{ee}$-, -$(CR^fR^g)_{n1}$-, -$(CR^{ff}R^{gg})$-, -($C(R^m)=C(R^n)$)-, -($C(R^{mm})=C(R^{nn})$)-, or -($C(R^p)=N$)-).

Specific examples of the compound represented by Chemical Formula 1 may include compounds of Group 3.

[Group 3]

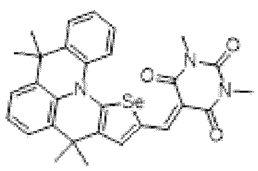 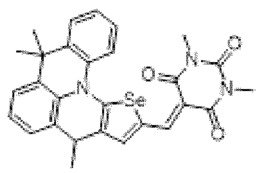 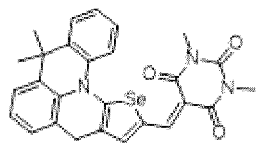 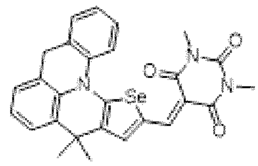

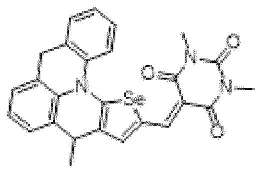 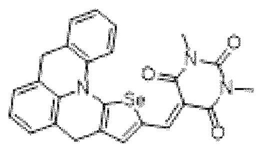 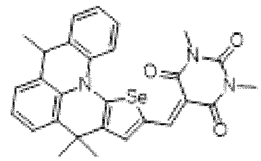 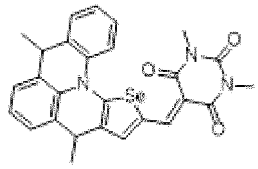

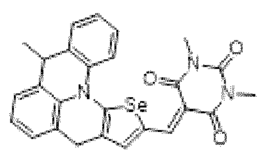 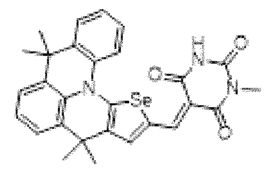 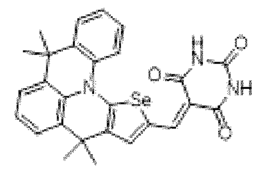 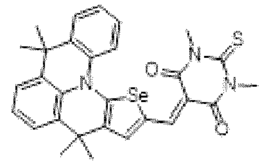

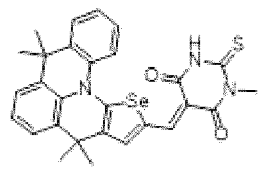 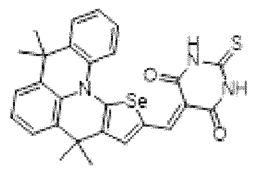 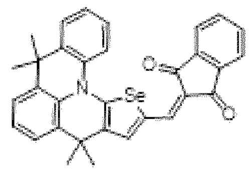 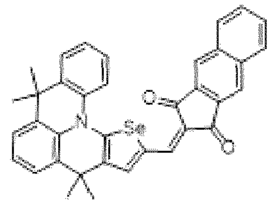

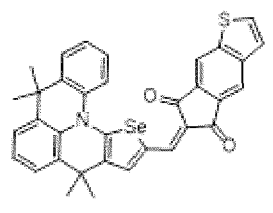 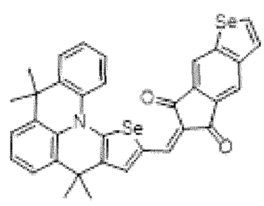 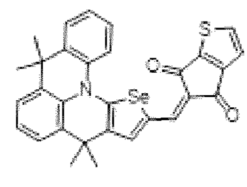 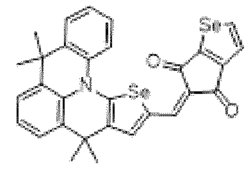

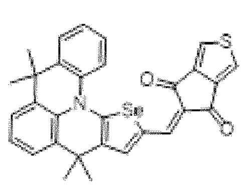 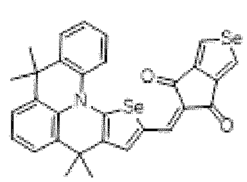 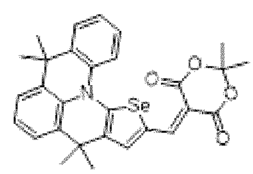 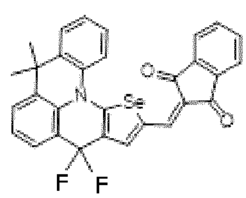

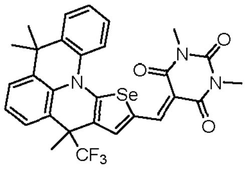
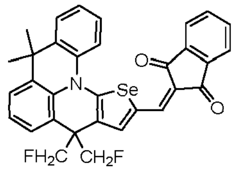
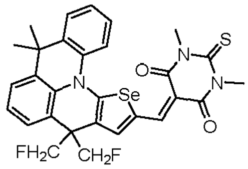
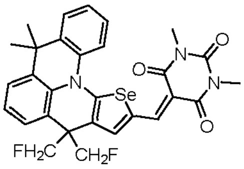

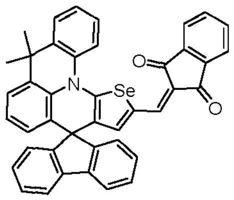
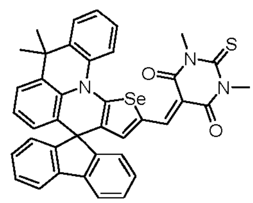
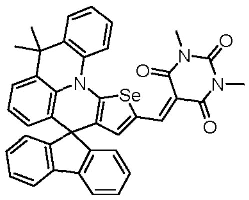
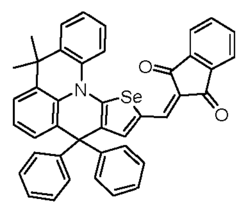

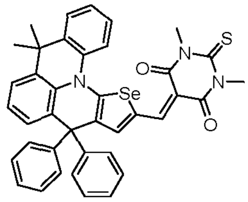
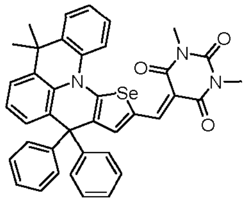
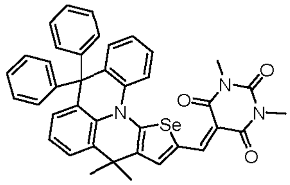
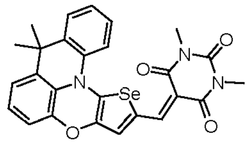

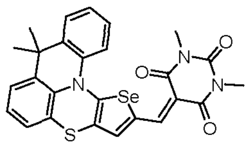
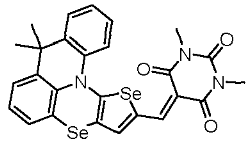
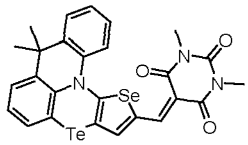
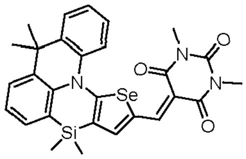

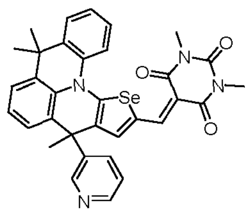
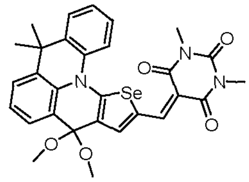
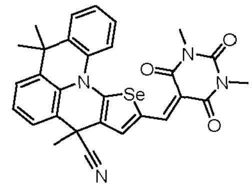
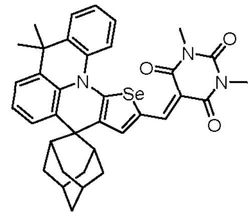

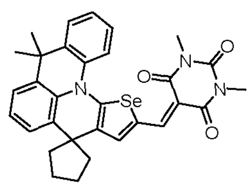
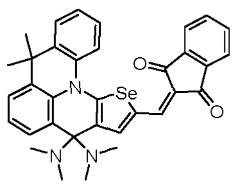
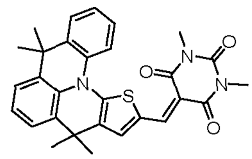
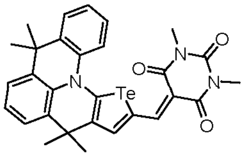

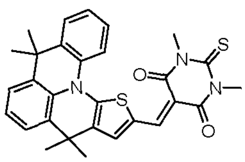
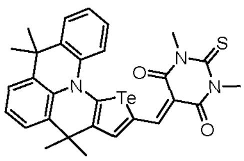
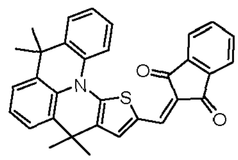
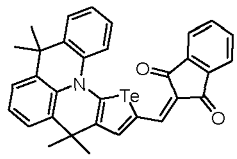

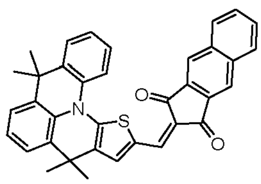 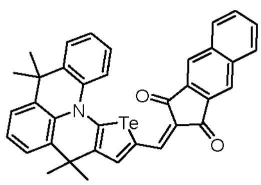 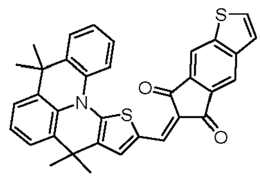 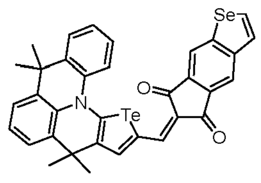

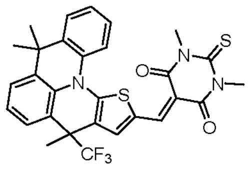 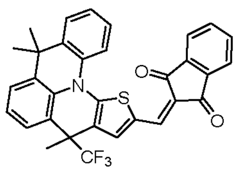 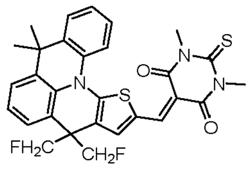 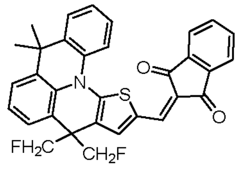

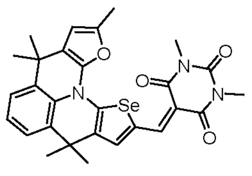 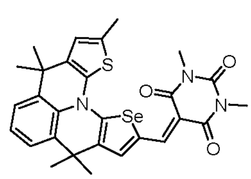 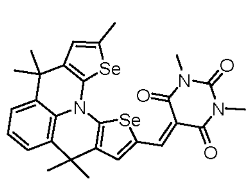 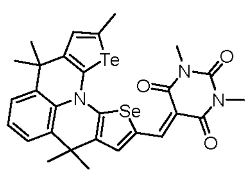

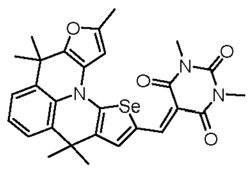 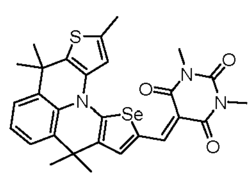 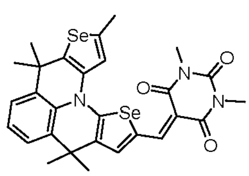 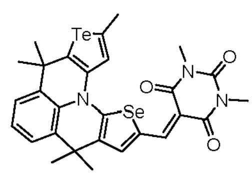

The n-type semiconductor compound may be sub-phthalocyanine or a sub-phthalocyanine derivative, fullerene or a fullerene derivative, thiophene or a thiophene derivative, or any combination thereof.

The fullerene may include C60, C70, C76, C78, C80, C82, C84, C90, C96, C240, C540, a mixture thereof, a fullerene nanotube, and the like. The fullerene derivative may refer to compounds of these fullerenes having a substituent thereof. The fullerene derivative may include a substituent such as an alkyl group (e.g., C1 to C30 alkyl group), an aryl group (e.g., C6 to C30 aryl group), a heterocyclic group (e.g., C3 to C30 heterocycloalkyl group), and the like. Examples of the aryl groups and heterocyclic groups may be a benzene ring, a naphthalene ring, an anthracene ring, a phenanthrene ring, a fluorene ring, a triphenylene ring, a naphthacene ring, a biphenyl ring, a pyrrole ring, a furan ring, a thiophene ring, an imidazole ring, an oxazole ring, a thiazole ring, a pyridine ring, a pyrazine ring, a pyrimidine ring, a pyridazine ring, an indolizine ring, an indole ring, a benzofuran ring, a benzothiophene ring, an isobenzofuran ring, a benzimidazole ring, an imidazopyridine ring, a quinolizidine ring, a quinoline ring, a phthalazine ring, a naphthyridine ring, a quinoxaline ring, a quinazoline ring, an isoquinoline ring, a carbazole ring, a phenanthridine ring, an acridine ring, a phenanthroline ring, a thianthrene ring, a chromene ring, an xanthene ring, a phenoxazine ring, a phenoxathin ring, a phenothiazine ring, or a phenazine ring.

The sub-phthalocyanine or the sub-phthalocyanine derivative may be represented by Chemical Formula 6.

[Chemical Formula 6]

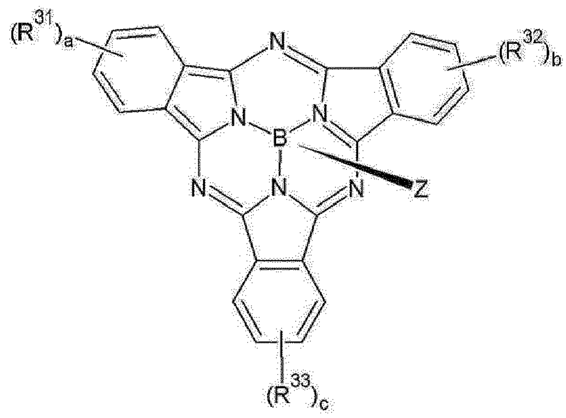

In Chemical Formula 6,

$R^{31}$ to $R^{33}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a halogen, a halogen-containing group, or any combination thereof,

a, b, and c are integers ranging from 1 to 3, and

Z is a monovalent substituent.

For example, Z may be a halogen or a halogen-containing group, for example F, Cl, an F-containing group, or a Cl-containing group.

The halogen refers to F, Cl, Br, or I and the halogen-containing group refers to alkyl group (C1 to C30 alkyl group) where at least one hydrogen of the alkyl group is replaced by F, Cl, Br, or I.

The thiophene derivative may be for example represented by Chemical Formula 7 or 8, but is not limited thereto.

[Chemical Formula 7]

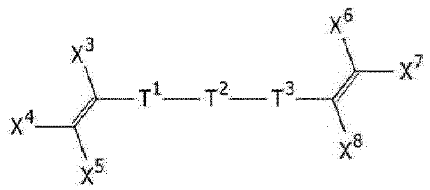

[Chemical Formula 8] $EWG^1$-$T^1$-$T^2$-$T^3$-$EWG^2$

In Chemical Formulas 7 and 8,

$T^1$, $T^2$, and $T^3$ may be aromatic rings including substituted or unsubstituted thiophene moieties,

$T^1$, $T^2$, and $T^3$ may each independently be present or may be fused to each other,

$X^3$ to $X^8$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a cyano group, a cyano-containing group, or any combination thereof, and

$EWG^1$ and $EWG^2$ may each independently be electron withdrawing groups.

For example, in Chemical Formula 7, at least one of $X^3$ to $X^8$ may be an electron withdrawing group, for example a cyano-containing group.

The composition for a photoelectric device may further include a second p-type semiconductor compound selectively absorbing green light. The second p-type semiconductor compound may be a compound represented by

Chemical Formula 9.

[Chemical Formula 9]

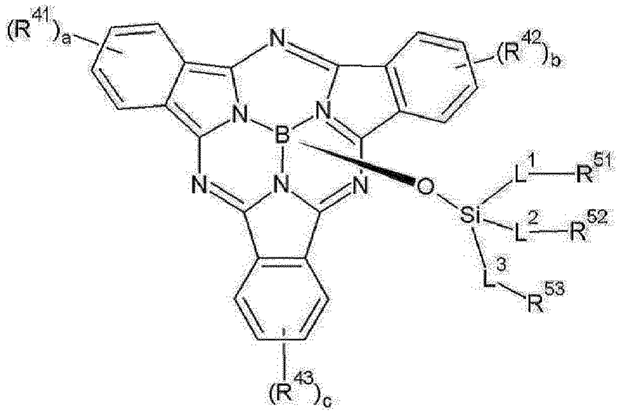

In Chemical Formula 9,

$R^{41}$ to $R^{43}$ may each independently be hydrogen, a substituted or unsubstituted C1 to C30 aliphatic hydrocarbon group, a substituted or unsubstituted C6 to C30 aromatic hydrocarbon group, a substituted or unsubstituted C1 to C30 aliphatic heterocyclic group, a substituted or unsubstituted C2 to C30 aromatic heterocyclic group, a substituted or unsubstituted C1 to C30 alkoxy group, a substituted or unsubstituted C6 to C30 aryloxy group, a thiol group, a substituted or unsubstituted C1 to C30 alkylthio group, a substituted or unsubstituted C6 to C30 arylthio group, a cyano group, a cyano-containing group, a halogen, a halogen-containing group, a substituted or unsubstituted sulfonyl group (e.g., a substituted or unsubstituted C0 to C30 aminosulfonyl group, a substituted or unsubstituted C1 to C30 alkylsulfonyl group or a substituted or unsubstituted C6 to C30 arylsulfonyl group), or any combination thereof, or two adjacent groups of $R^{41}$ to $R^{43}$ may be linked to each other to provide a fused ring,

$L^1$ to $L^3$ may each independently be a single bond, a substituted or unsubstituted C1 to C30 alkylene group, a substituted or unsubstituted C6 to C30 arylene group, a divalent a substituted or unsubstituted C3 to C30 heterocyclic group, or any combination thereof,

$R^{51}$ to $R^{53}$ may each independently be a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heterocyclic group, a substituted or unsubstituted amine group (e.g., a substituted or unsubstituted C1 to C30 alkylamine group, or a substituted or unsubstituted C6 to C30 arylamine group), a substituted or unsubstituted silyl group, or any combination thereof, and

a to c may each independently be an integer ranging from 0 to 4.

The second p-type semiconductor compound selectively absorbing green light may be included in an amount of about 500 to about 1500 parts by weight based on 100 parts by weight of the compound represented by Chemical Formula 1.

The composition for a photoelectric device may selectively absorb light in a green wavelength region and may have a maximum absorption wavelength ($\lambda_{max}$) in a wavelength region of greater than or equal to about 500 nm, for example greater than or equal to about 510 nm, greater than or equal to about 520 nm, greater than or equal to about 525 nm, or greater than or equal to about 530 nm and less than or equal to about 600 nm, less than or equal to about 590 nm, for example less than or equal to about 580 nm, less than or equal to about 570 nm, less than or equal to about 560 nm, less than or equal to about 555 nm, or less than or equal to about 550 nm, in a thin film state.

The composition for a photoelectric device may exhibit a light absorption curve having a full width at half maximum (FWHM) of about 50 nm to about 110 nm, for example about 50 nm to about 100 nm, in a thin film state. Herein, the FWHM is a width of a wavelength corresponding to half of a height of a maximum absorption point. When the FWHM is small, wavelength selectivity is increased by selectively absorbing light in a narrow wavelength region. As used herein, when specific definition is not otherwise provided, it may be defined by absorbance measured by UV-Vis spectroscopy. When the FWHM is within the range, selectivity in a green wavelength region may be increased. The thin film may be a thin film deposited under a vacuum condition.

The composition for a photoelectric device may have an absorption coefficient of greater than or equal to about $6.0\times10^4$ $cm^{-1}$, for example about $6.7\times10^4$ $cm^{-1}$ to about $10\times10^4$ $cm^{-1}$ or about $6.9\times10^4$ $cm^{-1}$ to about $10\times10^4$ $cm^{-1}$

when including the compound of Chemical Formula 1 and C60 in a volume ratio of about 0.9:1 to about 1.1:1, for example about 1:1.

In addition, the composition for a photoelectric device may exhibit improved residual charge characteristics while having high wavelength selectivity (narrow FWHM) and absorption coefficient, thereby reducing an after-image of the device.

The composition for a photoelectric device may be formed into a thin film by co-depositing a p-type semiconductor compound and an n-type semiconductor compound. The deposition method may provide a uniform thin film and have small inclusion possibility of impurities into the thin film, but when the compound has a lower melting point than a temperature for the deposition, a product decomposed from the compound may be deposited and thus performance of a device may be deteriorated. Accordingly, the compound desirably has a higher melting point than the deposition temperature. The compound has, for example, at least about 10 °C, for example at least about 20 °C, or at least about 30 °C higher melting point than the deposition temperature and thus may be desirably used for the deposition.

In more detail, the donor-acceptor type material represented by Chemical Formula 1 may be thermally decomposed at the melting point ($T_m$) of the material because the melting point ($T_m$) is similar to the decomposition temperature ($T_d$). Accordingly, if the temperature (sublimation temperature, deposition temperature, $T_s$) at which a film is formed by vacuum deposition is higher than $T_m$, decomposition occurs more preferentially than sublimation (deposition), and thus a normal device cannot be manufactured. Since it is impossible to produce a stable image sensor with these materials, $T_m$ should be higher than $T_s$, and desirably $T_m$-$T_s$≥10 °C.

In addition, a micro lens array (MLA) needs to be formed to concentrate light after manufacturing an organic photoelectric device during manufacture of an image sensor. This micro lens array requires a relatively high temperature (greater than or equal to about 160 °C, for example greater than or equal to about 170 °C, greater than or equal to about 180 °C, or greater than or equal to about 190 °C). The performance of the photoelectric devices (e.g., organic photoelectric devices) is required not to be deteriorated in these heat-treatment processes. The performance deterioration of the organic photoelectric device during the heat treatment of MLA may be caused not by chemical decomposition of an organic material but its morphology change. The morphology change is in general caused, when a material starts a thermal vibration due to a heat treatment, but a material having a firm molecule structure may not have the thermal vibration and be limited and/or prevented from the deterioration by the heat treatment. The compound may be suppressed from the thermal vibration of molecules due to a conjugation structure in the donor moiety and thus may be stably maintained during the MLA heat treatment and secure process stability.

Hereinafter, a photoelectric device including the compound according to some example embodiments is described with reference to drawings.

FIG. 1 is a cross-sectional view showing a photoelectric device according to some example embodiments.

Referring to FIG. 1, a photoelectric device 100 according to some example embodiments includes a first electrode 10 and a second electrode 20, and an active layer 30 between the first electrode 10 and the second electrode 20.

One of the first electrode 10 or the second electrode 20 is an anode and the other is a cathode. At least one of the first electrode 10 or the second electrode 20 may be a light-transmitting electrode, and the light-transmitting electrode may be made of, for example, a transparent conductor such as indium tin oxide (ITO) or indium zinc oxide (IZO), or a metal thin layer of a thin single layer or multilayer. When one of the first electrode 10 or the second electrode 20 is a non-light-transmitting electrode, it may be made of, for example, an opaque conductor such as aluminum (Al).

The active layer 30 includes a p-type semiconductor and an n-type semiconductor to form a pn junction, and absorbs external light to generate excitons and then separates the generated excitons into holes and electrons.

The active layer 30 includes the composition for a photoelectric device.

The active layer 30 may have a maximum absorption wavelength ($\lambda_{max}$) in a wavelength region of greater than or equal to about 500 nm, for example greater than or equal to about 510 nm, greater than or equal to about 520 nm, greater than or equal to about 525 nm, greater than or equal to about 530 nm, or greater than or equal to about 535 nm and less than or equal to about 590 nm, for example less than or equal to about 580 nm, less than or equal to about 570 nm, or less than or equal to about 560 nm.

The active layer 30 may exhibit a light absorption curve having a relatively narrow full width at half maximum (FWHM) of about 50 nm to about 110 nm, for example about 50 nm to about 100 nm. Accordingly, the active layer 30 has high selectivity for light in a green wavelength region.

The active layer 30 may be a single layer or a multilayer. The active layer 30 may be, for example, an intrinsic layer (I layer), a p-type layer/I layer, an I layer/n-type layer, a p-type layer/I layer/n-type layer, a p-type layer/n-type layer, and the like.

The intrinsic layer (I layer) may include the compound of Chemical Formula 1 and the n-type semiconductor compound in a ratio (volume ratio or thickness ratio) of about 1:100 to about 100:1. The compound of Chemical Formula 1 and the n-type semiconductor compound may be included in a ratio ranging from about 1:50 to about 50:1 within the range, specifically, about 1:10 to about 10:1, and more specifically, about 1:1. When the compound of Chemical Formula 1 and the n-type semiconductor compound have a ratio within the range, an exciton may be effectively produced, and

a pn junction may be effectively formed.

The p-type layer may include the semiconductor compound of Chemical Formula 1, and the n-type layer may include the n-type semiconductor compound.

The active layer 30 may have a thickness of about 1 nm to about 500 nm and specifically, about 5 nm to about 300 nm, about 20 nm to about 250 nm, about 30 nm to about 200 nm, about 50 nm to about 150 nm, about 70 nm to about 130 nm, or about 80 nm to about 120 nm. When the active layer 30 has a thickness within the range, the active layer may effectively absorb light, effectively separate holes from electrons, and deliver them, thereby effectively improving photoelectric conversion efficiency. A desirable thickness of the active layer 30 may be, for example, determined by an absorption coefficient of the active layer 30, and may be, for example, a thickness being capable of absorbing light of at least about 70% or more, for example about 80% or more, and for another example about 90% or more.

In the photoelectric device 100, when light enters from the first electrode 10 and/or second electrode 20, and when the active layer 30 absorbs light in a desired and/or alternatively predetermined wavelength region, excitons may be produced from the inside. The excitons are separated into holes and electrons in the active layer 30, and the separated holes are transported to an anode that is one of the first electrode 10 or the second electrode 20 and the separated electrons are transported to the cathode that is the other of the first electrode 10 and the second electrode 20 so as to flow a current in the photoelectric device.

Hereinafter, a photoelectric device according to some example embodiments is described with reference to FIG. 2.

FIG. 2 is a cross-sectional view showing a photoelectric device according to some example embodiments.

Referring to FIG. 2, a photoelectric device 200 according to some example embodiments, including the example embodiments shown in FIG. 2 includes a first electrode 10 and a second electrode 20 facing each other, and an active layer 30 between the first electrode 10 and the second electrode 20, like some example embodiments, including the example embodiments shown in FIG. 1.

However, the photoelectric device 200 according to some example embodiments, including the example embodiments shown in FIG. 2 further includes charge auxiliary layers 40 and 45 between the first electrode 10 and the active layer 30, and the second electrode 20 and the active layer 30, unlike some example embodiments, including the example embodiments shown in FIG. 1. The charge auxiliary layers 40 and 45 may facilitate the transfer of holes and electrons separated from the active layer 30, so as to increase efficiency.

The charge auxiliary layers 40 and 45 may be at least one selected from a hole injection layer (HIL) for facilitating hole injection, a hole transport layer (HTL) for facilitating hole transport, an electron blocking layer (EBL) for preventing electron transport, an electron injection layer (EIL) for facilitating electron injection, an electron transport layer (ETL) for facilitating electron transport, and a hole blocking layer (HBL) for preventing hole transport.

The charge auxiliary layers 40 and 45 may include, for example, an organic material, an inorganic material, or an organic/inorganic material. The organic material may be an organic compound having hole or electron characteristics, and the inorganic material may be, for example, a metal oxide such as molybdenum oxide, tungsten oxide, nickel oxide, and the like.

The hole transport layer (HTL) may include one selected from, for example, poly(3,4-ethylenedioxythiophene):poly(styrenesulfonate) (PEDOT:PSS), polyarylamine, poly(N-vinylcarbazole), polyaniline, polypyrrole, N,N,N',N'-tetrakis(4-methoxyphenyl)-benzidine (TPD), 4,4'-bis[N-(1-naphthyl)-N-phenyl-amino]biphenyl ($\alpha$-NPD), m-MTDATA, 4,4',4"-tris(N-carbazolyl)-triphenylamine (TCTA), and any combination thereof, but is not limited thereto.

The electron blocking layer (EBL) may include one selected from, for example, poly(3,4-ethylenedioxythiophene):poly(styrenesulfonate) (PEDOT:PSS), polyarylamine, poly(N-vinylcarbazole), polyaniline, polypyrrole, N,N,N',N'-tetrakis(4-methoxyphenyl)-benzidine (TPD), 4,4'-bis[N-(1-naphthyl)-N-phenyl-amino]biphenyl ($\alpha$-NPD), m-MTDATA, 4,4',4"-tris(N-carbazolyl)-triphenylamine (TCTA), and any combination thereof, but is not limited thereto.

The electron transport layer (ETL) may include one selected from, for example, 1,4,5,8-naphthalene-tetracarboxylic dianhydride (NTCDA), bathocuproine (BCP), LiF, $Alq_3$, $Gaq_3$, $Inq_3$, $Znq_2$, $Zn(BTZ)_2$, $BeBq_2$, and any combination thereof, but is not limited thereto.

The hole blocking layer (HBL) may include one selected from, for example, 1,4,5,8-naphthalene-tetracarboxylic dianhydride (NTCDA), bathocuproine (BCP), LiF, $Alq_3$, $Gaq_3$, $Inq_3$, $Znq_2$, $Zn(BTZ)_2$, $BeBq_2$, and any combination thereof, but is not limited thereto.

Either one of the charge auxiliary layers 40 or 45 may be omitted.

The photoelectric device may be applied to various fields, for example a solar cell, an image sensor, a photodetector, a photo-sensor, and an organic light emitting diode (OLED), but is not limited thereto.

Hereinafter, an example of an image sensor including the organic photoelectric device is described referring to drawings. As an example of an image sensor, an organic CMOS image sensor is described.

FIG. 3 is a schematic top plan view showing an organic CMOS image sensor according to some example embodiments, and FIG. 4 is a cross-sectional view showing the organic CMOS image sensor of FIG. 3.

Referring to FIGS. 3 and 4, an organic CMOS image sensor 300 according to some example embodiments

includes a semiconductor substrate 310 integrated with photo-sensing device 50, which may include photo-sensing devices 50B and 50R, a transmission transistor (not shown), a charge storage 55, a lower insulation layer 60, a color filter layer 70, an upper insulation layer 80 (also referred to herein as an insulation layer), and a photoelectric device 100. The photoelectric device 100 may be the photoelectric device according to any of the example embodiments and is on (e.g., directly or indirectly on) the semiconductor substrate 310.

The semiconductor substrate 310 may be a silicon substrate, and is integrated with a photo-sensing device 50, the transmission transistor (not shown), and the charge storage 55. The photo-sensing device 50 may include the photo-sensing devices 50R and 50B. The photo-sensing devices 50R and 50B may be photodiodes.

The photo-sensing devices 50B and 50R, the transmission transistor, and/or the charge storage 55 may be integrated in each pixel, and as shown in the drawing, the photo-sensing devices 50B and 50R may be respectively included in a blue pixel and a red pixel and the charge storage 55 may be included in a green pixel.

The photo-sensing devices 50B and 50R sense light, the information sensed by the photo-sensing devices may be transferred by the transmission transistor, the charge storage 55 is electrically connected to the photoelectric device 100, and the information of the charge storage 55 may be transferred by the transmission transistor.

In the drawings, the photo-sensing devices 50B and 50R are, for example, arranged in parallel without limitation, or the blue photo-sensing device 50B and the red photo-sensing device 50R may be stacked in a vertical direction.

A metal wire (not shown) and a pad (not shown) are formed on the semiconductor substrate 310. In order to decrease signal delay, the metal wire and pad may be made of a metal having low resistivity, for example, aluminum (Al), copper (Cu), silver (Ag), and alloys thereof, but are not limited thereto. Further, it is not limited to the structure, and the metal wire and pad may be positioned under the photo-sensing devices 50B and 50R.

The lower insulation layer 60 is formed on the metal wire and the pad. The lower insulation layer 60 may be made of an inorganic insulating material such as a silicon oxide and/or a silicon nitride, or a low dielectric constant (low K) material such as SiC, SiCOH, SiCO, and SiOF. The lower insulation layer 60 has a trench exposing the charge storage 55. The trench may be filled with fillers.

A color filter layer 70 is formed on the lower insulation layer 60 and may be located between the photoelectric device 100 and the semiconductor substrate 310. The color filter layer 70 includes a blue filter 70B formed in the blue pixel and configured to selectively transmit blue light and a red filter 70R formed in the red pixel and configured to selectively transmit red light. In some example embodiments, a cyan filter and a yellow filter may be disposed instead of the blue filter 70B and red filter 70R. In some example embodiments, including the example embodiments shown in FIGS. 3-4, a green filter is not included, but a green filter may be further included.

The color filter layer 70 may be omitted. For example, when the blue photo-sensing device 50B and the red photo-sensing device 50R are stacked in a vertical direction, the blue photo-sensing device 50B and the red photo-sensing device 50R may selectively absorb light in each wavelength region depending on their stack depth, and the color filter layer 70 may not be equipped.

The upper insulation layer 80 is formed on the color filter layer 70. The upper insulation layer 80 eliminates a step caused by the color filter layer 70 and smoothens the surface. The upper insulation layer 80 and the lower insulation layer 60 may include a contact hole (not shown) exposing a pad, and a through-hole 85 exposing the charge storage 55 of the green pixel.

The aforementioned photoelectric device 100 is formed on (e.g., directly or indirectly on) the upper insulation layer 80. The photoelectric device 100 includes the first electrode 10, the active layer 30, and the second electrode 20 as described above.

The first electrode 10 and the second electrode 20 may be transparent electrodes, and the active layer 30 is the same as described above. The active layer 30 selectively absorbs and/or senses light in a green wavelength region and replaces a color filter of a green pixel. Accordingly, the photoelectric device 100 may be configured to selectively sense light in the green wavelength region.

When light enters from the second electrode 20, the light in a green wavelength region may be mainly absorbed in the active layer 30 and photoelectrically converted, while the light in the rest of the wavelength regions passes through first electrode 10 and may be sensed in the photo-sensing devices 50B and 50R.

As described above, the photoelectric devices selectively absorbing light in a green wavelength region are stacked and thereby a size of an image sensor may be decreased and a down-sized image sensor may be realized.

As described above, the compound represented by the Chemical Formula 1 may be used as a p type semiconductor compound, aggregation between compounds in a thin film state is inhibited, and thereby light absorption characteristics depending on a wavelength may be maintained. Thereby, green wavelength selectivity may be maintained, crosstalk caused by unnecessary absorption of other light except a green wavelength region may be decreased and sensitivity may be increased.

In some example embodiments, in FIG. 4, additional color filters may be further disposed on the photoelectric device 100. The additional color filters may include a blue filter 70B and a red filter 70R or a cyan filter and a yellow filter.

The organic CMOS image sensor with the color filters disposed on the photoelectric device is shown in FIG.

5. FIG. 5 is a schematic cross-sectional view showing an organic CMOS image sensor according to some example embodiments. Referring to FIG. 5, an organic CMOS image sensor 400 has the same structure as FIG. 4 except that a color filter layer 72 including the blue filter 72B and the red filter 72R is disposed on the photoelectric device 100 such that the photoelectric device 100 is between the color filter layer 72 and the semiconductor substrate 310. Instead of the blue filter 72B and the red filter 72R, a cyan filter and a yellow filter may be disposed respectively.

In FIGS. 4 and 5, the photoelectric device 100 of FIG. 1 is included, but it is not limited thereto, and thus the photoelectric device 200 of FIG. 2 may be applied in the same manner.

FIG. 6 is a cross-sectional view showing an organic CMOS image sensor 500 to which the photoelectric device 200 is applied.

Referring to FIG. 6, the organic CMOS image sensor 500 includes a semiconductor substrate 310 integrated with photo-sensing devices 50B and 50R, a transmission transistor (not shown), a charge storage 55, an insulation layer 80, and a photoelectric device 200, like some example embodiments, including the example embodiments shown in FIG. 4.

However, the organic CMOS image sensor 500 according to some example embodiments, including the example embodiments shown in FIG. 6, includes the photoelectric device 200, unlike some example embodiments, including the example embodiments shown in FIG. 4, which include the photoelectric device 100.

FIG. 7 is a schematic view showing an organic CMOS image sensor according to some example embodiments.

Referring to FIG. 7, the organic CMOS image sensor 600 includes a semiconductor substrate 310 integrated with photo-sensing devices 50B and 50R, a transmission transistor (not shown), a charge storage 55, an insulation layer 80, and a photoelectric device 100, like some example embodiments, including the example embodiments shown in FIG. 5.

However, the organic CMOS image sensor 600 according to some example embodiments, including the example embodiments shown in FIG. 7, includes the blue photo-sensing device 50B and the red photo-sensing device 50R that are stacked in a vertical direction in the semiconductor substrate 310 (e.g., perpendicular to a direction in which the upper surface of the semiconductor substrate 310 extends as shown in FIG. 7) and does not include a color filter layer 70, unlike some example embodiments, including the example embodiments shown in FIG. 5. For example, each red photo-sensing device 50R of a plurality of red photo-sensing devices 50R integrated in the semiconductor substrate 310 may be stacked in a vertical direction with a separate blue photo-sensing device 50B of a plurality of blue photo-sensing devices 50B in the semiconductor substrate 310. The blue photo-sensing device 50B and the red photo-sensing device 50R are electrically connected with the charge storage 55, and the information of the charge storage 55 may be transferred by the transmission transistor (not shown). The blue photo-sensing device 50B and the red photo-sensing device 50R may selectively absorb light in each wavelength region depending on a stack depth.

As described above, the photoelectric devices selectively absorbing light in a green wavelength region are stacked and the red photo-sensing device and the blue photo-sensing device are stacked, and thereby a size of an image sensor may be decreased and a down-sized image sensor may be realized. As described above, the photoelectric device 100 has improved green wavelength selectivity, and crosstalk caused by unnecessary absorption light in a wavelength region except green may be decreased while increasing sensitivity.

In FIG. 7, the photoelectric device 100 of FIG. 1 is included, but it is not limited thereto, and thus the photoelectric device 200 of FIG. 2 may be applied in the same manner.

FIG. 8 is a schematic view showing an organic CMOS image sensor according to some example embodiments.

Referring to FIG. 8, the organic CMOS image sensor according to some example embodiments, including the example embodiments shown in FIG. 8 includes a green photoelectric device (G) selectively absorbing light in a green wavelength region, a blue photoelectric device (B) selectively absorbing light in a blue wavelength region, and a red photoelectric device (R) selectively absorbing light in a red wavelength region that are stacked. Restated, the organic CMOS image sensor may include a green photoelectric device configured to selectively sense light in a green wavelength region, a blue photoelectric device configured to selectively sense light in a blue wavelength region, and a red photoelectric device configured to selectively sense light in a red wavelength region, where the green photoelectric device, the blue photoelectric device, and the red photoelectric device are stacked as shown in at least FIG. 8.

In the drawing, the green photoelectric device, the blue photoelectric device, and the red photoelectric device are sequentially stacked, but the stack order may be changed without limitation.

The green photoelectric device (G) may be the aforementioned photoelectric device 100 or photoelectric device 200, the blue photoelectric device (B) may include electrodes facing each other and an active layer therebetween and including an organic material selectively absorbing light in a blue wavelength region, and the red photoelectric device (R) may include electrodes facing each other and an active layer therebetween and including an organic material selectively absorbing light in a red wavelength region.

As described above, the green photoelectric device (G) selectively absorbing light in a green wavelength region, the blue photoelectric device (B) selectively absorbing light in a blue wavelength region, and the red photoelectric device (R) selectively absorbing light in a red wavelength region are stacked, and thereby a size of an image sensor may be

decreased and a down-sized image sensor may be realized.

The image sensor absorbs light in an appropriate wavelength region and may show all improved sensitivity (YSNR10) and color reproducibility (ΔE*ab) despite a stacked structure.

Herein, the YSNR10 indicates sensitivity of the image sensor, which is measured in a method described in Juha Alakarhu's "Image Sensors and Image Quality in Mobile Phones" printed in 2007 International Image Sensor Workshop (Ogunquit Maine, USA) but minimum illuminance expressed by lux at a ratio of 10 between signal and noise. Accordingly, the smaller the YSNR10 is, the higher sensitivity is.

On the other hand, the color reproducibility (ΔE*ab) shows a difference from standard colors in an X-Rite chart, and the ΔE*ab is defined as a distance between two points on a L*a*b* color space by CIE ( Commission International de L' Eclairage ) in 1976. For example, the color difference may be calculated according to Equation 1.

[Equation 1]

$$\Delta E = \sqrt{(\Delta L^*)^2 + (\Delta a^*)^2 + (\Delta b^*)^2}$$

In Equation 1,

ΔL* denotes a change of a color coordinate L* compared with the color coordinate L* at room temperature (about 20 °C to about 25 °C),
Δa* denotes a change of a color coordinate a* compared with the color coordinate a* at room temperature (about 20 °C to about 25 °C), and
Δb* denotes a change of a color coordinate b* compared with the color coordinate b* at room temperature (about 20 °C to about 25 °C).

In order to manufacture an image sensor having high sensitivity and high color reproducibility, YSNR10≤100lux at ΔE*ab≤3, and herein, the compound may realize YSNR10≤100 lux of sensitivity and color reproducibility at ΔE*ab≤3.

The image sensor may be applied to various electronic devices, for example, a mobile phone, a digital camera, and the like but is not limited thereto.

FIG. 9 is a block diagram of a digital camera including an image sensor according to some example embodiments.

Referring to FIG. 9, a digital camera 1000 includes a lens 1010, an image sensor 1020, a motor 1030, and an engine 1040. The image sensor 1020 may be an image sensor according to any of the example embodiments, including one of the image sensors according to any of the example embodiments shown in FIGS. 3 to 8.

The lens 1010 concentrates incident light on the image sensor 1020. The image sensor 1020 generates RGB data for received light through the lens 1010.

In some example embodiments, the image sensor 1020 may interface with the engine 1040.

The motor 1030 may adjust the focus of the lens 1010 or perform shuttering in response to a control signal received from the engine 1040. The engine 1040 may control the image sensor 1020 and the motor 1030.

The engine 1040 may be connected to a host/application 1050.

FIG. 10 is a schematic diagram showing an electronic device according to some example embodiments.

Referring to FIG. 10, an electronic device 1100 may include a processor 1120, a memory 1130, and an image sensor 1140 that are electrically coupled together via a bus 1110.

The image sensor 1140 may be an image sensor according to any of the example embodiments. The memory 1130 may be a non-transitory computer readable medium and may store a program of instructions. The memory 1130 may be a nonvolatile memory, such as a flash memory, a phase-change random access memory (PRAM), a magneto-resistive RAM (MRAM), a resistive RAM (ReRAM), or a ferro-electric RAM (FRAM), or a volatile memory, such as a static RAM (SRAM), a dynamic RAM (DRAM), or a synchronous DRAM (SDRAM). The processor 1120 may execute the stored program of instructions to perform one or more functions. For example, the processor 1120 may be configured to process electrical signals generated by the image sensor 1140. The processor 1120 may include processing circuitry such as hardware including logic circuits; a hardware/software combination such as a processor executing software; or any combination thereof. For example, the processing circuitry more specifically may include, but is not limited to, a central processing unit (CPU), an arithmetic logic unit (ALU), a digital signal processor, a microcomputer, a field programmable gate array (FPGA), a System-on-Chip (SoC), a programmable logic unit, a microprocessor, application-specific integrated circuit (ASIC), etc. The processor 1120 may be configured to generate an output (e.g., an image to be displayed on a display interface) based on such processing.

One or more of the processor 1120, memory 1130, motor 1030, engine 1040, or host/application 1050 may be

included in, include, and/or implement one or more instances of processing circuitry such as hardware including logic circuits, a hardware/software combination such as a processor executing software; or any combination thereof. In some example embodiments, said one or more instances of processing circuitry may include, but are not limited to, a central processing unit (CPU), an application processor (AP), an arithmetic logic unit (ALU), a graphic processing unit (GPU), a digital signal processor, a microcomputer, a field programmable gate array (FPGA), a System-on-Chip (SoC) a programmable logic unit, a microprocessor, or an application-specific integrated circuit (ASIC), etc. In some example embodiments, any of the memories, memory units, or the like as described herein may include a non-transitory computer readable storage device, for example a solid state drive (SSD), storing a program of instructions, and the one or more instances of processing circuitry may be configured to execute the program of instructions to implement the functionality of some or all of any of the processor 1120, memory 1130, motor 1030, engine 1040, or host/application 1050, or the like according to any of the example embodiments as described herein.

Hereinafter, some example embodiments are illustrated in more detail with reference to examples. However, these examples are non-limiting, and inventive concepts are not limited thereto.

**Synthesis Example 1: Synthesis of Compound Represented by Chemical Formula 1-1**

[Chemical Formula 1-1]

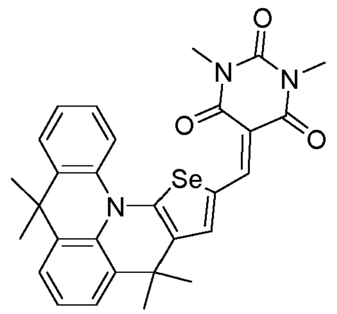

[Reaction Scheme 1-1]

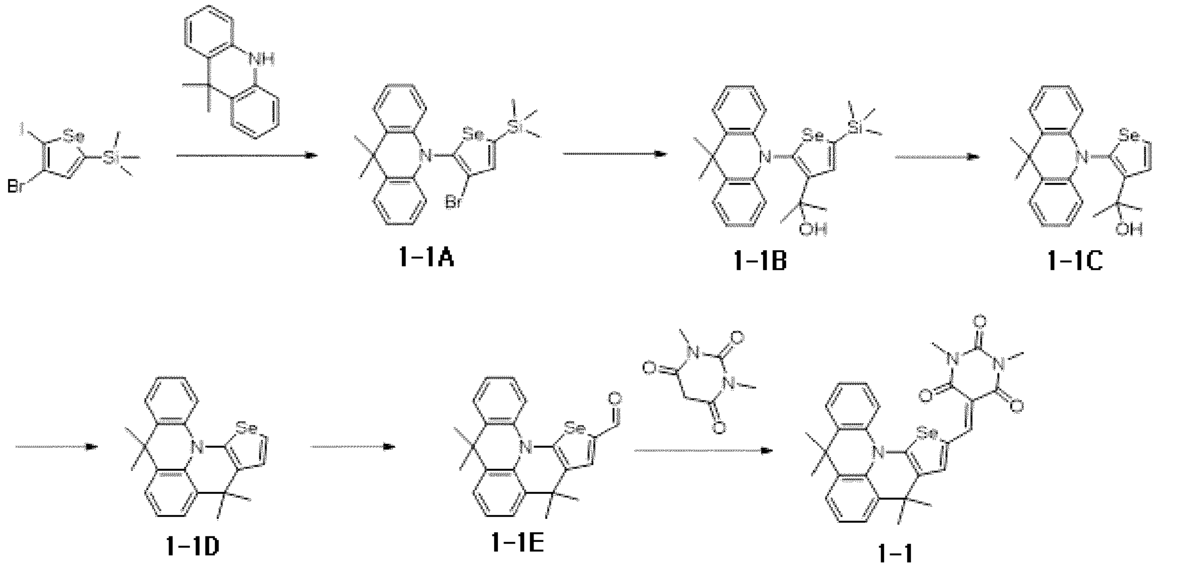

1-1A

1-1B

1-1C

1-1D

1-1E

1-1

(i) Synthesis of Compound 1-1A

2.10 g ( 5.16 mmol) of (4-bromo-5-iodoselenophen-2-yl)trimethylsilane and 0.900 g (4.30 mmol) of 9,9-dimethyl-9,10-dihydroacridine are placed in a microwave vial and are dissolved in 12 ml of toluene. 0.247g (0.43 mmol) of bis(dibenzylideneacetone)palladium ($Pd(dba)_2$), 0.34ml (0.86 mmol) of 50 wt% of triphenylphosphine (($PPh_3$) solution, and 1.24 g (12.9 mmol) of sodium tert-butoxide (NaOtBu) are added thereto and reacted in a microwave reactor at 100 °C for 30 minutes. Then, the product is filtered using Celite and the filtrate is concentrated, and separated and purified through silica gel column chromatography (hexane:dichloromethane = volume ratio of 19:1). Through this, 1.2 g of Compound 1-1A (Yield: 57%) is obtained. The above processes are repeated to obtain a sufficient amount of Compound 1-1A.

(ii) Synthesis of Compound 1-1B

7.60 g (15.5 mmol) of Compound 1-1A is dissolved in 150 ml of dehydrated diethyl ether. At -70°C, 7.45 ml (18.6 mmol) of 2.5 M normal butyllithium hexane solution (n-BuLi (n-butyl lithium) in hexane solution) is slowly added thereto and stirred for 1 hour. 1.71 ml (23.3 mmol) of dehydrated acetone is added at -70°C, and the mixture is stirred at room temperature for 1 hour. The product is washed with an aqueous sodium chloride solution, extracted with ethyl acetate, and dried by adding anhydrous magnesium sulfate. The obtained product is separated and purified through silica gel column chromatography (hexane:dichloromethane=volume ratio of 2:3) to obtain 4.00 g of Compound 1-1B (Yield: 55%). The above processes are repeated to obtain a sufficient amount of Compound 1-1B.

(iii) Synthesis of Compound 1-1C

5.43 g (12.1 mmol) of Compound 1-1B is dissolved in 120 ml of tetrahydrofuran. 13.3 ml (13.3 mmol) of tetra-n-butylammonium fluoride (TBAF) is added dropwise thereto, followed by stirring for 1 hour. After washing the reactants with an aqueous sodium chloride solution, anhydrous magnesium sulfate is added to the organic layer and then dried. The obtained product is separated and purified through silica gel column chromatography (hexane:dichloromethane = volume ratio of 9:1) to obtain 4.2 g of Compound 1-1C (Yield: 92%).

(iv) Synthesis of Compound 1-1D

2.16 g (5.71 mmol) of Compound 1-1C is dissolved in 250 ml of toluene. 3.71 ml (57.1 mmol) of methanesulfonic acid is added dropwise thereto, and the mixture is stirred for 12 hours. The product is poured into ice water, neutralized by adding a 2 M aqueous sodium hydroxide solution, and then the organic layer extracted with toluene is washed with an aqueous sodium chloride solution. Anhydrous magnesium sulfate is added to the organic layer and then the resultant is dried, concentrated and purified through silica gel column chromatography (hexane:dichloromethane=volume ratio of 9:1) to obtain 1.4 g of Compound 1-1D (Yield: 63%).

(v) Synthesis of Compound 1-1E

0.74 ml (7.9 mmol) of phosphoryl chloride is added dropwise to 2.00 ml (25.7 mmol) of N,N-dimethylformamide at 0 °C, followed by stirring at room temperature for 2 hours. This solution is slowly added to a solution of 1.50 g (3.96 mmol) of Compound 1-1D in 50 ml of dichloromethane at 0 °C, followed by stirring at room temperature for 1 hour. Water is added to the obtained product, and 2M aqueous sodium hydroxide solution is added until the pH value is 14, followed by stirring at room temperature for 2 hours. The organic layer extracted with dichloromethane is washed with aqueous sodium chloride solution, dried with anhydrous magnesium sulfate, and then concentrated. The obtained product is separated and purified through silica gel column chromatography (volume ratio is changed from hexane:dichloromethane= volume ratio of 3:2 to 100% of dichloromethane) to obtain 1.42 g of Compound 1-1E (Yield: 88%).

(vi) Synthesis of Compound Represented by Chemical Formula 1-1

1.00 g (2.46 mmol) of Compound 1-1E is suspended in 100 ml of ethanol, 0.420 g (2.69 mmol) of 1,3-dimethylbarbituric acid is added thereto, and the mixture is stirred at 50 °C for 4 hours and concentrated under reduced pressure. The resultant is dissolved in chloroform and the obtained mixture is filtered by a silica gel filter. The filtrate is concentrated under reduced pressure, and the obtained product is recrystallized using chloroform and ethanol to obtain 1.05 g of the compound represented by Chemical Formula 1-1 (Yield: 78.4%). The obtained compound is purified by sublimation to a purity of 99.9%.

$^1$H-NMR (500 MHz, CD2Cl2): δ 8.63(s, 1H), 8.17 (d, 1H), 8.12 (s, 1H), 7.59 (d, 1H), 7.42-7.27 (m, 5H), 3.34 (s, 3H), 3.29 (s, 3H), 1.62 (s, 12H).

**Synthesis Example 2: Synthesis of Compound Represented by Chemical Formula 1-2**

[Chemical Formula 1-2]

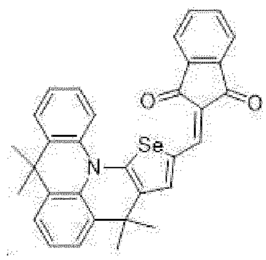

A compound represented by Chemical Formula 1-2 is obtained in the same manner as in Synthesis Example 1 except that 1H-indene-1,3(2H)-dione is used instead of 1,3-dimethylbarbituric acid in step (vi) of Synthesis Example 1. The obtained compound is purified by sublimation to a purity of 99.9%.

$^1$H-NMR (500 MHz, CD2Cl2): δ 8.16(d, 1H), 8.03 (s, 1H), 8.00 (s, 1H), 7.82-7.79 (m, 2H), 7.71-7.69 (m, 2H), 7.60 (d, 1H), 7.42-7.32 (m, 4H), 7.27 (t, 1H), 1.62 (s, 12H).

**Synthesis Example 3: Synthesis of Compound Represented by Chemical Formula 1-3**

[Chemical Formula 1-3]

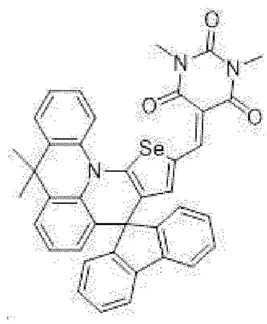

A compound represented by Chemical Formula 1-3 is obtained in the same manner as in Synthesis Example 1 except that 9H-fluoren-9-one is used instead of acetone in the step (ii) of Synthesis Example 1. The obtained compound is purified by sublimation to a purity of 99.9%.

$^1$H-NMR (500 MHz, CD2Cl2): δ 8.38 (d, 1H), 8.28 (s, 1H), 7.85 (d, 2H), 7.67 (d, 1H), 7.46-7.40 (m, 5H), 7.25 (s, 4H), 7.22 (s, 1H), 6.96 (t, 1H), 6.45 (d, 1H), 3.28 (s, 3H), 3.27 (s, 3H), 1.67 (s, 6H).

**Synthesis Example 4: Synthesis of Compound Represented by Chemical Formula 1-4**

[Chemical Formula 1-4]

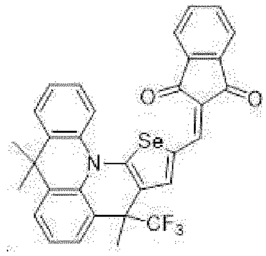

A compound represented by Chemical Formula 1-4 is obtained in the same manner as in Synthesis Example 1 except that 1,1, 1-trifluoroacetone is used instead of acetone in step (ii) of Synthesis Example 1, and 1H-indene-1,3(2H)-dione is used instead of 1,3-dimethylbarbituric acid) in step (vi) of Synthesis Example 1. The obtained compound is purified by sublimation to a purity of 99.9%.

$^1$H-NMR (500 MHz, CD2Cl2): δ 8.13 (d, 1H), 8.09 (s, 1H), 8.02 (d, 1H), 7.86-7.83 (m, 2H), 7.75-7.73 (m, 2H), 7.61-7.50 (m, 3H), 7.36-7.31 (m, 3H), 2.11 (s, 3H), 1.95 (s, 3H), 1.26 (s, 3H).

**Reference Synthesis Example 1: Synthesis of Compound Represented by Chemical Formula 2-1**

[Chemical Formula 2-1]

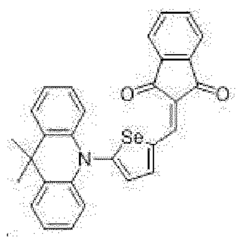

[Reaction Scheme 2-1]

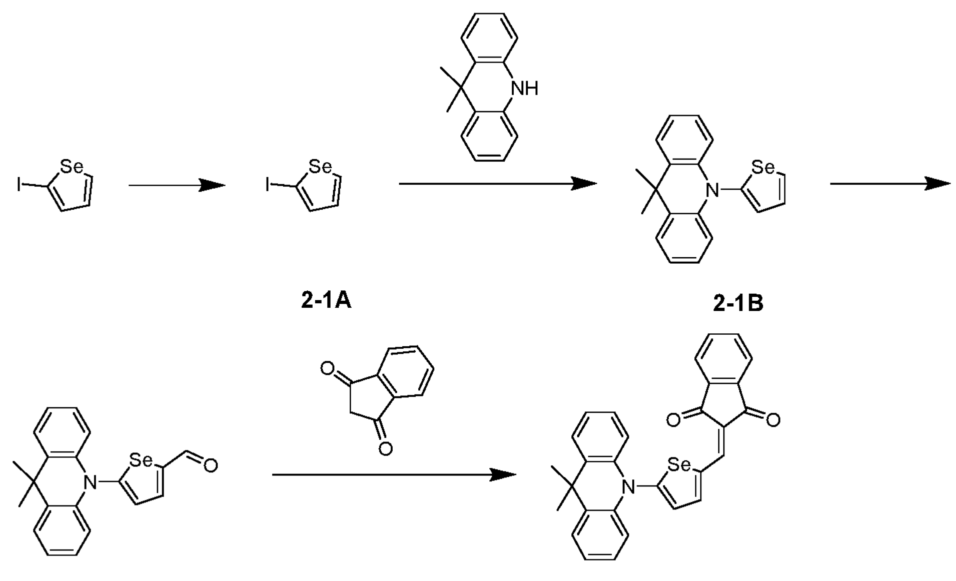

2-1A

2-1B

2-1C

2-1D

(i) Synthesis of Compound 2-1A

Compound 2-1A (2-iodoselenophene) is synthesized referring to the method disclosed in Efficient Synthesis of 2-Iodo and 2-Dicyanomethyl Derivatives of Thiophene, Selenophene, Tellurophene, and Thieno[3,2-b]thiophene, Takahashi, K.; Tarutani, S. Heterocycles 1996, 43, 1927-1935.

(ii) Synthesis of Compound 2-1B

7.47 g (28.7 mmol) of 2-iodoselenophene and 5.00 g (23.9 mmol) of 9,9-dimethyl-9,10-dihydroacridine are dissolved in 80 ml of toluene in the presence of 5 mol% of $Pd(dba)_2$, 10 mol% of $P(tBu)_3$ and 6.89 g (71.7 mmol) of NaOtBu, and the mixture is heated under reflux for 2 hours. The obtained product is separated and purified by silica gel column chromatography (toluene: hexane = volume ratio of 1:4) to obtain 3.1 g of Compound 2-1B (9,9-dimethyl-10-(selenophen-2-yl)-9,10-dihydroacridine) (Yield: 39.5%).

(iii) Synthesis of Compound 2-1C

1.7 ml of phosphoryl chloride is added dropwise to 4.4 ml of N,N-dimethylformamide at 0 °C and stirred at room temperature (24 °C) for 1 hour. The mixture is slowly added dropwise to 90 ml of 3.00 g of Compound 2-1B dissolved in dichloromethane at 0 °C, followed by stirring at room temperature for 1 hour. 100 ml of water is added thereto, and an aqueous sodium hydroxide solution is added thereto until the pH value becomes 14, followed by stirring at room temperature for 2 hours. The organic layer extracted with dichloromethane is washed with an aqueous sodium chloride solution, and then dried with anhydrous magnesium sulfate. The obtained product is separated and purified by silica gel column chromatography (volume ratio is changed from dichloromethane: hexane = volume ratio of 3:2 to 100% of dichloromethane) to obtain 2.90 g (Yield: 89.3%) of Compound 2-1C (5-(9,9-dimethylacridin-10(9H)-yl)selenophene-2-carbaldehyde).

(iv) Synthesis of Compound 2-1D

1.00 g (2.73 mmol) of the obtained Compound 2-1C is suspended in ethanol, and 0.440 g (30.1 mmol) of 1H-indene-1,3(2H)-dione is added thereto and reacted at 50 °C for 2 hours, followed by filtering with a silica gel filter. Thereafter, 1.05 g (Yield: 77.8%) of the compound represented by Chemical Formula 2-1, also referred to here as "Compound 2-1D", is obtained by recrystallization from chloroform and ethanol. The obtained compound is purified by sublimation to a purity of 99.9%.

$^1$H-NMR (300 MHz, $CDCl_3$): δ 7.9 (s, 1H), 7.8 (m, 5H), 7.7 (m, 2H), 7.6 (d, 2H), 7.4 (m, 4H), 7.0 (d, 1H), 1.6 (s, 6H).

**Reference Synthesis Example 2: Synthesis of Compound Represented by Chemical Formula 2-2**

[Chemical Formula 2-2]

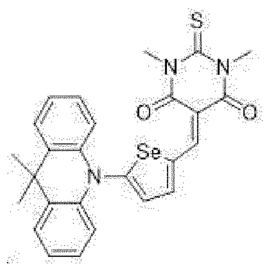

A compound represented by Chemical Formula 2-2 is synthesized in the same manner as in Reference Synthesis Example 1, except that 1,3-dimethyl-2-thiobarbituric acid is used instead of 1H-indene-1,3(2H)-dione in step (iv) of Reference Synthesis Example 1.

$^1$H-NMR (300 MHz, $CDCl_3$): δ 8.5 (s, 1H), 77.9 (d, 1H), 7.8 (d, 2H), 7.6 (d, 2H), 7.4 (m, 4H), 7.1 (d, 1H), 3.8 (d, 6H), 1.6 (s, 6H).

**Reference Synthesis Example 3: Synthesis of Compound Represented by Chemical Formula 2-3**

[Chemical Formula 2-3]

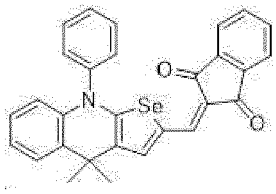

2.0 g (Yield: 70%) of a compound represented by Chemical Formula 2-3 is synthesized in the same manner as in Synthesis Example 2 except that diphenyl amine is used instead of 9,9-dimethyl-9,10-dihydroacridine in step (i) of Synthesis Example 2.

$^1$H-NMR (300 MHz, CD2Cl2): δ 7.86 (m, 6H), 7.35 (m, 4H), 7.23 (m, 6H), 4.99 (s, 1H), 4.86 (s, 1H), 1.81 (s, 3H).

**Reference Synthesis Example 4: Synthesis of Compound Represented by Chemical Formula 2-4**

[Chemical Formula 2-4]

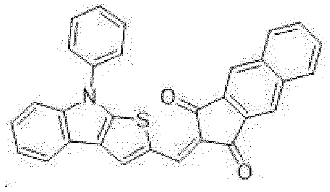

[Reaction Scheme 2-4]

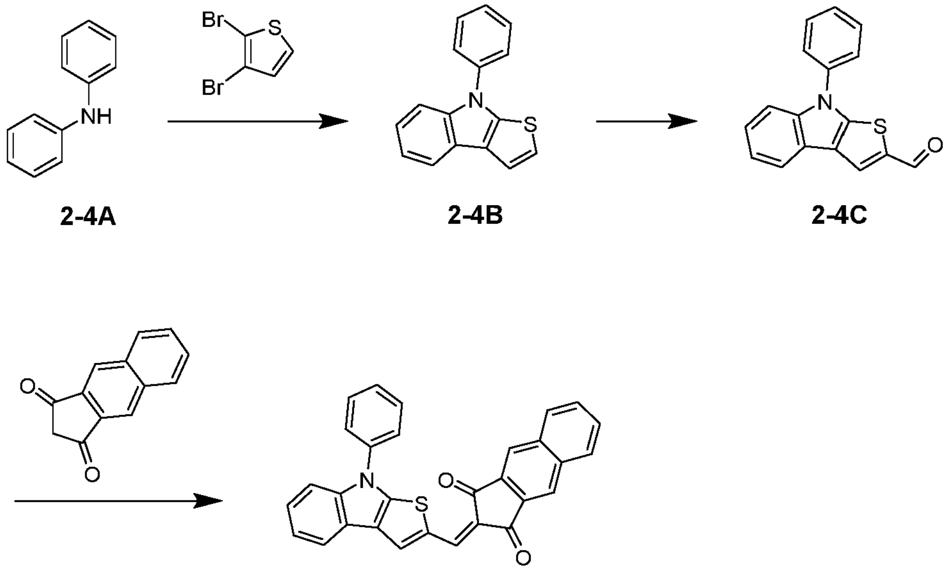

2-4A 2-4B 2-4C

2-4D

As in Reaction Scheme 2-4, diphenylamine and 2,3-dibromothiophene are used to synthesize Compound 2-4B with reference to Angewante chem., Int. Ed. Compound 2-4B 2007, 46, 1627-1629. Then, 0.74 ml of phosphoryl chloride

is added dropwise to 2.3 ml of N,N-dimethylformamide at 0 °C, followed by stirring at room temperature (24 °C) for 1 hour. Accordingly, a solution of 2.1 g of Compound 2-4B dissolved in 2.0 ml of dimethylformamide is slowly added dropwise thereto at 0 °C and stirred at 80 °C for 2 hours. The resultant is cooled down to room temperature (25 °C) and neutralized with ammonium acetate. After filtering and removing solids precipitated therein, the filtrate is extracted with toluene and dried with magnesium sulfate, and a compound in an oil state is obtained therefrom and then, separated by using dichloromethane through silica gel column chromatography to obtain Compound 2-4C (1.1 g, Yield: 48%).

Subsequently, 1.0 g of Compound 2-4C and 0.9 g of 1H-cyclopenta[b]naphthalene-1,3(2H)-dione are dissolved in 50 ml of ethanol, and three drops of piperidine are added thereto and then, heated under reflux for 3 hours. After removing the solvent under a reduced pressure, the residue was purified through silica gel column chromatography to obtain 1.0 g (Yield: 75%) of a compound represented by Chemical Formula 2-4, also shown herein as "Compound 2-4D".

**Example 1: Manufacture of Photoelectric Device**

ITO is laminated on a glass substrate through sputtering to form an about 150 nm-thick anode, and the ITO glass substrate is ultrasonic wave-cleaned with acetone/isopropyl alcohol/pure water respectively for 15 minutes and then, UV ozone-cleaned. Subsequently, the compound according to Synthesis Example 1 and C60 are codeposited in a volume ratio of 1.2:1 on the ITO glass substrate to form a 120 nm-thick active layer, and ITO is vacuum-deposited on the active layer to be 7 nm thick to manufacture a photoelectric device having a structure of ITO (150 nm)/active layer (120 nm)/ITO (7 nm).

**Examples 2 to 4 and Reference Examples 1 to 4: Manufacture of Photoelectric Device**

Photoelectric devices according to Examples 2 to 4 and Reference Examples 2 to 4 are manufactured according to the same method as Example 1 except that each of compounds according to Synthesis Examples 2 to 4 and Reference Synthesis Examples 1 to 4 is respectively used instead of the compound of Synthesis Example 1.

**Evaluation 1: Light Absorption Characteristics of Compound**

Light absorption characteristics (a maximum absorption wavelength ($\lambda_{max}$), a full width at half maximum (FWHM), and an absorption coefficient) of the compounds according to Synthesis Examples 1 to 4 depending on a wavelength are evaluated. Each compound according to Synthesis Examples 1 to 4 and C60 are deposited in a volume ratio of 1:1 to form 100 nm-thick thin films, and each thin film is evaluated with respect to light absorption characteristics in an ultraviolet (UV)-visible ray (UV-Vis) region by using Cary 5000 UV spectroscopy (Varian Inc.). The results are shown in Table 1.

(Table 1)

| Compound | $\lambda_{max}$ (nm) | FWHM (nm) | Absorption coefficient ($10^4$ $cm^{-1}$) |
|---|---|---|---|
| Synthesis Example 1 | 545 | 107 | 6.64 |
| Synthesis Example 2 | 570 | 108 | 6.09 |
| Synthesis Example 3 | 546 | 102 | 5.07 |
| Synthesis Example 4 | 552 | 113 | 5.66 |

Referring to Table 1, the compounds of Synthesis Examples 1 to 4 exhibit a maximum absorption wavelength in a green wavelength region, a small full width at half maximum (FWHM), and high absorption coefficient. Accordingly, the compounds of Synthesis Examples 1 to 4 exhibit excellent absorption selectivity in the green wavelength region.

**Evaluation 2: Thermal Stability of Compound**

Thermal stability of the compounds according to Synthesis Examples 1 to 4 is evaluated by measuring a melting point ($T_m$) and a deposition temperature thereof. The deposition temperature is measured through a thermogravimetric analysis (TGA), and deposition characteristics are evaluated from a weight decrease according to a temperature increase by subliming the compounds under high vacuum of less than or equal to 10 Pa. The results are shown in Table 2.

(Table 2)

| | $T_m$ (°C) | $T_{s10}$ (10 wt%, 10 Pa) (°C) | △T (Tm - $T_{s10}$) (°C) |
|---|---|---|---|
| Synthesis Example 1 | 306 | 218 | 88 |
| Synthesis Example 2 | 345 | 245 | 100 |
| Synthesis Example 3 | 337 | 257 | 80 |
| Synthesis Example 4 | 302 | 213 | 89 |
| * $T_{s10}$ (10 wt%) (°C): A temperature at which a weight of a sample decreases by 10 wt% | | | |

Referring to Table 2, the compounds according to Synthesis Examples 1 to 4 exhibit sufficient thermal stability.

**Evaluation 3: Quantum Efficiencies of Photoelectric Device**

Quantum efficiencies of the organic photoelectric devices according to Examples 1 to 4 and Reference Examples 1 to 4 depending on a wavelength and a voltage are evaluated.

The quantum efficiencies are measured by using IPCE Measurement System (McScience Inc., Korea). First, the IPCE Measurement System is calibrated by using an Si photodiode (Hamamatsu Photonics K.K., Japan) and then, mounted on the photoelectric devices according to Examples 1 to 4 and Reference Examples 1 to 4, and internal quantum efficiency (IQE) and external quantum efficiency (EQE) thereof within a wavelength range of about 350 to about 750 nm are measured at room temperature. In addition, after annealing the photoelectric devices at 180 °C and 200 °C, external quantum efficiency thereof is measured in the aforementioned method. Among them, the results of Examples 1 to 3 and Reference Examples 1 to 4 are shown in Table 3.

(Table 3)

| | IQE (room temperature, %) | EQE (room temperature, %) | EQE (180 °C, %) | EQE (200 °C, %) |
|---|---|---|---|---|
| Example 1 | 86 | 65 | 60 | 59 |
| Example 2 | 84 | 61 | 59 | 58 |
| Example 3 | 79 | 50 | 49 | 49 |
| Reference Example 1 | 87 | 50 | Not measurable | Not measurable |
| Reference Example 2 | 80 | 63 | Not measurable | Not measurable |
| Reference Example 3 | 78 | 60 | 53 | Not measurable |
| Reference Example 4 | 63 | 35 | Not measurable | Not measurable |

Referring to Table 3, the photoelectric devices according to Examples 1 to 3 exhibit excellent (internal and external) quantum efficiencies at room temperature and after the annealing at a temperature of greater than or equal to 180 °C as well as room temperature. In contrast, the photoelectric device of Reference Example 3 exhibits deteriorated characteristics after annealing at 200 °C, and thus EQE is not measured. The photoelectric devices according to Reference Example 1, Reference Example 2, and Reference Example 4 exhibits deteriorated characteristics after annealing at 180 °C, and 200 °C, and thus EQE is not measured.

**Evaluation 4: Residual Charge Characteristics of Photoelectric Device**

When photoelectrically converted charges are not all used for signal treatment but remain in one frame, the charges in the former frame are overlapped and read with charges in the following frame, and herein, an amount of the charges in the following frame is called to be an amount of residual charges. The amount of the residual charges is

measured by irradiating light in the green wavelength region of 532 nm where the photoelectric conversion may occur for desired and/or alternatively predetermined time, turning off the light, and integrating the current measured in units of $10^{-6}$ seconds with an oscilloscope equipment, by time. The amount of the residual charges is evaluated by a ea/$\mu m^2$ unit based on 5000 lux light.
Table 4 shows measurement results of residual charges at room temperature of the photoelectric devices according to Examples 1 to 4 and Reference Examples 1 to 3. In addition, after the photoelectric devices according to Examples 1 to 3 and Reference Examples 1 to 3 are annealed at 180 °C and 200 °C, residual charges are measured and the results are shown in Table 5.

(Table 4)

| | Residual charge (room temperature, ea/$\mu m^2$) |
|---|---|
| Example 1 | 20 |
| Example 2 | 36 |
| Example 3 | 32 |
| Example 4 | 20 |
| Reference Example 1 | 508 |
| Reference Example 2 | 550 |
| Reference Example 3 | 54 |

(Table 5)

| | Residual charge (180 °C, ea/$\mu m^2$) | Residual charge (200 °C, ea/$\mu m^2$) |
|---|---|---|
| Example 1 | 15 | 12 |
| Example 2 | 12 | 18 |
| Example 3 | 16 | 17 |
| Reference Example 1 | Not measurable | Not measurable |
| Reference Example 2 | Not measurable | Not measurable |
| Reference Example 3 | 26 | Not measurable |

Referring to Tables 4 and 5, the photoelectric devices according to Examples 1 to 4 exhibit low residual charges at room temperature and high temperature. In contrast, the photoelectric devices according to Reference Examples 1 to 3 exhibit high residual charge at room temperature, and the residual charges at high temperature of the photoelectric devices according to Reference Examples 1 to 3 cannot be measured because device characteristics of Reference Examples 1 and 2 are deteriorated at 180 °C, and device characteristics of Reference Example 3 are deteriorated at 200 °C.

**Evaluation 5: Dark Current of Photoelectric Device**

The dark current (DC) of the photoelectric devices according to Examples 1 to 3 and Reference Examples 1 to 4 are evaluated. The dark current is measured by using the IPCE measurement system (McScience Inc., Korea). After calibrating the equipment with the Si photodiode (Hamamatsu Photonics K.K., Japan), the photoelectric devices according to Examples 1 to 3 and Reference Examples 1 to 4 are mounted on the equipment and measured with respect to the dark current in a wavelength region ranging from about 350 nm to 750 nm.
In addition, after annealing the photoelectric devices according to Examples 1 to 3 and Reference Examples 1 to 4 at 180 °C for 3 hours, 190 °C for 3 hours, and 200 °C for 3 hours, respectively, in the wavelength region ranging from about 350 nm to about 750 nm to measure the dark current using the IPCE measurement system.
Table 6 shows the results of Examples 1 to 3 and Reference Examples 1 to 4. In Table 6, the dark current is the measured value at the maximum absorption wavelength when a voltage of -3 V is applied.

(Table 6)

| | Dark current (180 °C, h/s/μm$^2$) | Dark current (190 °C, h/s/μm$^2$) | Dark current (200 °C, h/s/μm$^2$) |
|---|---|---|---|
| Example 1 | 1.6 | 1 | 71 |
| Example 2 | 5 | 2 | 1 |
| Example 3 | 50 | 5 | 5 |
| Reference Example 1 | Not measurable | Not measurable | Not measurable |
| Reference Example 2 | Not measurable | Not measurable | Not measurable |
| Reference Example 3 | 2 | Not measurable | Not measurable |
| Reference Example 4 | Not measurable | Not measurable | Not measurable |

Referring to Table 6, the photoelectric devices according to Examples 1 to 3 exhibit low dark current at high temperature. On the contrary, the device characteristics of Reference Example 3 are deteriorated after annealing at 190 °C or higher temperature and the device characteristics of Reference Example 1, Reference Example 2, and Reference Example 4 are deteriorated after annealing at 180 °C or higher temperature, dark current cannot be measured.

**Evaluation 6: Mobility of Photoelectric Device**

Charge mobility is evaluated by measuring TDCF (time-delayed collection field) mobility. The photoelectric devices according to Examples 1 to 4 and Reference Example 1 are irradiated by a laser of 550 nm (a pulse width: 6 nm) with a light source, and then, a bias voltage (V) is applied thereto to measure a photocurrent. Equation 2 is used to obtain the TDCF mobility.

[Equation 2]

$$\text{Mobility} = (T)^2/(t*V)$$

In Equation 2,
T is a thickness of an active layer (e.g., in cm), t is a period of taken from the time the laser is irradiated to the time when the photo current is maximized (e.g., in seconds), and V is the bias voltage (e.g., in Volts).
TDCF mobility results of the photoelectric devices according to Examples 1 to 4 and Reference Example 1 are shown in Table 7.

(Table 7)

| | TDCF mobility (cm$^2$/V·sec) |
|---|---|
| Example 1 | $2.6 \times 10^{-5}$ |
| Example 2 | $2.1 \times 10^{-5}$ |
| Example 3 | $1.3 \times 10^{-5}$ |
| Example 4 | $3.3 \times 10^{-5}$ |
| Reference Example 1 | $6.8 \times 10^{-6}$ |

Referring to Table 7, the photoelectric devices according to Examples 1 to 4 exhibit improved mobility, compared with the photoelectric device according to Reference Example 1.
While the inventive concepts have been described in connection with what is presently considered to be practical example embodiments, it is to be understood that the inventive concepts are not limited to such example embodiments.

On the contrary, the inventive concepts are intended to cover various modifications included within the scope of the appended claims.

**<Description of symbols>**

| | | | | | |
|---|---|---|---|---|---|
| 10: | first electrode | 20: | second electrode | | |
| 30: | active layer | 40, 45: | charge auxiliary layer | | |
| 100, 200: | photoelectric device | 300, 400, 500, 600: | organic CMOS image sensor | | |
| 310: | semiconductor substrate | 70B, 72B: | blue filter | 70R, 72R: | redfilter |
| 70, 72: | color filter layer | 85: | through-hole | | |
| 60: | lower insulation layer | 80: | upper insulation layer | | |
| 50B, 50R: | photo-sensing device | 55: | charge storage | | |
| 1000: | digital camera | 1010: | lens | | |
| 1020: | image sensor | 1030: | motor | | |
| 1040: | engine | 1050: | host/application | | |

## Claims

**1.** A composition for a photoelectric device, the composition comprising:

a p-type semiconductor compound represented by Chemical Formula 1 and an n-type semiconductor compound:

[Chemical Formula 1]

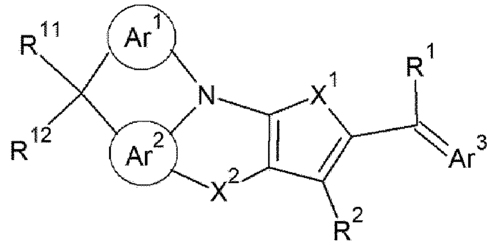

wherein, in Chemical Formula 1,

$Ar^1$ and $Ar^2$ are each independently a substituted or unsubstituted C6 to C30 arene group, a substituted or unsubstituted C3 to C30 heteroarene group, or a condensed ring thereof,
$X^1$ is -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ is linked to each other to provide a ring structure,
$X^2$ is -O-, -S-, -Se-, -Te-, -S(=O)-, -S(=O)2-, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-(CR^fR^g)_{n1}-$, $-(CR^{ff}R^{gg})-$, $-(C(R^m)=C(R^n))-$, - $(C(R^{mm})=C(R^{nn}))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and

$R^{ee}$, $R^{ff}$ and $R^{gg}$, or $R^{mm}$ and $R^{nn}$ are linked to each other to provide a ring structure, and n1 of $-(CR^fR^g)_{n1}-$ is 1 or 2,

$R^{11}$ and $R^{12}$ are each independently hydrogen, deuterium, a halogen, a cyano group, a substituted or unsubstituted C1 to C20 alkyl group, a substituted or unsubstituted C6 to C20 aryl group, or a substituted or unsubstituted C6 to C20 aryloxy group, wherein $R^{11}$ and $R^{12}$ are each independently present or are linked to each other to provide a ring structure, or $-CR^{11}R^{12}-$ is optionally linked with $Ar^1$ or $Ar^2$ to provide a ring structure

$Ar^3$ is a substituted or unsubstituted C6 to C30 hydrocarbon cyclic group having at least one functional group selected from C=O, C=S, C=Se, and C=Te, a substituted or unsubstituted C2 to C30 heterocyclic group having at least one functional group selected from C=O, C=S, C=Se, and C=Te, or a fused ring thereof, and

$R^1$ and $R^2$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group ($-SF_5$), a hydroxyl group, an amine group, a hydrazine group, a hydrazone group, a carboxyl group or a salt thereof, a sulfonic acid group or a salt thereof, a phosphoric acid group or a salt thereof, $-SiR^aR^bR^c$, wherein $R^a$, $R^b$, and $R^c$ are each independently hydrogen or a substituted or unsubstituted C1 to C10 alkyl group, or any combination thereof.

**2.** The composition of claim 1, wherein the p-type semiconductor compound of Chemical Formula 1 is represented by Chemical Formula 2A:

[Chemical Formula 2A]

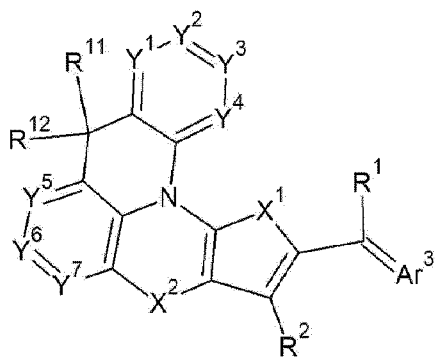

wherein, in Chemical Formula 2A,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and

$Y^1$ to $Y^7$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof;

preferably wherein

(i) in Chemical Formula 2A, $Y^4$ is N or $CR^k$, wherein $R^k$ is a halogen, a cyano group, a C1 to C10 haloalkyl group, or a C1 to C10 cyanoalkyl group, or

in Chemical Formula 2A, $Y^7$ is N or $CR^k$, wherein $R^k$ is a halogen, a cyano group, a C1 to C10 haloalkyl group, or a C1 to C10 cyanoalkyl group, and $X^2$ is -O-, - S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ are each independently a halogen, a C1 to C20 haloalkyl group, or a C1 to C20 cyanoalkyl group, and n1 of $-(CR^fR^g)_{n1}-$ is 1 or 2; or

(ii) the p-type semiconductor compound of Chemical Formula 2A is represented by Chemical Formula 2A-1 or

Chemical Formula 2A-2:

[Chemical Formula 2A-1]

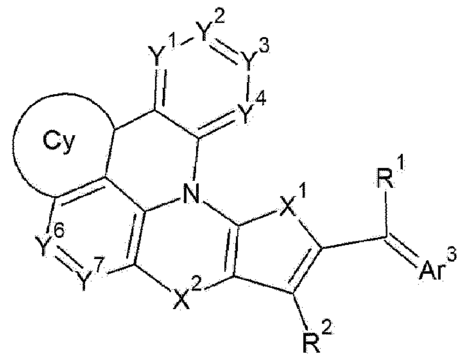

wherein, in Chemical Formula 2A-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^1$ to $Y^4$, $Y^6$, and $Y^7$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof,

[Chemical Formula 2A-2]

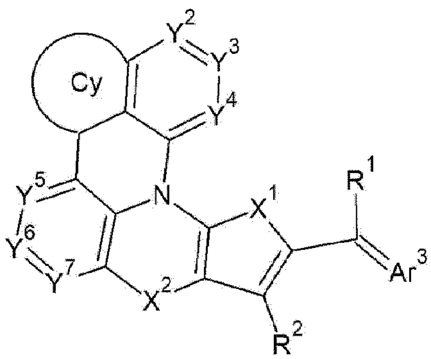

wherein, in Chemical Formula 2A-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$Y^2$ to $Y^7$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

**3.** The composition of claim 1, wherein the p-type semiconductor compound of Chemical Formula 1 is represented by Chemical Formula 2B:

[Chemical Formula 2B]

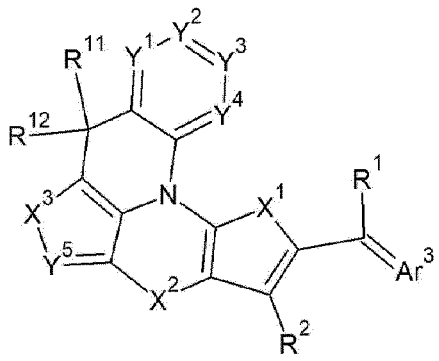

wherein, in Chemical Formula 2B,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1,
$Y^1$ to $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
$X^3$ is -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ are linked to each other to provide a ring structure;

preferably wherein

(i) in Chemical Formula 2B, $Y^4$ is N or $CR^k$, wherein $R^k$ is a halogen, a cyano group, a C1 to C10 haloalkyl group, or a C1 to C10 cyanoalkyl group, or
in Chemical Formula 2B, $Y^5$ is N or $CR^k$, wherein $R^k$ is a halogen, a cyano group, a C1 to C10 haloalkyl group, or a C1 to C10 cyanoalkyl group and $X^2$ is -O-, - S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, - $(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ are each independently a halogen, a C1 to C20 haloalkyl group, or a C1 to C20 cyanoalkyl group, and n1 of $-(CR^fR^g)_{n1}-$ is 1 or 2; or
(ii) the p-type semiconductor compound of Chemical Formula 2B is represented by Chemical Formula 2B-1 or Chemical Formula 2B-2:

[Chemical Formula 2B-1]

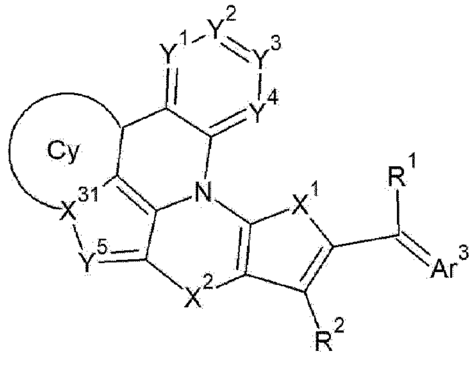

wherein, in Chemical Formula 2B-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^{31}$ is N, B, $SiR^b$, $GeR^d$, $CR^f$, Si, Ge, or C, wherein $R^b$, $R^d$, and $R^f$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,
$Y^1$ to $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof,

[Chemical Formula 2B-2]

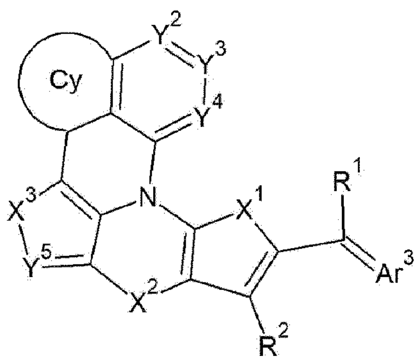

wherein, in Chemical Formula 2B-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ and $Y^5$ are the same as $X^3$ and $Y^5$, respectively, in Chemical Formula 2B,
$Y^2$ to $Y^4$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

**4.** The composition of claim 1, wherein the p-type semiconductor compound of Chemical Formula 1 is represented by Chemical Formula 2C:

[Chemical Formula 2C]

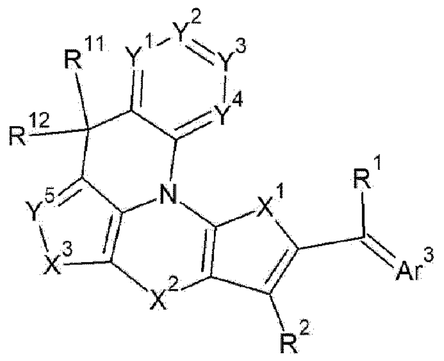

wherein, in Chemical Formula 2C,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1,
$Y^1$ to $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
$X^3$ is -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ is linked to each other to provide a ring structure;

preferably wherein

(i) in Chemical Formula 2C, $Y^4$ is N or $CR^k$, wherein $R^k$ is a halogen, a cyano group, a C1 to C10 haloalkyl group, or a C1 to C10 cyanoalkyl group), or
in Chemical Formula 2C, $X^3$ is -O-, -S-, -Se-, -Te-, -S(=O)-, $-S(=O)_2-$, $-NR^{a1}-$, $-BR^{a2}-$, $-SiR^bR^c-$, $-GeR^dR^e-$, or $-CR^fR^g-$, wherein $R^{a1}$, $R^{a2}$, $R^b$, $R^c$, $R^d$, $R^e$, $R^f$, and $R^g$ are each independently a halogen, a C1 to C20 haloalkyl group, or a C1 to C20 cyanoalkyl group, and $X^2$ is -O-, -S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, $-(C(R^m)=C(R^n))-$, or $-(C(R^p)=N)-$, wherein $R^{a1}$, $R^{a2}$, $R^b$, $R^c$, $R^d$, $R^e$, $R^f$, $R^g$, $R^m$, $R^n$, and $R^p$ are each independently a halogen, a C1 to C20 haloalkyl group, or a C1 to C20 cyanoalkyl group, and n1 of $-(CR^fR^g)_{n1}-$ is 1 or 2; or
(ii) the p-type semiconductor compound is represented by Chemical Formula 2C-1 or Chemical Formula 2C-2:

[Chemical Formula 2C-1]

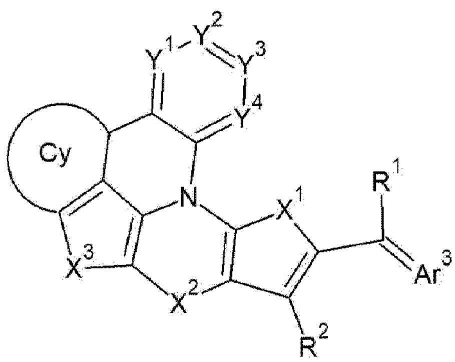

wherein, in Chemical Formula 2C-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2C,
$Y^1$ to $Y^4$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof,

[Chemical Formula 2C-2]

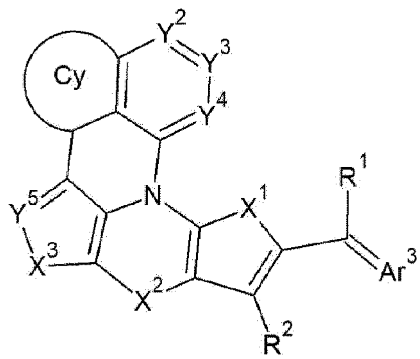

wherein, in Chemical Formula 2C-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ and $Y^5$ are the same as $X^3$ and $Y^5$, respectively, in Chemical Formula 2C,
$Y^2$ to $Y^4$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

**5.** The composition of claim 1, wherein the p-type semiconductor compound of Chemical Formula 1 is represented by Chemical Formula 2D:

[Chemical Formula 2D]

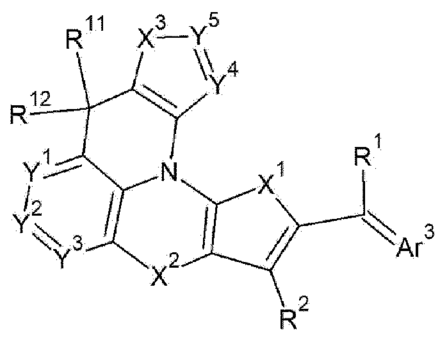

wherein, in Chemical Formula 2D,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1,
$Y^1$ to $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
$X^3$ is -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ is linked to each other to provide a ring structure;

preferably wherein

(i) in Chemical Formula 2D, $Y^4$ is N or $CR^k$, wherein $R^k$ is a halogen, a cyano group, a C1 to C10 haloalkyl group, or a C1 to C10 cyanoalkyl group, or
in Chemical Formula 2D, $Y^3$ is N or $CR^k$, wherein $R^k$ is a halogen, a cyano group, a C1 to C10 haloalkyl group, or a C1 to C10 cyanoalkyl group and $X^2$ is -O-, - S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, - $(C(R^m)=C(R^n))$-, or -$(C(R^p)=N)$-, wherein $R^{a1}$, $R^{a2}$, $R^b$, $R^c$, $R^d$, $R^e$, $R^f$, $R^g$, $R^m$, $R^n$, and $R^p$ are each independently a halogen, a C1 to C20 haloalkyl group, or a C1 to C20 cyanoalkyl group, and n1 of $-(CR^fR^g)_{n1}-$ is 1 or 2; or
(ii) the p-type semiconductor compound is represented by Chemical Formula 2D-1 or Chemical Formula 2D-2:

[Chemical Formula 2D-1]

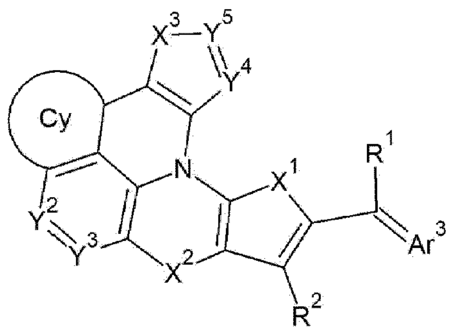

wherein, in Chemical Formula 2D-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2D,
$Y^2$ to $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof,

[Chemical Formula 2D-2]

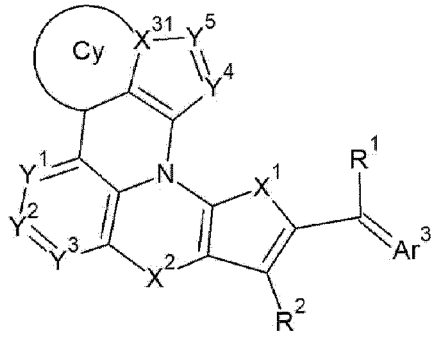

wherein, in Chemical Formula 2D-2,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^{31}$ is N, B, $SiR^b$, $GeR^d$, $CR^f$, Si, Ge, or C, wherein $R^b$, $R^d$, and $R^f$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,
$Y^1$ to $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

**6.** The composition of claim 1, wherein the p-type semiconductor compound of Chemical Formula 1 is represented by Chemical Formula 2E:

[Chemical Formula 2E]

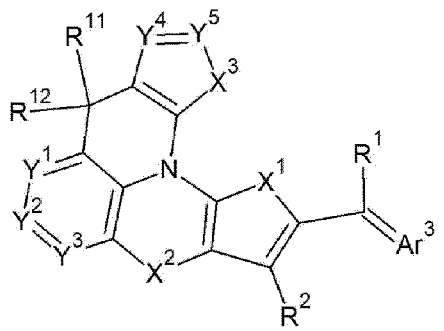

wherein, in Chemical Formula 2E,

$X^1$, $X^2$, $Ar^3$, $R^1$, $R^2$, $R^{11}$, and $R^{12}$ are the same as $X^1$, $X^2$, $Ar^3$, $R^1$, $R^2$, $R^{11}$, and $R^{12}$, respectively, in Chemical Formula 1, and
$X^3$ is -O-, -S-, -Se-, -Te-, -S(=O)-, -S(=O)2-, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-CR^fR^g-$, or $-CR^{ff}R^{gg}-$, wherein $R^{a1}$, $R^{a2}$, $R^b$, $R^c$, $R^d$, $R^e$, $R^f$, and $R^g$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$, $R^{dd}$ and $R^{ee}$, or $R^{ff}$ and $R^{gg}$ is linked to each other to provide a ring structure, and
$Y^1$ to $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a

substituted or unsubstituted C6 to C30 arene group, a substituted or unsubstituted C3 to C30 heteroarene group, or a condensed ring thereof;

preferably wherein

(i) in Chemical Formula 2E, $X^3$ is -O-, -S-, -Se-, -Te-, -S(=O)-, $-S(=O)_2-$, - $NR^{a1}$-, -$BR^{a2}$-, -$SiR^bR^c$-, -$GeR^dR^e$-, or -$CR^fR^g$-, wherein $R^{a1}$, $R^{a2}$, $R^b$, $R^c$, $R^d$, $R^e$, $R^f$, and $R^g$ are each independently a halogen, a C1 to C20 haloalkyl group, or a C1 to C20 cyanoalkyl group, or
in Chemical Formula 2E, $Y^3$ is N or $CR^k$, wherein $R^k$ is a halogen, a cyano group, a C1 to C10 haloalkyl group, or a C1 to C10 cyanoalkyl group, and $X^2$ is -O-, - S-, -Se-, -Te-, -S(=O)-, $-S(=O)_2-$, -$NR^{a1}$-, -$BR^{a2}$-, -$SiR^bR^c$-, -$GeR^dR^e$-, $-(CR^fR^g)_{n1}-$, - $(C(R^m)=C(R^n))$-, or -$(C(R^p)=N)$-, wherein $R^{a1}$, $R^{a2}$, $R^b$, $R^c$, $R^d$, $R^e$, $R^f$, $R^g$, $R^m$, $R^n$, and $R^p$ are each independently a halogen, a C1 to C20 haloalkyl group, or a C1 to C20 cyanoalkyl group, and n1 of $-(CR^fR^g)_{n1}-$ is 1 or 2; or
(ii) the p-type semiconductor compound is represented by Chemical Formula 2E-1 or Chemical Formula 2E-2:

[Chemical Formula 2E-1]

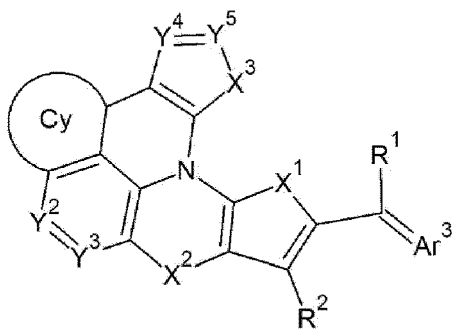

wherein, in Chemical Formula 2E-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2E,
$Y^2$ to $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof,

[Chemical Formula 2E-2]

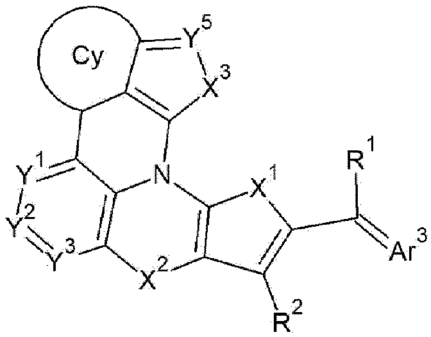

wherein, in Chemical Formula 2E-1,

$X^1$, $X^2$, $Ar^3$, $R^1$, and $R^2$ are the same as $X^1$, $X^2$, $Ar^3$, $R^1$, and $R^2$, respectively, in Chemical Formula 1,
$X^3$ is the same as $X^3$ in Chemical Formula 2E,
$Y^1$ to $Y^3$ and $Y^5$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^{k}$'s are linked to each other to provide a substituted or unsubstituted C6 to C30 arene group, a substituted or unsubstituted C3 to C30 heteroarene group, or a condensed ring thereof, and
Cy is a substituted or unsubstituted C6 to C30 arene group, a substituted or unsubstituted C3 to C30 heteroarene group, a substituted or unsubstituted C5 to C30 cycloalkene group, a substituted or unsubstituted C3 to C30 heterocycloalkene group, or a condensed ring thereof.

**7.** The composition of any of claims 1 to 6, wherein in $X^1$, $X^2$, and $-CR^{11}R^{12}-$ of Chemical Formula 1, each ring structure of the ring structure of $X^1$, the ring structure of $X^2$, and the ring structure of $-CR^{11}R^{12}-$ is a spiro structure or a fused ring structure;

preferably wherein the spiro structure comprises a moiety represented by Chemical Formula 3:

[Chemical Formula 3]

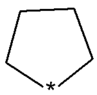
(1)
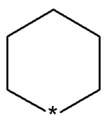
(2)
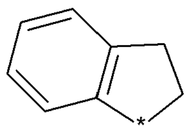
(3)
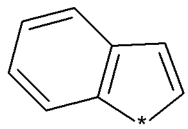
(4)
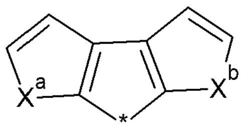
(5)
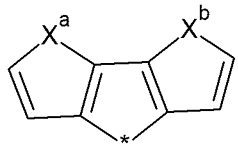
(6)
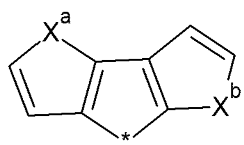
(7)
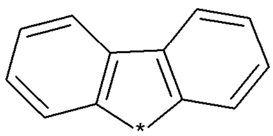
(8)
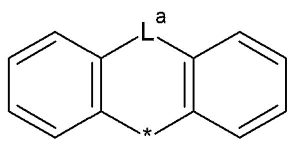
(9)
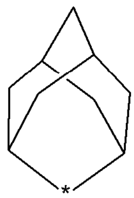
(10)

wherein, in Chemical Formula 3,

$X^a$ and $X^b$ are each independently -O-, -S-, -Se-, -Te-, -S(=O)-, - $S(=O)_2$-, $-NR^{a1}-$, $-BR^{a2}-$, $-SiR^bR^c-$, $-SiR^{bb}R^{cc}-$, $-GeR^dR^e-$, or $-GeR^{dd}R^{ee}-$, wherein $R^{a1}$, $R^{a2}$, $R^b$, $R^c$, $R^d$, and $R^e$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, a substituted or unsubstituted C6 to C20 aryloxy group, or a substituted or unsubstituted C3 to C20 heteroaryl group, and at least one pair of $R^{bb}$ and $R^{cc}$ or $R^{dd}$ and $R^{ee}$ is linked to each other to provide a ring structure,
$L^a$ is -O-, -S-, -Se-, -Te-, $-NR^{a1}-$, $-BR^{a2}-$, $-SiR^bR^c-$, $-GeR^dR^e-$, - $(CR^fR^g)_{n1}$-, $-(C(R^p)=N)-$, or a single bond, wherein $R^{a1}$, $R^{a2}$, $R^b$, $R^c$, $R^d$, $R^e$, $R^f$, $R^g$, and $R^p$ are each independently hydrogen, deuterium, a halogen, a cyano group, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and n1 of $-(CR^fR^g)_{n1}-$ is 1 or 2,
hydrogen of each ring is optionally replaced by at least one substituent selected from deuterium, a halogen,

a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group, and

one or more CH present in the aromatic ring of the moieties (3), (4), (5), (6), (7), (8), or (9) is optionally replaced by N.

**8.** The composition of claim 7, wherein at least one hydrogen of each ring is replaced by at least one substituent selected from deuterium, a halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

**9.** The composition of any of claims 1 to 8, wherein $Ar^3$ is a cyclic group represented by Chemical Formula 4:

[Chemical Formula 4]

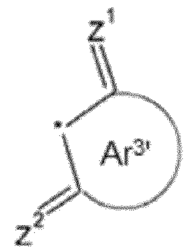

wherein, in Chemical Formula 4,

$Ar^{3'}$ is a substituted or unsubstituted C6 to C30 aryl group or a substituted or unsubstituted C3 to C30 heteroaryl group,

$Z^1$ is O, S, Se, or Te, and

$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group; or

wherein in Chemical Formula 1, $Ar^3$ is a cyclic group represented by one of Chemical Formula 5A to Chemical Formula 5G:

[Chemical Formula 5A]

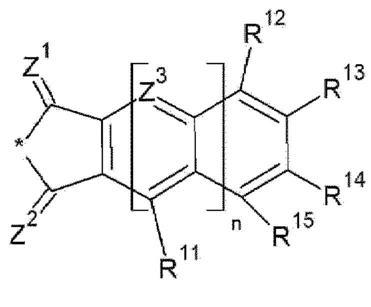

wherein, in Chemical Formula 5A,

$Z^1$ is O, S, Se, or Te,

$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,

$Z^3$ is N or $CR^c$, wherein $R^c$ is hydrogen, deuterium, or a substituted or unsubstituted C1 to C10 alkyl group,

$R^{11}$, $R^{12}$, $R^{13}$, $R^{14}$, and $R^{15}$ are each independently hydrogen, deuterium, a substituted or unsubstituted C1 to

C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ are each independently present or are linked to each other to provide a fused aromatic ring,
n is 0 or 1, and
* is a linking point,

[Chemical Formula 5B]

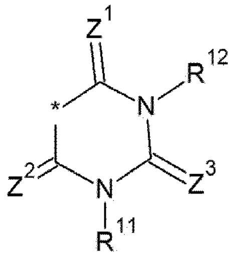

wherein, in Chemical Formula 5B,

$Z^1$ is O, S, Se, or Te,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ is O, S, Se, Te, and $C(R^a)(CN)$, wherein $R^a$ is hydrogen, a cyano group(-CN), or a C1 to C10 alkyl group,
$R^{11}$ and $R^{12}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* is a linking point,

[Chemical Formula 5C]

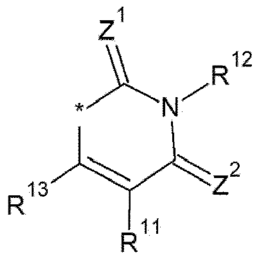

wherein, in Chemical Formula 5C,

$Z^1$ is O, S, Se, or Te,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* is a linking point,

[Chemical Formula 5D]

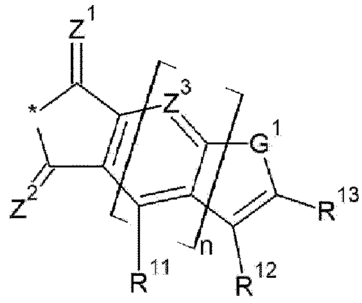

wherein, in Chemical Formula 5D,

$Z^1$ is O, S, Se, or Te,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ is N or $CR^c$, wherein $R^c$ is hydrogen or a substituted or unsubstituted C1 to C10 alkyl group,
$G^1$ is O, S, Se, Te, $SiR^xR^y$, or $GeR^zR^w$, wherein $R^x$, $R^y$, $R^z$, and $R^w$ are each independently hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, wherein $R^{12}$ and $R^{13}$ are each independently present or are linked to each other to provide a fused aromatic ring,
n is 0 or 1, and
* is a linking point,

[Chemical Formula 5E]

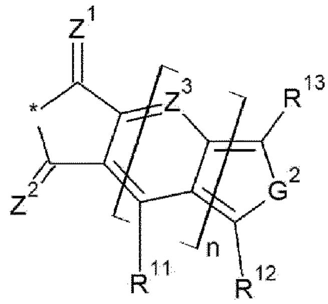

wherein, in Chemical Formula 5E,

$Z^1$ is O, S, Se, or Te,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$Z^3$ is N or $CR^c$, wherein $R^c$ is hydrogen or a substituted or unsubstituted C1 to C10 alkyl group,
$G^2$ is O, S, Se, Te, $SiR^xR^y$, or $GeR^zR^w$, wherein $R^x$, $R^y$, $R^z$, and $R^w$ are each independently hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group

group, or any combination thereof, and
* is a linking point,

[Chemical Formula 5F]

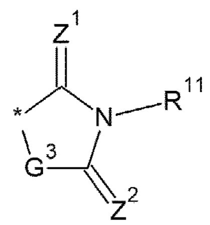

wherein, in Chemical Formula 5F,

$Z^1$ is O, S, Se, or Te,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group, provided that when $Z^2$ is $CR^aR^b$, at least one of $R^a$ or $R^b$ is a cyano group or a cyano-containing group,
$R^{11}$ is hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, and
$G^3$ is O, S, Se, Te, $SiR^xR^y$, or $GeR^zR^w$, wherein $R^x$, $R^y$, $R^z$, and $R^w$ are each independently hydrogen, deuterium, a halogen, a substituted or unsubstituted C1 to C20 alkyl group, or a substituted or unsubstituted C6 to C20 aryl group,

[Chemical Formula 5G]

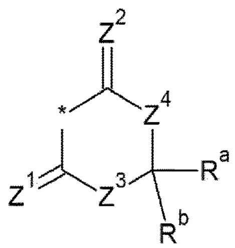

wherein, in Chemical Formula 5G,

$Z^1$ is O, S, Se, or Te,
$R^a$ and $R^b$ are independently hydrogen, a substituted or unsubstituted C1 to C10 alkyl group, a cyano group, or a cyano-containing group, and
$Z^2$ to $Z^4$ are each independently O, S, Se, Te, or $CR^cR^d$, wherein $R^c$ and $R^d$ are each independently hydrogen, a substituted or unsubstituted C1 to C10 alkyl group, a cyano group, or a cyano-containing group, provided that when $Z^2$ is $CR^cR^d$, at least one of $R^c$ or $R^d$ is a cyano group or a cyano-containing group.

**10.** The composition of any of claims 1 to 9, wherein the composition has a maximum absorption wavelength ($\lambda_{max}$) in a wavelength range of greater than or equal to 500 nm and less than or equal to 600 nm in a thin film state; and/or wherein the composition is configured to exhibit a light absorption curve having a full width at half maximum (FWHM) in a thin film state of 50 nm to 110 nm.

**11.** A photoelectric device (100, 200), comprising:

a first electrode (10) and a second electrode (20) facing each other, and
an active layer (30) between the first electrode and the second electrode wherein the active layer includes the composition of any of claims 1 to 10.

**12.** An image sensor (300, 400, 500, 600) comprising the photoelectric device of claim 11 preferably:

wherein the photoelectric device is a green photoelectric device configured to selectively absorb light in a green wavelength region,
the image sensor further includes a blue photoelectric device (50B) configured to selectively sense light in a blue wavelength region, and a red photoelectric device (50R) configured to selectively sense light in a red wavelength region,
wherein the green photoelectric device, the blue photoelectric device, and the red photoelectric device are stacked.

**13.** The image sensor of claim 12, further comprising:

a semiconductor substrate (310) integrated with a plurality of first photo-sensing devices (50B) configured to sense light in a blue wavelength region and a plurality of second photo-sensing devices (50R) configured to sense light in a red wavelength region
wherein the photoelectric device is on the semiconductor substrate and is configured to selectively sense light in a green wavelength region.

**14.** The image sensor of claim 13, further comprising:

a color filter layer (70, 72),
wherein the color filter layer includes a blue filter (70B, 72B) configured to selectively transmit light in the blue wavelength region and a red filter (70R, 72R) configured to selectively transmit light in the red wavelength region; and/or
wherein the plurality of first photo-sensing devices and the plurality of second photo-sensing devices are stacked in a vertical direction in the semiconductor substrate.

**15.** An electronic device comprising the image sensor of any of claims 12 to 14.

## Patentansprüche

**1.** Zusammensetzung für eine fotoelektrische Vorrichtung, wobei die Zusammensetzung Folgendes umfasst:

eine Halbleiterverbindung vom p-Typ, dargestellt durch die chemische Formel 1, und eine Halbleiterverbindung vom n-Typ:

[Chemische Formel 1]

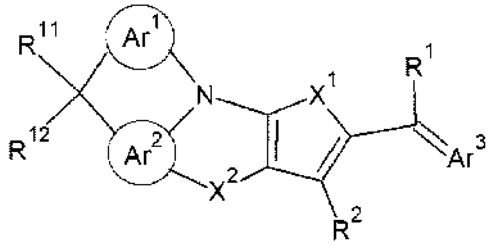

wobei in der chemischen Formel 1
$Ar^1$ und $Ar^2$ jeweils unabhängig eine substituierte oder unsubstituierte C6- bis C30-Arengruppe, eine substituierte oder unsubstituierte C3- bis C30-Heteroarengruppe oder ein kondensierter Ring davon sind,
$X^1$ -S-, -Se-, -Te-, -S(=O)-, -S(=O)$_2$-, -$NR^{a1}$-, -$BR^{a2}$-, -$SiR^bR^c$-, - $SiR^{bb}R^{cc}$-, -$GeR^dR^e$-, - $GeR^{dd}R^{ee}$-, -$CR^fR^g$- oder -$CR^{ff}R^{gg}$- ist, wobei $R^{a1}$, $R^{a2}$, $R^b$, $R^c$, $R^d$, $R^e$, $R^f$ und $R^g$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe, eine substituierte oder unsubstituierte C6- bis C20-Aryloxygruppe oder eine substituierte oder unsubstituierte C3- bis C20-Heteroarylgruppe sind und zumindest ein Paar von $R^{bb}$ und $R^{cc}$, $R^{dd}$ und $R^{ee}$ oder $R^{ff}$ und $R^{gg}$ miteinander verknüpft ist, um eine Ringstruktur bereitzustellen,

$X^2$ -O-, -S-, -Se-, -Te-, -S(=O)-, $-S(=O)_2-$, $-NR^{a1}-$, $-BR^{a2}-$, $-SiR^bR^c-$, - $SiR^{bb}R^{cc}-$, $-GeR^dR^e-$, $-GeR^{dd}R^{ee}-$, $-(CR^fR^g)_{n1}-$, $-(CR^{ff}R^{gg})-$, $-(C(R^m)=C(R^n))-$, $-(C(R^{mm})=C(R^{nn}))-$ oder $-(C(R^p)=N)-$ ist, wobei $R^{a1}$, $R^{a2}$, $R^b$, $R^c$, $R^d$, $R^e$, $R^f$, $R^g$, $R^m$, $R^n$ und $R^p$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe, eine substituierte oder unsubstituierte C6- bis C20-Aryloxygruppe oder eine substituierte oder unsubstituierte C3- bis C20-Heteroarylgruppe sind, und zumindest ein Paar von $R^{bb}$ und $R^{cc}$, $R^{dd}$ und $R^{ee}$, $R^{ff}$ und $R^{gg}$ oder $R^{mm}$ und $R^{nn}$ miteinander verknüpft sind, um eine Ringstruktur bereitzustellen, und n1 von $-(CR^fR^g)_{n1}-$ 1 oder 2 ist,

$R^{11}$ und $R^{12}$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C6- bis C20-Arylgruppe oder eine substituierte oder unsubstituierte C6- bis C20-Aryloxygruppe sind, wobei $R^{11}$ und $R^{12}$ jeweils unabhängig vorhanden sind oder miteinander verknüpft sind, um eine Ringstruktur bereitzustellen, oder $-CR^{11}R^{12}-$ optional mit $Ar^1$ oder $Ar^2$ verknüpft ist, um eine Ringstruktur bereitzustellen,

$Ar^3$ eine substituierte oder unsubstituierte cyclische C6- bis C30-Kohlenwasserstoffgruppe, die zumindest eine funktionelle Gruppe ausgewählt aus C=O, C=S, C=Se und C=Te aufweist, eine substituierte oder unsubstituierte heterocyclische C2- bis C30-Gruppe, die zumindest eine funktionelle Gruppe ausgewählt aus C=O, C=S, C=Se und C=Te aufweist, oder ein kondensierter Ring davon ist, und

$R^1$ und $R^2$ jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C3- bis C30-Heteroarylgruppe, eine substituierte oder unsubstituierte C2- bis C30-Acylgruppe, ein Halogen, eine Cyanogruppe (-CN), eine cyanohaltige Gruppe, eine Nitrogruppe, eine Pentafluorsulfanylgruppe ($-SF_5$), eine Hydroxylgruppe, eine Amingruppe, eine Hydrazingruppe, eine Hydrazongruppe, eine Carboxylgruppe oder ein Salz davon, eine Sulfonsäuregruppe oder ein Salz davon, eine Phosphorsäuregruppe oder ein Salz davon, - $SiR^aR^bR^c$, wobei $R^a$, $R^b$ und $R^c$ jeweils unabhängig Wasserstoff oder eine substituierte oder unsubstituierte C1- bis C10-Alkylgruppe sind, oder eine beliebige Kombination davon sind.

**2.** Zusammensetzung nach Anspruch 1, wobei die Halbleiterverbindung vom p-Typ der chemischen Formel 1 durch die chemische Formel 2A dargestellt ist:

[Chemische Formel 2A]

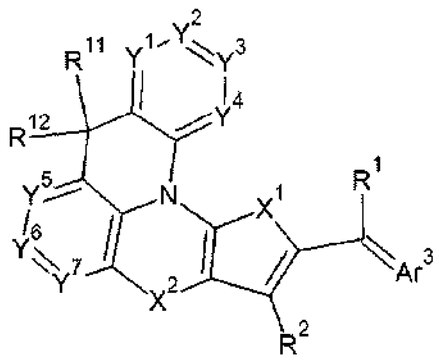

wobei in der chemischen Formel 2a

$X^1$, $X^2$, $Ar^3$, $R^1$, $R^2$, $R^{11}$ und $R^{12}$ jeweils die gleichen wie $X^1$, $X^2$, $Ar^3$, $R^1$, $R^2$, $R^{11}$ und $R^{12}$ in der chemischen Formel 1 sind, und

$Y^1$ bis $Y^7$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe sind oder benachbarte $R^k$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen,

wobei bevorzugt

(i) in der chemischen Formel 2A $Y^4$ N oder $CR^k$ ist, wobei $R^k$ ein Halogen, eine Cyanogruppe, eine C1- bis

C10-Haloalkylgruppe oder eine C1- bis C10-Cyanoalkylgruppe ist, oder
in der chemischen Formel 2A, $Y^7$, N oder $CR^k$ ist, wobei $R^k$ ein Halogen, eine Cyanogruppe, eine C1- bis C10-Haloalkylgruppe oder eine C1- bis C10-Cyanoalkylgruppe ist und $X^2$ -O-, - S-, -Se-, -Te- -S(=O)-, $-S(=O)_2-$ $-NR^{a1}-$, $-BR^{a2}-$, $-SiR^bR^c-$, $-GeR^dR^e-$, $-(CR^fR^g)_{n1}-$, - $(C(R^m)=C(R^n))$- oder $-(C(R^p)=N)$- ist, wobei $R^{a1}$, $R^{a2}$, $R^b$, $R^c$, $R^d$, $R^e$, $R^f$, $R^g$, $R^m$, $R^n$ und $R^p$ jeweils unabhängig ein Halogen, eine C1- bis C20-Haloalkylgruppe oder eine C1- bis C20-Cyanoalkylgruppe sind und n1 von $-(CR^fR^g)_{n1}-$ 1 oder 2 ist, oder
(ii) die Halbleiterverbindung vom p-Typ der chemischen Formel 2A durch die chemische Formel 2A-1 oder die chemische Formel 2A-2 dargestellt ist:

[Chemische Formel 2A-1]

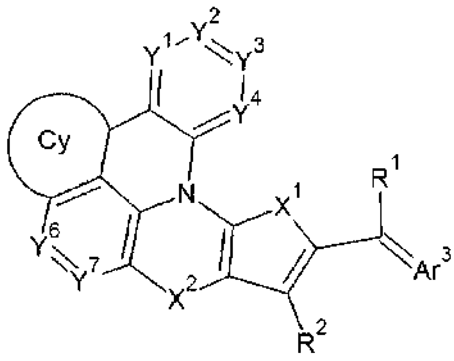

wobei in der chemischen Formel 2A-1,
$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,
$Y^1$ bis $Y^4$, $Y^6$ und $Y^7$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^{k'}$s
miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist,

[Chemische Formel 2A-2]

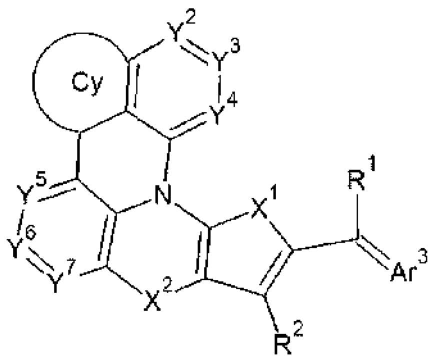

wobei in der chemischen Formel 2A-2,
$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,
$Y^2$ bis $Y^7$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist, oder benachbarte $R^{k'}$s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist.

**3.** Zusammensetzung nach Anspruch 1, wobei die Halbleiterverbindung vom p-Typ der chemischen Formel 1 durch die chemische Formel 2B dargestellt ist:

[Chemische Formel 2B]

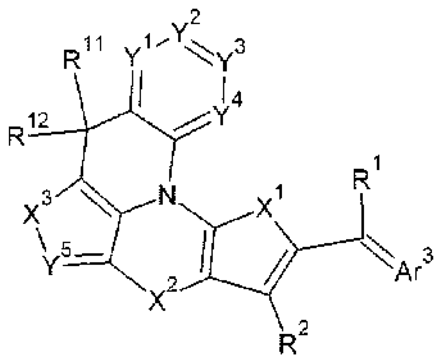

wobei in der chemischen Formel 2B,

$X^1$, $X^2$, $Ar^3$, $R^1$, $R^2$, $R^{11}$ und $R^{12}$ jeweils die gleichen wie $X^1$, $X^2$, $Ar^3$, $R^1$, $R^2$, $R^{11}$ und $R^{12}$ in der chemischen Formel 1 sind,
$Y^1$ bis $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^k$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
$X^3$ -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$ oder $-CR^{ff}R^{gg}-$ ist, wobei $R^{a1}$, $R^{a2}$, $R^b$, $R^c$, $R^d$, $R^e$, $R^f$ und $R^g$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe, eine substituierte oder unsubstituierte C6- bis C20-Aryloxygruppe oder eine substituierte oder unsubstituierte C3-bis C20-Heteroarylgruppe sind und zumindest ein Paar aus $R^{bb}$ und $R^{cc}$, $R^{dd}$ und $R^{ee}$ oder $R^{ff}$ und $R^{gg}$ miteinander verknüpft sind, um eine Ringstruktur bereitzustellen,
wobei bevorzugt

(i) in der chemischen Formel 2B $Y^4$ N oder $CR^k$ ist, wobei $R^k$ ein Halogen, eine Cyanogruppe, eine C1- bis C10-Halogenalkylgruppe oder eine C1- bis C10-Cyanoalkylgruppe ist, oder
in der chemischen Formel 2B, $Y^5$, N oder $CR^k$ ist, wobei $R^k$ ein Halogen, eine Cyanogruppe, eine C1- bis C10-Haloalkylgruppe oder eine C1- bis C10-Cyanoalkylgruppe ist und $X^2$ -O-, -S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, - $(C(R^m)=C(R^n))$- oder $-(C(R^p)=N)-$ ist, wobei $R^{a1}$, $R^{a2}$, $R^b$, $R^c$, $R^d$, $R^e$, $R^f$, $R^g$, $R^m$, $R^n$ und $R^p$ jeweils unabhängig ein Halogen, eine C1- bis C20-Haloalkylgruppe oder eine C1- bis C20-Cyanoalkylgruppe sind und n1 von $-(CR^fR^g)_{n1}-$ 1 oder 2 ist, oder
(ii) die Halbleiterverbindung vom p-Typ der chemischen Formel 2B durch die chemische Formel 2B-1 oder die chemische Formel 2B-2 dargestellt ist:

[Chemische Formel 2B-1]

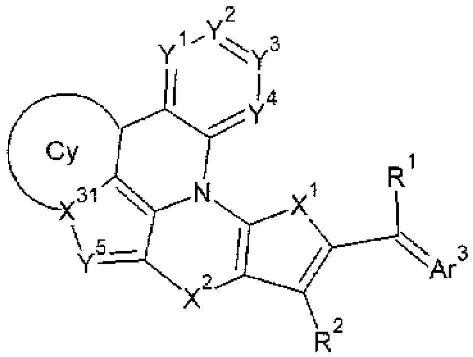

wobei in der chemischen Formel 2B-1,
$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,
$X^{31}$ N, B, $SiR^b$, $GeR^d$, $CR^f$, Si, Ge oder C ist, wobei $R^b$, $R^d$ und $R^f$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe oder eine substituierte oder unsubstituierte C6- bis C20-Aryloxygruppe sind,
$Y^1$ bis $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^k$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist,

[Chemische Formel 2B-2]

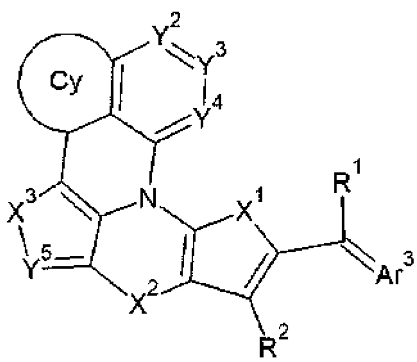

wobei in der chemischen Formel 2B-2,
$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,
$X^3$ und $Y^5$ jeweils die gleichen wie $X^3$ und $Y^5$ in der chemischen Formel 2B sind,
$Y^2$ bis $Y^4$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^k$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist.

**4.** Zusammensetzung nach Anspruch 1, wobei die Halbleiterverbindung vom p-Typ der chemischen Formel 1 durch die chemische Formel 2C dargestellt ist:

[Chemische Formel 2C]

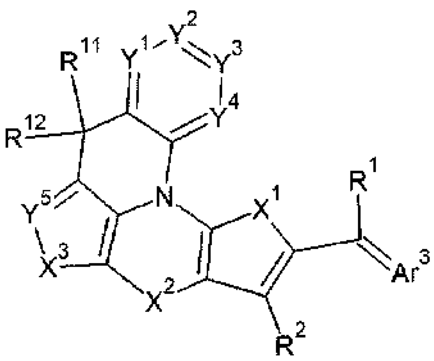

wobei in der chemischen Formel 2C,

$X^1$, $X^2$, $Ar^3$, $R^1$, $R^2$, $R^{11}$ und $R^{12}$ jeweils die gleichen wie $X^1$, $X^2$, $Ar^3$, $R^1$, $R^2$, $R^{11}$ und $R^{12}$ in der chemischen Formel 1 sind,
$Y^1$ bis $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^k$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
$X^3$ -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$ oder $-CR^{ff}R^{gg}-$ ist, wobei $R^{a1}$, $R^{a2}$, $R^b$, $R^c$, $R^d$, $R^e$, $R^f$ und $R^g$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe, eine substituierte oder unsubstituierte C6- bis C20-Aryloxygruppe oder eine substituierte oder unsubstituierte C3-bis C20-Heteroarylgruppe sind und zumindest ein Paar aus $R^{bb}$ und $R^{cc}$, $R^{dd}$ und $R^{ee}$ oder $R^{ff}$ und $R^{gg}$ miteinander verknüpft ist, um eine Ringstruktur bereitzustellen,
wobei bevorzugt

(i) in der chemischen Formel 2C $Y^4$ N oder $CR^k$ ist, wobei $R^k$ ein Halogen, eine Cyanogruppe, eine C1- bis C10-Halogenalkylgruppe oder eine C1- bis C10-Cyanoalkylgruppe ist), oder
in der chemischen Formel 2C, $X^3$ -O-, -S-, -Se-, -Te-, -S(=O)-, $-S(=O)_2-$, $-NR^{a1}-$, $- BR^{a2}-$, $-SiR^bR^c-$, $-GeR^dR^e-$ oder $-CR^fR^g-$ ist, wobei $R^{a1}$, $R^{a2}$, $R^b$, $R^c$, $R^d$, $R^e$, $R^f$ und $R^g$ jeweils unabhängig ein Halogen, eine C1- bis C20-Haloalkylgruppe oder eine C1- bis C20-Cyanoalkylgruppe sind und $X^2$ -O-, -S-, -Se-, -Te-, -S(=O)-, $-S(=O)_2-$, $-NR^{a1}-$, $-BR^{a2}-$, $- SiR^bR^c-$, $-GeR^dR^e-$, $-(CR^fR^g)_{n1}-$, $- (C(R^m)=C(R^n))-$ oder $-(C(R^p)=N)-$ ist, wobei $R^{a1}$, $R^{a2}$, $R^b$, $R^c$, $R^d$, $R^e$, $R^f$, $R^g$, $R^m$, $R^n$ und $R^p$ jeweils unabhängig ein Halogen, eine C1- bis C20-Haloalkylgruppe oder eine C1- bis C20-Cyanoalkylgruppe sind und n1 von $-(CR^fR^g)_{n1}-$ 1 oder 2 ist, oder
(ii) die Halbleiterverbindung vom p-Typ durch die chemische Formel 2C-1 oder die chemische Formel 2C-2 dargestellt ist:

[Chemische Formel 2C-1]

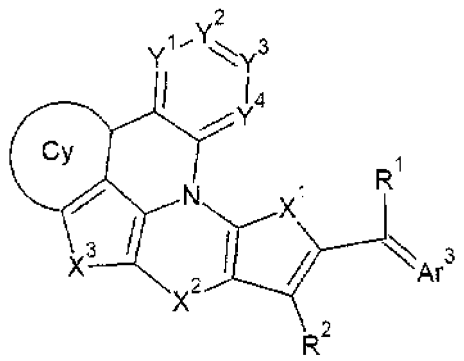

wobei in der chemischen Formel 2C-1,
$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,
$X^3$ das gleiche wie $X^3$ in der chemischen Formel 2C ist,
$Y^1$ bis $Y^4$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^{k}$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist,

[Chemische Formel 2C-2]

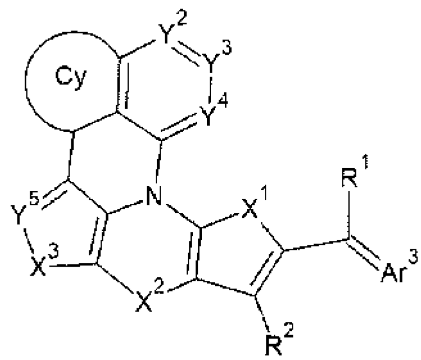

wobei in der chemischen Formel 2C-2
$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,
$X^3$ und $Y^5$ jeweils die gleichen wie $X^3$ und $Y^5$ in der chemischen Formel 2C sind,
$Y^2$ bis $Y^4$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^{k}$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist.

5. Zusammensetzung nach Anspruch 1, wobei die Halbleiterverbindung vom p-Typ der chemischen Formel 1 durch die chemische Formel 2D dargestellt ist:

[Chemische Formel 2D]

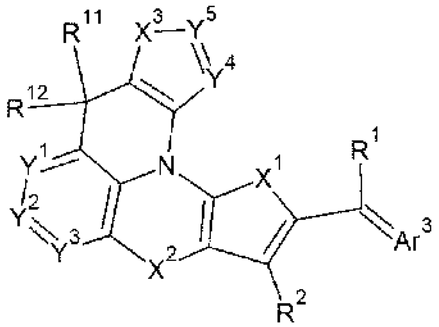

wobei in der chemischen Formel 2D

$X^1$, $X^2$, $Ar^3$, $R^1$, $R^2$, $R^{11}$ und $R^{12}$ jeweils die gleichen wie $X^1$, $X^2$, $Ar^3$, $R^1$, $R^2$, $R^{11}$ und $R^{12}$ in der chemischen Formel 1 sind,
$Y^1$ bis $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^{k}$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
$X^3$ -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$ oder $-CR^{ff}R^{gg}-$ ist, wobei $R^{a1}$, $R^{a2}$, $R^b$, $R^c$, $R^d$, $R^e$, $R^f$ und $R^g$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe, eine substituierte oder unsubstituierte C6- bis C20-Aryloxygruppe oder eine substituierte oder unsubstituierte C3-bis C20-Heteroarylgruppe sind, und zumindest ein Paar aus $R^{bb}$ und $R^{cc}$, $R^{dd}$ und $R^{ee}$ oder $R^{ff}$ und $R^{gg}$ miteinander verbunden ist, um eine Ringstruktur bereitzustellen;
wobei bevorzugt

(i) in der chemischen Formel 2D $Y^4$ N oder $CR^k$ ist, wobei $R^k$ ein Halogen, eine Cyanogruppe, eine C1- bis C10-Halogenalkylgruppe oder eine C1- bis C10-Cyanoalkylgruppe ist, oder
in der chemischen Formel 2D $Y^3$ N oder $CR^k$ ist, wobei $R^k$ ein Halogen, eine Cyanogruppe, eine C1- bis C10-Haloalkylgruppe oder eine C1- bis C10-Cyanoalkylgruppe ist und $X^2$ -O-, -S-, - Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, $-(C(R^m)=C(R^n))-$ oder $-(C(R^p)=N)-$ ist, wobei $R^{a1}$, $R^{a2}$, $R^b$, $R^c$, $R^d$, $R^e$, $R^f$, $R^g$, $R^m$, $R^n$ und $R^p$ jeweils unabhängig ein Halogen, eine C1- bis C20-Haloalkylgruppe oder eine C1- bis C20-Cyanoalkylgruppe sind und n1 von $-(CR^fR^g)_{n1}-$ 1 oder 2 ist; oder
(ii) die Halbleiterverbindung vom p-Typ durch die chemische Formel 2D-1 oder die chemische Formel 2D-2 dargestellt ist:

[Chemische Formel 2D-1]

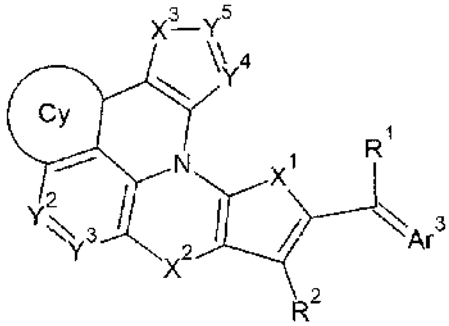

wobei in der chemischen Formel 2D-1,

$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,
$X^3$ das gleiche wie $X^3$ in der chemischen Formel 2D ist,
$Y^2$ bis $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^{k}$'s miteinander verbunden sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist,

[Chemische Formel 2D-2]

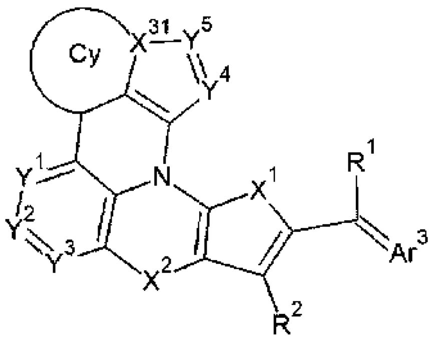

wobei in der chemischen Formel 2D-2
$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,
$X^{31}$ N, B, $SiR^b$, $GeR^d$, $CR^f$, Si, Ge oder C ist, wobei $R^b$, $R^d$ und $R^f$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe oder eine substituierte oder unsubstituierte C6- bis C20-Aryloxygruppe sind,
$Y^1$ bis $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^{k}$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und
Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist.

**6.** Zusammensetzung nach Anspruch 1, wobei die Halbleiterverbindung vom p-Typ der chemischen Formel 1 durch die chemische Formel 2E dargestellt ist:

[Chemische Formel 2E]

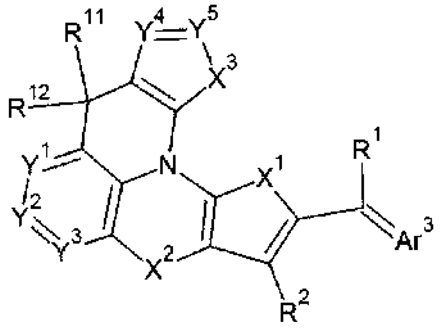

wobei in der chemischen Formel 2E,

$X^1$, $X^2$, $Ar^3$, $R^1$, $R^2$, $R^{11}$ und $R^{12}$ jeweils die gleichen wie $X^1$, $X^2$, $Ar^3$, $R^1$, $R^2$, $R^{11}$ und $R^{12}$ in der chemischen Formel 1 sind, und

$X^3$ -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$ oder $-CR^{ff}R^{gg}-$ ist, wobei $R^{a1}$, $R^{a2}$, $R^b$, $R^c$, $R^d$, $R^e$, $R^f$ und $R^g$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe, eine substituierte oder unsubstituierte C6- bis C20-Aryloxygruppe oder eine substituierte oder unsubstituierte C3-bis C20-Heteroarylgruppe sind und zumindest ein Paar aus $R^{bb}$ und $R^{cc}$, $R^{dd}$ und $R^{ee}$ oder $R^{ff}$ und $R^{gg}$ miteinander verbunden ist, um eine Ringstruktur bereitzustellen, und

$Y^1$ bis $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^{k}$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen,

wobei bevorzugt

(i) in der chemischen Formel 2E $X^3$ -O-, -S-, -Se-, -Te-, -S(=O)-, $-S(=O)_2-$, $-NR^{a1}-$, $-BR^{a2}-$, $-SiR^bR^c-$, $-GeR^dR^e-$ oder $-CR^fR^g-$ ist, wobei $R^{a1}$, $R^{a2}$, $R^b$, $R^c$, $R^d$, $R^e$, $R^f$ und $R^g$ jeweils unabhängig ein Halogen, eine C1- bis C20-Halogenalkylgruppe oder eine C1- bis C20-Cyanoalkylgruppe sind, oder

in der chemischen Formel 2E $Y^3$ N oder $CR^k$ ist, wobei $R^k$ ein Halogen, eine Cyanogruppe, eine C1- bis C10-Haloalkylgruppe oder eine C1- bis C10-Cyanoalkylgruppe ist und $X^2$ -O-, -S-, - Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, $-(C(R^m)=C(R^n))-$ oder $-(C(R^p)=N)-$ ist, wobei $R^{a1}$, $R^{a2}$, $R^b$, $R^c$, $R^d$, $R^e$, $R^f$, $R^g$, $R^m$, $R^n$ und $R^p$ jeweils unabhängig ein Halogen, eine C1- bis C20-Haloalkylgruppe oder eine C1- bis C20-Cyanoalkylgruppe sind und n1 von $-(CR^fR^g)_{n1}-$ 1 oder 2 ist; oder

(ii) die Halbleiterverbindung vom p-Typ durch die chemische Formel 2E-1 oder die chemische Formel 2E-2 dargestellt ist:

[Chemische Formel 2E-1]

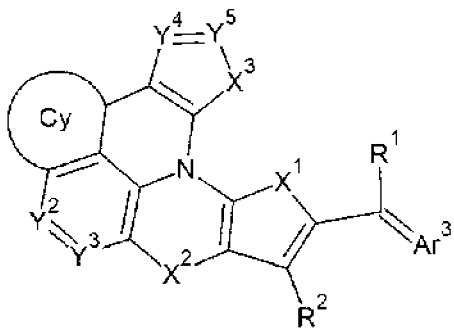

wobei in der chemischen Formel 2E-1,

$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,

$X^3$ das gleiche wie $X^3$ in der chemischen Formel 2E ist,

$Y^2$ bis $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^k$'s miteinander verbunden sind, um eine substituierte oder unsubstituierte C6- bis C30-Arengruppe, eine substituierte oder unsubstituierte C3- bis C30-Heteroarengruppe oder einen kondensierten Ring davon bereitzustellen, und

Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist,

[Chemische Formel 2E-2]

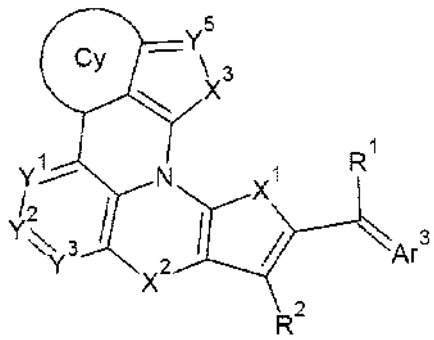

wobei in der chemischen Formel 2E-2,

$X^1$, $X^2$, $Ar^3$, $R^1$ und $R^2$ jeweils die gleichen wie $X^1$, $X^2$, $Ar^3$, $R^1$ und $R^2$ in der chemischen Formel 1 sind,

$X^3$ das gleiche wie $X^3$ in der chemischen Formel 2E ist,

$Y^1$ bis $Y^3$ und $Y^5$ jeweils unabhängig N oder $CR^k$ sind, wobei $R^k$ Wasserstoff, Deuterium, ein Halogen, eine Cyanogruppe, eine Nitrogruppe, eine Hydroxylgruppe, eine Amingruppe, eine substituierte oder unsubstituierte C1- bis C10-Alkylgruppe oder eine substituierte oder unsubstituierte C1- bis C10-Alkoxygruppe ist oder benachbarte $R^k$'s miteinander verknüpft sind, um eine substituierte oder unsubstituierte C6- bis C30-Arengruppe, eine substituierte oder unsubstituierte C3- bis C30-Heteroarengruppe oder einen kondensierten Ring davon bereitzustellen, und

Cy eine substituierte oder unsubstituierte C6- bis C30-Arengruppe, eine substituierte oder unsubstituierte C3- bis C30-Heteroarengruppe, eine substituierte oder unsubstituierte C5- bis C30-Cycloalkengruppe, eine substituierte oder unsubstituierte C3- bis C30-Heterocycloalkengruppe oder ein kondensierter Ring davon ist.

**7.** Zusammensetzung nach einem der Ansprüche 1 bis 6, wobei in $X^1$, $X^2$ und $-CR^{11}R^{12}-$ der chemischen Formel 1 jede Ringstruktur der Ringstruktur von $X^1$, der Ringstruktur von $X^2$ und der Ringstruktur von $-CR^{11}R^{12}-$ eine Spirostruktur oder eine kondensierte Ringstruktur ist;

wobei bevorzugt die Spirostruktur eine Einheit umfasst, die dargestellt ist durch die chemische Formel 3:

[chemische Formel 3]

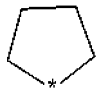
(1)

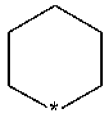
(2)

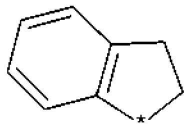
(3)

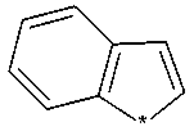
(4)

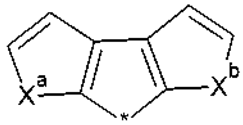
(5)

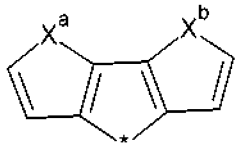
(6)

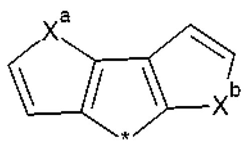
(7)

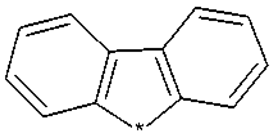
(8)

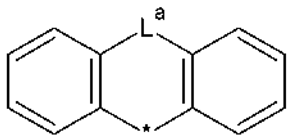
(9)

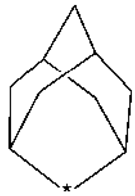
(10)

wobei in der chemischen Formel 3,

$X^a$ und $X^b$ jeweils unabhängig -O-, -S-, -Se-, -Te-, -S(=O)-, $-S(=O)_2-$, $-NR^{a1}-$, $-BR^{a2}-$, - $SiR^bR^c-$, $-SiR^{bb}R^{cc}-$, $-GeR^dR^e-$ oder $-GeR^{dd}R^{ee}-$ sind, wobei $R^{a1}$, $R^{a2}$, $R^b$, $R^c$, $R^d$ und $R^e$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe, eine substituierte oder unsubstituierte C6- bis C20-Aryloxygruppe oder eine substituierte oder unsubstituierte C3-bis C20-Heteroarylgruppe sind und zumindest ein Paar aus $R^{bb}$ und $R^{cc}$ oder $R^{dd}$ und $R^{ee}$ miteinander verknüpft ist, um eine Ringstruktur bereitzustellen,

$L^a$ -O-, -S-, -Se-, -Te-, $-NR^{a1}-$, $-BR^{a2}-$, $-SiR^bR^c-$, $-GeR^dR^e-$, $-(CR^fR^g)_{n1}-$, $-(C(R^p)=N)-$ oder eine Einfachbindung ist, wobei $R^{a1}$, $R^{a2}$, $R^b$, $R^c$, $R^d$, $R^e$, $R^f$, $R^g$ und $R^p$ jeweils unabhängig Wasserstoff, Deuterium, ein Halogen, eine Cyanogruppe, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe oder eine substituierte oder unsubstituierte C6- bis C20-Aryloxygruppe sind und n1 von $-(CR^fR^g)_{n1}-$ 1 oder 2 ist,

Wasserstoff von jedem Ring optional durch zumindest einen Substituenten ersetzt ist, ausgewählt aus Deuterium, einem Halogen, einer substituierten oder unsubstituierten C1- bis C20-Alkylgruppe, einer substituierten oder unsubstituierten C1- bis C20-Alkoxygruppe, einer substituierten oder unsubstituierten C6- bis C20-Arylgruppe und einer substituierten oder unsubstituierten C6- bis C20-Aryloxygruppe, und

ein oder mehrere CH, die in dem aromatischen Ring der Einheiten (3), (4), (5), (6), (7), (8) oder (9) vorhanden sind, optional durch N ersetzt sind.

**8.** Zusammensetzung nach Anspruch 7, wobei zumindest ein Wasserstoff von jedem Ring durch zumindest einen Substituenten ersetzt ist, ausgewählt aus Deuterium, einem Halogen, einer substituierten oder unsubstituierten C1- bis C20-Alkylgruppe, einer substituierten oder unsubstituierten C1- bis C20-Alkoxygruppe, einer substituierten oder unsubstituierten C6- bis C20-Arylgruppe und einer substituierten oder unsubstituierten C6- bis C20-Aryloxygruppe.

**9.** Zusammensetzung nach einem der Ansprüche 1 bis 8, wobei $Ar^3$ eine cyclische Gruppe ist, die durch die chemische Formel 4 dargestellt ist:

[Chemische Formel 4]

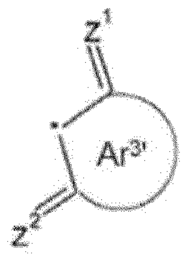

wobei in der chemischen Formel 4,

$Ar^{3'}$ eine substituierte oder unsubstituierte C6- bis C30-Arylgruppe oder eine substituierte oder unsubstituierte C3- bis C30-Heteroarylgruppe ist,
$Z^1$ O, S, Se oder Te ist, und
$Z^2$ O, S, Se, Te oder $CR^aR^b$ ist, wobei $R^a$ und $R^b$ jeweils unabhängig Wasserstoff, eine substituierte oder unsubstituierte C1- bis C10-Alkylgruppe, eine Cyanogruppe oder eine cyanhaltige Gruppe sind, vorausgesetzt, dass, wenn $Z^2$ $CR^aR^b$ ist, zumindest eines von $R^a$ oder $R^b$ eine Cyanogruppe oder eine cyanohaltige Gruppe ist; oder wobei in der chemischen Formel 1 $Ar^3$ eine cyclische Gruppe ist, die durch eine der chemischen Formeln 5A bis 5G dargestellt ist:

[Chemische Formel 5A]

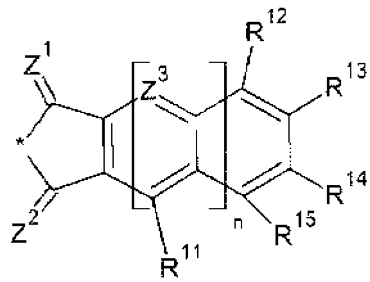

wobei in der chemischen Formel 5A,
$Z^1$ O, S, Se oder Te ist,
$Z^2$ O, S, Se, Te oder $CR^aR^b$ ist, wobei $R^a$ und $R^b$ jeweils unabhängig Wasserstoff, eine substituierte oder unsubstituierte C1- bis C10-Alkylgruppe, eine Cyanogruppe oder eine cyanohaltige Gruppe sind, vorausgesetzt, dass, wenn $Z^2$ $CR^aR^b$ ist, zumindest eines von $R^a$ oder $R^b$ eine Cyanogruppe oder eine cyanohaltige Gruppe ist,
$Z^3$ N oder $CR^c$ ist, wobei $R^c$ Wasserstoff, Deuterium oder eine substituierte oder unsubstituierte C1- bis C10-Alkylgruppe ist,
$R^{11}$, $R^{12}$, $R^{13}$, $R^{14}$ und $R^{15}$ jeweils unabhängig Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte C4- bis C30-Heteroarylgruppe, ein Halogen, eine Cyanogruppe (-CN), eine cyanohaltige Gruppe oder eine beliebige Kombination davon sind, wobei $R^{12}$ und $R^{13}$ und $R^{14}$ und $R^{15}$ jeweils unabhängig vorhanden sind oder miteinander verknüpft sind, um einen kondensierten aromatischen Ring bereitzustellen,
n 0 oder 1 ist, und
* ein Verbindungspunkt ist,

[Chemische Formel 5B]

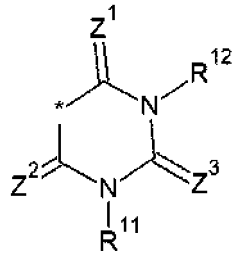

wobei in der chemischen Formel 5B,

$Z^1$ O, S, Se oder Te ist,

$Z^2$ O, S, Se, Te oder $CR^aR^b$ ist, wobei $R^a$ und $R^b$ jeweils unabhängig Wasserstoff, eine substituierte oder unsubstituierte C1- bis C10-Alkylgruppe, eine Cyanogruppe oder eine cyanohaltige Gruppe sind, vorausgesetzt, dass, wenn $Z^2$ $CR^aR^b$ ist, zumindest eines von $R^a$ oder $R^b$ eine Cyanogruppe oder eine cyanohaltige Gruppe ist,

$Z^3$ O, S, Se, Te und $C(R^a)(CN)$ ist, wobei $R^a$ Wasserstoff, eine Cyanogruppe (-CN) oder eine C1- bis C10-Alkylgruppe ist,

$R^{11}$ und $R^{12}$ jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe (-CN) oder eine beliebige Kombination davon sind, und

* ein Verbindungspunkt ist,

[Chemische Formel 5C]

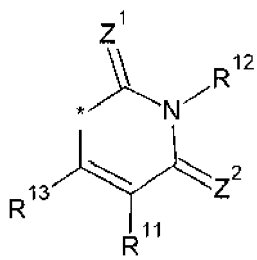

wobei in der chemischen Formel 5C,

$Z^1$ O, S, Se oder Te ist,

$Z^2$ O, S, Se, Te oder $CR^aR^b$ ist, wobei $R^a$ und $R^b$ jeweils unabhängig Wasserstoff, eine substituierte oder unsubstituierte C1- bis C10-Alkylgruppe, eine Cyanogruppe oder eine cyanohaltige Gruppe sind, vorausgesetzt, dass, wenn $Z^2$ $CR^aR^b$ ist, zumindest eines von $R^a$ oder $R^b$ eine Cyanogruppe oder eine cyanohaltige Gruppe ist,

$R^{11}$, $R^{12}$ und $R^{13}$ jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe (-CN) oder eine beliebige Kombination davon sind, und

* ein Verbindungspunkt ist,

[Chemische Formel 5D]

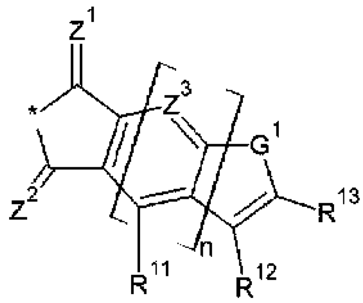

wobei in der chemischen Formel 5D
$Z^1$ O, S, Se oder Te ist,
$Z^2$ O, S, Se, Te oder $CR^aR^b$ ist, wobei $R^a$ und $R^b$ jeweils unabhängig Wasserstoff, eine substituierte oder unsubstituierte C1- bis C10-Alkylgruppe, eine Cyanogruppe oder eine cyanohaltige Gruppe sind, vorausgesetzt, dass, wenn $Z^2$ $CR^aR^b$ ist, zumindest eines von $R^a$ oder $R^b$ eine Cyanogruppe oder eine cyanohaltige Gruppe ist,
$Z^3$ N oder $CR^c$ ist, wobei $R^c$ Wasserstoff oder eine substituierte oder unsubstituierte C1-bis C10-Alkylgruppe ist,
$G^1$ O, S, Se, Te, $SiR^xR^y$ oder $GeR^zR^w$ ist, wobei $R^x$, $R^y$, $R^z$ und $R^w$ jeweils unabhängig Wasserstoff, Deuterium, ein Halogen, eine substituierte oder unsubstituierte C1- bis C20-Alkylgruppe oder eine substituierte oder unsubstituierte C6- bis C20-Arylgruppe sind,
$R^{11}$, $R^{12}$ und $R^{13}$ jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe, eine cyanohaltige Gruppe oder eine beliebige Kombination davon sind, wobei $R^{12}$ und $R^{13}$ jeweils unabhängig vorhanden sind oder miteinander verknüpft sind, um einen kondensierten aromatischen Ring bereitzustellen,
n 0 oder 1 ist, und
* ein Verbindungspunkt ist,

[Chemische Formel 5E]

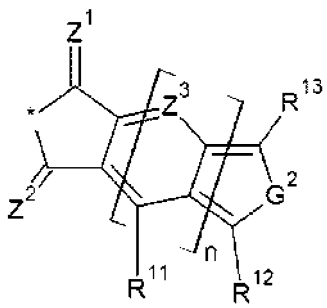

wobei in der chemischen Formel 5E,
$Z^1$ O, S, Se oder Te ist,
$Z^2$ O, S, Se, Te oder $CR^aR^b$ ist, wobei $R^a$ und $R^b$ jeweils unabhängig Wasserstoff, eine substituierte oder unsubstituierte C1- bis C10-Alkylgruppe, eine Cyanogruppe oder eine cyanohaltige Gruppe sind, vorausgesetzt, dass, wenn $Z^2$ $CR^aR^b$ ist, zumindest eines von $R^a$ oder $R^b$ eine Cyanogruppe oder eine cyanohaltige Gruppe ist,
$Z^3$ N oder $CR^c$ ist, wobei $R^c$ Wasserstoff oder eine substituierte oder unsubstituierte C1-bis C10-Alkylgruppe ist,
$G^2$ O, S, Se, Te, $SiR^xR^y$ oder $GeR^zR^w$ ist, wobei $R^x$, $R^y$, $R^z$ und $R^w$ jeweils unabhängig Wasserstoff, Deuterium, ein Halogen, eine substituierte oder unsubstituierte C1- bis C20-Alkylgruppe oder eine substituierte oder unsubstituierte C6- bis C20-Arylgruppe sind,
$R^{11}$, $R^{12}$ und $R^{13}$ jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe, eine cyanohaltigen Gruppe oder eine beliebige Kombination davon sind, und

* ein Verbindungspunkt ist,

[Chemische Formel 5F]

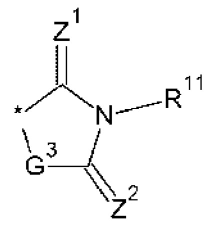

wobei in der chemischen Formel 5F,
$Z^1$ O, S, Se oder Te ist,
$Z^2$ O, S, Se, Te oder $CR^aR^b$ ist, wobei $R^a$ und $R^b$ jeweils unabhängig Wasserstoff, eine substituierte oder unsubstituierte C1- bis C10-Alkylgruppe, eine Cyanogruppe oder eine cyanohaltige Gruppe sind, vorausgesetzt, dass, wenn $Z^2$ $CR^aR^b$ ist, zumindest eines von $R^a$ oder $R^b$ eine Cyanogruppe oder eine cyanohaltige Gruppe ist,
$R^{11}$ Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte C4- bis C30-Heteroarylgruppe, ein Halogen, eine Cyanogruppe (-CN), eine cyanohaltige Gruppe oder eine beliebige Kombination davon ist, und
$G^3$ O, S, Se, Te, $SiR^xR^y$ oder $GeR^zR^w$ ist, wobei $R^x$, $R^y$, $R^z$ und $R^w$ jeweils unabhängig Wasserstoff, Deuterium, ein Halogen, eine substituierte oder unsubstituierte C1- bis C20-Alkylgruppe oder eine substituierte oder unsubstituierte C6- bis C20-Arylgruppe sind,

[Chemische Formel 5G]

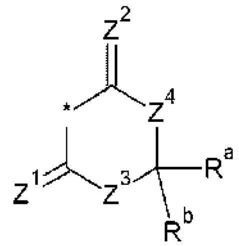

wobei in der chemischen Formel 5G,
$Z^1$ O, S, Se oder Te ist,
$R^a$ und $R^b$ unabhängig Wasserstoff, eine substituierte oder unsubstituierte C1- bis C10-Alkylgruppe, eine Cyanogruppe oder eine cyanohaltige Gruppe sind, und
$Z^2$ bis $Z^4$ jeweils unabhängig O, S, Se, Te oder $CR^cR^d$ sind, wobei $R^c$ und $R^d$ jeweils unabhängig Wasserstoff, eine substituierte oder unsubstituierte C1- bis C10-Alkylgruppe, eine Cyanogruppe oder eine cyanohaltige Gruppe sind, vorausgesetzt, dass wenn $Z^2$ $CR^cR^d$ ist, zumindest eines von $R^c$ oder $R^d$ eine Cyanogruppe oder eine cyanohaltige Gruppe ist.

**10.** Zusammensetzung nach einem der Ansprüche 1 bis 9, wobei die Zusammensetzung eine maximale Absorptionswellenlänge ($\lambda_{max}$) in einem Wellenlängenbereich von mehr als oder gleich 500 nm und weniger als oder gleich 600 nm in einem Dünnfilmzustand aufweist; und/oder
wobei die Zusammensetzung konfiguriert ist, um eine Lichtabsorptionskurve mit einer vollen Breite bei halber Maximalbreite (FWHM) in einem Dünnfilmzustand von 50 nm bis 110 nm zu zeigen.

**11.** Fotoelektrische Vorrichtung (100, 200), umfassend:

eine erste Elektrode (10) und eine zweite Elektrode (20),
die einander zugewandt sind, und
eine aktive Schicht (30)
zwischen der ersten Elektrode und der zweiten Elektrode,

wobei die aktive Schicht die Zusammensetzung nach einem der Ansprüche 1 bis 10 beinhaltet.

**12.** Bildsensor (300, 400, 500, 600),

umfassend die fotoelektrische Vorrichtung nach Anspruch 11;
bevorzugt:

wobei die fotoelektrische Vorrichtung eine grüne fotoelektrische Vorrichtung ist, die konfiguriert ist, um Licht in einem grünen Wellenlängenbereich selektiv zu absorbieren,
der Bildsensor ferner eine blaue fotoelektrische Vorrichtung (50B), die konfiguriert ist, um
Licht in einem blauen Wellenlängenbereich selektiv zu erfassen, und eine rote fotoelektrische Vorrichtung (50R),
die konfiguriert ist, um Licht in einem roten Wellenlängenbereich selektiv zu erfassen, beinhaltet,
wobei die grüne fotoelektrische Vorrichtung, die blaue fotoelektrische Vorrichtung und die rote fotoelektrische Vorrichtung gestapelt sind.

**13.** Bildsensor nach Anspruch 12, ferner umfassend:

ein Halbleitersubstrat (310),
das mit einer Vielzahl von ersten Fotoerfassungsvorrichtung (50B),
die konfiguriert ist, um Licht in einem blauen Wellenlängenbereich zu erfassen, und einer Vielzahl von zweiten Fotoerfassungsvorrichtung (50R),
die konfiguriert ist, um Licht in einem roten Wellenlängenbereich zu erfassen, integriert ist,
wobei die fotoelektrische Vorrichtung auf dem Halbleitersubstrat ist und konfiguriert ist, um Licht in einem grünen Wellenlängenbereich selektiv zu erfassen.

**14.** Bildsensor nach Anspruch 13, ferner umfassend:

eine Farbfilterschicht (70, 72),
wobei die Farbfilterschicht einen Blaufilter (70B, 72B), der
konfiguriert ist, um selektiv
Licht in dem blauen Wellenlängenbereich zu übertragen, und einen Rotfilter (70R, 72R), der
konfiguriert ist, um selektiv
Licht in dem roten Wellenlängenbereich zu übertragen, beinhaltet; und/oder
wobei die Vielzahl von ersten Fotoerfassungsvorrichtungen und die Vielzahl von zweiten Fotoerfassungsvorrichtungen in einer vertikalen Richtung in dem Halbleitersubstrat gestapelt sind.

**15.** Elektronische Vorrichtung, umfassend den Bildsensor nach einem der Ansprüche 12 bis 14.

**Revendications**

**1.** Composition pour un dispositif photoélectrique, la composition comprenant :

un composé semi-conducteur de type p représenté par la formule chimique 1 et un composé semi-conducteur de type n :

[Formule chimique 1]

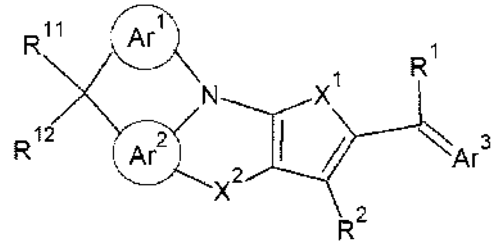

dans laquelle, dans la formule chimique 1,

$Ar^1$ et $Ar^2$ sont chacun indépendamment un groupe arène en C6 à C30 substitué ou non substitué, un groupe hétéroarène en C3 à C30 substitué ou non substitué, ou un cycle condensé de celui-ci,

$X^1$ est -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$, ou $-CR^{ff}R^{gg}-$, dans laquelle $R^{a1}$, $R^{a2}$, $R^b$, $R^c$, $R^d$, $R^e$, $R^f$, et $R^g$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, un groupe aryloxy en C6 à C20 substitué ou non substitué, ou un groupe hétéroaryle en C3 à C20 substitué ou non substitué, et au moins une paire de $R^{bb}$ et $R^{cc}$, $R^{dd}$ et $R^{ee}$, ou $R^{ff}$ et $R^{gg}$ sont liés l'un à l'autre pour fournir une structure cyclique,

$X^2$ est -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-(CR^fR^g)_{n1}-$, $-(CR^{ff}R^{gg})-$, $-(C(R^m)=C(R^n))-$, $-(C(R^{mm})=C(R^{nn}))-$, ou $-(C(R^p)=N)-$, dans laquelle $R^{a1}$, $R^{a2}$, $R^b$, $R^c$, $R^d$, $R^e$, $R^f$, $R^g$, $R^m$, $R^n$, et $R^p$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, un groupe aryloxy en C6 à C20 substitué ou non substitué, ou un groupe hétéroaryle en C3 à C20 substitué ou non substitué, et au moins une paire de $R^{bb}$ et $R^{cc}$, $R^{dd}$ et $R^{ee}$, $R^{ff}$ et $R^{gg}$, ou $R^{mm}$ et $R^{nn}$ sont liés l'un à l'autre pour former une structure cyclique, et n1 de $-(CR^fR^g)_{n1}-$ est 1 ou 2,

$R^{11}$ et $R^{12}$ sont chacun indépendamment l'hydrogène, le deutérium, un halogène, un groupe cyano, un groupe alkyle en C1 à C20 substitué ou non substitué, un groupe aryle en C6 à C20 substitué ou non substitué, ou un groupe aryloxy en C6 à C20 substitué ou non substitué, dans laquelle $R^{11}$ et $R^{12}$ sont chacun indépendamment présents ou sont liés l'un à l'autre pour fournir une structure cyclique, ou $-CR^{11}R^{12}-$ est éventuellement lié à $Ar^1$ ou $Ar^2$ pour fournir une structure cyclique,

$Ar^3$ est un groupe hydrocarbure cyclique en C6 à C30 substitué ou non substitué comportant au moins un groupe fonctionnel choisi parmi C=O, C=S, C=Se et C=Te, un groupe hétérocyclique en C2 à C30 substitué ou non substitué comportant au moins un groupe fonctionnel choisi parmi C=O, C=S, C=Se et C=Te, ou un cycle fusionné de celui-ci, et

$R^1$ et $R^2$ sont chacun indépendamment l'hydrogène, le deutérium, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C3 à C30 substitué ou non substitué, un groupe acyle en C2 à C30 substitué ou non substitué, un halogène, un groupe cyano (-CN), un groupe contenant un cyano, un groupe nitro, un groupe pentafluorosulfanyle ($-SF_5$), un groupe hydroxyle, un groupe amine, un groupe hydrazine, un groupe hydrazone, un groupe carboxyle ou un sel de celui-ci, un groupe acide sulfonique ou un sel de celui-ci, un groupe acide phosphorique ou un sel de celui-ci, $-SiR^aR^bR^c$, dans laquelle $R^a$, $R^b$, et $R^c$ sont chacun indépendamment l'hydrogène ou un groupe alkyle en C1 à C10 substitué ou non substitué, ou toute combinaison de ceux-ci.

**2.** Composition selon la revendication 1, dans laquelle le composé semi-conducteur de type p de formule chimique 1 est représenté par la formule chimique 2A :

[Formule chimique 2A]

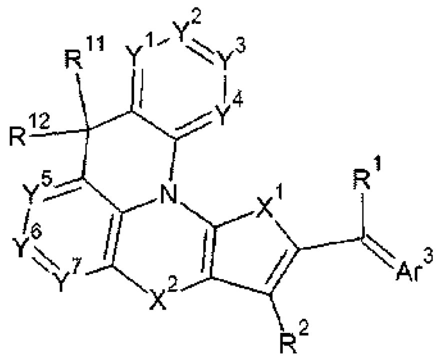

dans laquelle, dans la formule chimique 2A,

$X^1$, $X^2$, $Ar^3$, $R^1$, $R^2$, $R^{11}$, et $R^{12}$ sont les mêmes que $X^1$, $X^2$, $Ar^3$, $R^1$, $R^2$, $R^{11}$, et $R^{12}$, respectivement, dans la

formule chimique 1, et
$Y^1$ à $Y^7$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué, ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci ;
de préférence dans laquelle

(i) dans la formule chimique 2A, $Y^4$ est N ou $CR^k$, dans laquelle $R^k$ est un halogène, un groupe cyano, un groupe haloalkyle en C1 à C10 ou un groupe cyanoalkyle en C1 à C10, ou
dans la Formule Chimique 2A, $Y^7$ est N ou $CR^k$, dans laquelle $R^k$ est un halogène, un groupe cyano, un groupe haloalkyle en C1 à C10 ou un groupe cyanoalkyle en C1 à C10, et $X^2$ est -O-, - S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, - $(C(R^m)=C(R^n))-$, ou $-(C(R^p)=N)-$, dans laquelle $R^{a1}$, $R^{a2}$, $R^b$, $R^c$, $R^d$, $R^e$, $R^f$, $R^g$, $R^m$, $R^n$, et $R^p$ sont chacun indépendamment un halogène, un groupe haloalkyle en C1 à C20 ou un groupe cyanoalkyle en C1 à C20 et n1 de $-(CR^fR^g)_{n1}-$ est 1 ou 2 ; ou
(ii) le composé semi-conducteur de type p de la formule chimique 2A est représenté par la formule chimique 2A-1 ou la formule chimique 2A-2 :

[Formule chimique 2A-1]

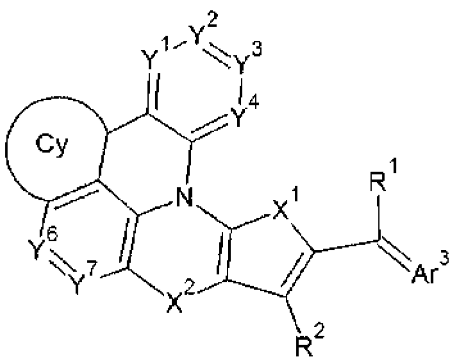

dans laquelle, dans la formule chimique 2A-1,
$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,
$Y^1$ à $Y^4$, $Y^6$, et $Y^7$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué, ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci,

[Formule chimique 2A-2]

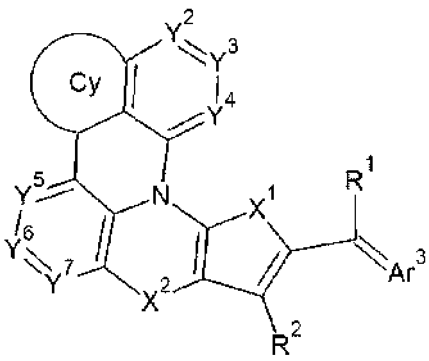

dans laquelle, dans la formule chimique 2A-2,

$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,
$Y^2$ à $Y^7$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci.

**3.** Composition selon la revendication 1, dans laquelle le composé semi-conducteur de type p de formule chimique 1 est représenté par la formule chimique 2B :

[Formule chimique 2B]

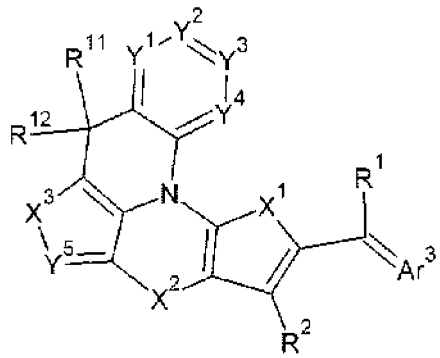

dans laquelle, dans la formule chimique 2B,

$X^1$, $X^2$, $Ar^3$, $R^1$, $R^2$, $R^{11}$, et $R^{12}$ sont les mêmes que $X^1$, $X^2$, $Ar^3$, $R^1$, $R^2$, $R^{11}$, et $R^{12}$ respectivement, dans la formule chimique 1,
$Y^1$ à $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
$X^3$ est -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-CR^fR^g-$, ou $-CR^{ff}R^{gg}-$, dans laquelle $R^{a1}$, $R^{a2}$, $R^b$, $R^c$, $R^d$, $R^e$, $R^f$, et $R^g$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, un groupe aryloxy en C6 à C20 substitué ou non substitué, un groupe hétéroaryle en C3 à C20 substitué ou non substitué, et au moins une paire de $R^{bb}$ et $R^{cc}$, $R^{dd}$ et $R^{ee}$, ou $R^{ff}$ et $R^{gg}$ sont liés l'un à l'autre pour former une structure cyclique ;

de préférence dans laquelle

(i) dans la formule chimique 2B, $Y^4$ est N ou $CR^k$, dans laquelle $R^k$ est un halogène, un groupe cyano, un groupe haloalkyle en C1 à C10 ou un groupe cyanoalkyle en C1 à C10, ou
dans la formule chimique 2B, $Y^5$ est N ou $CR^k$, dans laquelle $R^k$ est un halogène, un groupe cyano, un groupe haloalkyle en C1à C10 ou un groupe cyanoalkyle en C1 à C10 et $X^2$ est -O-, - S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, - $(C(R^m)=C(R^n))$-, ou $-(C(R^p)=N)-$, dans laquelle $R^{a1}$, $R^{a2}$, $R^b$, $R^c$, $R^d$, $R^e$, $R^f$, $R^g$, $R^m$, $R^n$, et $R^p$ sont chacun indépendamment un halogène, un groupe haloalkyle en C1 à C20, ou un groupe cyanoalkyle en C1 à C20, et n1 de $-(CR^fR^g)_{n1}-$ est 1 ou 2 ; ou
(ii) le composé semi-conducteur de type p de la formule chimique 2B est représenté par la formule chimique 2B-1 ou la formule chimique 2B-2 :

[Formule chimique 2B-1]

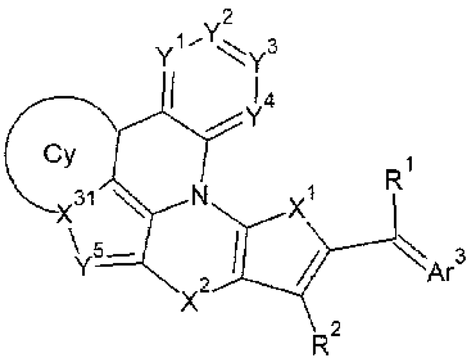

dans laquelle, dans la formule chimique 2B-1,
$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,
$X^{31}$ est N, B, $SiR^b$, $GeR^d$, $CR^f$, Si, Ge, ou C, dans laquelle $R^b$, $R^d$, et $R^f$ sont chacun indépendamment un hydrogène, un deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, ou un groupe aryloxy en C6 à C20 substitué ou non substitué,
$Y^1$ à $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci,

[Formule chimique 2B-2]

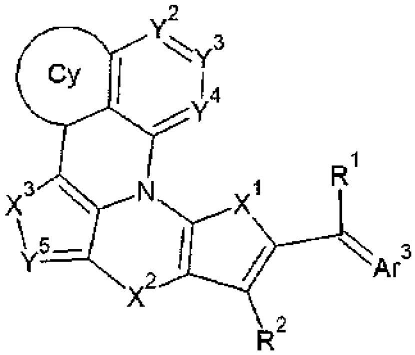

dans laquelle, dans la formule chimique 2B-2,

$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,
$X^3$ et $Y^5$ sont les mêmes que $X^3$ et $Y^5$, respectivement, dans la formule chimique 2B,
$Y^2$ à $Y^4$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci.

**4.** Composition selon la revendication 1, dans laquelle le composé semi-conducteur de type p de formule chimique 1 est représenté par la formule chimique 2C :

[Formule chimique 2C]

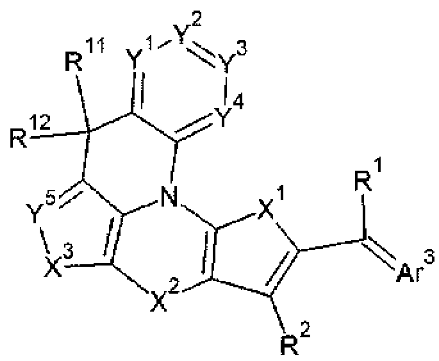

dans laquelle, dans la formule chimique 2C,

$X^1$, $X^2$, $Ar^3$, $R^1$, $R^2$, $R^{11}$, et $R^{12}$ sont les mêmes que $X^1$, $X^2$, $Ar^3$, $R^1$, $R^2$, $R^{11}$, et $R^{12}$ respectivement, dans la formule chimique 1,
$Y^1$ à $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
$X^3$ est -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, - $GeR^dR^e-$, $-GeR^{dd}R^{ee}-$, $-CR^fR^g-$, ou $-CR^{ff}R^{gg}-$, dans laquelle $R^{a1}$, $R^{a2}$, $R^b$, $R^c$, $R^d$, $R^e$, $R^f$, et $R^g$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, ou un groupe aryloxy en C6 à 20 substitué ou non substitué ou un groupe hétéroaryle en C3 à C20 substitué ou non substitué, et au moins une paire de $R^{bb}$ et $R^{cc}$, $R^{dd}$ et $R^{ee}$, ou $R^{ff}$ et $R^{gg}$ sont liés l'un à l'autre pour former une structure cyclique ;
de préférence dans laquelle

(i) dans la formule chimique 2C, $Y^4$ est N ou $CR^k$, dans laquelle $R^k$ est un halogène, un groupe cyano, un groupe haloalkyle en C1 à C10 ou un groupe cyanoalkyle en C1 à C10, ou
dans la formule chimique 2C, $X^3$ est -O-, -S-, -Se-, -Te-, -S(=O)-, $-S(=O)_2-$, $-NR^{a1}-$, $-BR^{a2}-$, $-SiR^bR^c-$, $-GeR^dR^e-$, ou $-CR^fR^g-$, dans laquelle $R^{a1}$, $R^{a2}$, $R^b$, $R^c$, $R^d$, $R^e$, $R^f$, et $R^g$ sont chacun indépendamment un halogène, un groupe haloalkyle en C1 à C20 ou un groupe cyanoalkyle en C1 à C20, et $X^2$ est -O-, -S-, -Se-, -Te-, -S(=O)-, $-S(=O)_2-$, $-NR^{a1}-$, $-BR^{a2}-$, $-SiR^bR^c-$, $-GeR^dR^e-$, - $(CR^fR^g)_{n1}-$, $-(C(R^m)=C(R^n))-$, ou $-(C(R^p)=N)-$, dans laquelle $R^{a1}$, $R^{a2}$, $R^b$, $R^c$, $R^d$, $R^e$, $R^f$, $R^g$, $R^m$, $R^n$, et $R^p$ sont chacun indépendamment un halogène, un groupe haloalkyle en C1 à C20 ou un groupe cyanoalkyle en C1 à C20, et n1 de $-(CR^fR^g)_{n1}-$ est 1 ou 2 ; ou
(ii) le composé semi-conducteur de type p est représenté par la formule chimique 2C-1 ou la formule chimique 2C-2 :

[Formule chimique 2C-1]

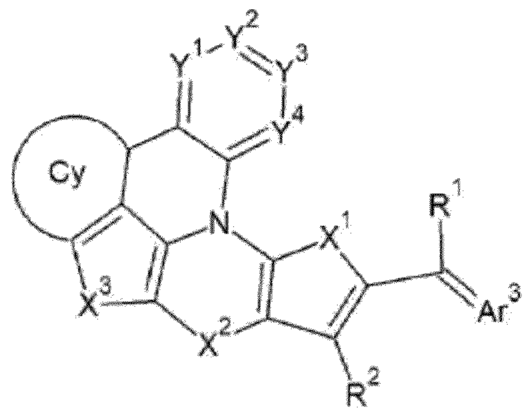

dans laquelle, dans la formule chimique 2C-1,

$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,

$X^3$ est le même que $X^3$ dans la formule chimique 2C,

$Y^1$ à $Y^4$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et

Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci,

[Formule chimique 2C-2]

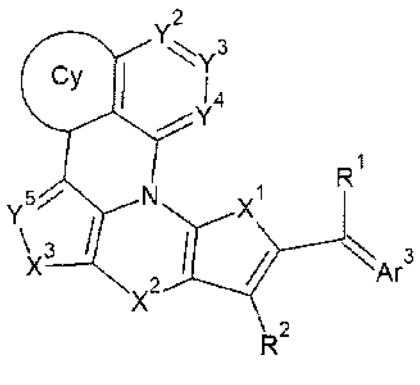

dans laquelle, dans la formule chimique 2C-2,

$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,

$X^3$ et $Y^5$ sont les mêmes que $X^3$ et $Y^5$, respectivement, dans la formule chimique 2C,

$Y^2$ à $Y^4$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et

Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci.

**5.** Composition selon la revendication 1, dans laquelle le composé semi-conducteur de type p de formule chimique 1 est représenté par la formule chimique 2D :

[Formule chimique 2D]

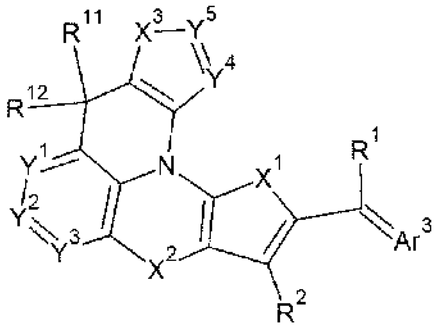

dans laquelle, dans la formule chimique 2D,

$X^1$, $X^2$, $Ar^3$, $R^1$, $R^2$, $R^{11}$, et $R^{12}$ sont les mêmes que $X^1$, $X^2$, $Ar^3$, $R^1$, $R^2$, $R^{11}$, et $R^{12}$ respectivement, dans la formule chimique 1,
$Y^1$ à $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
$X^3$ est -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, - $GeR^dR^e-$, $-GeR^{dd}R^{ee}-$, $-CR^fR^g-$, ou $-CR^{ff}R^{gg}-$, dans laquelle $R^{a1}$, $R^{a2}$, $R^b$, $R^c$, $R^d$, $R^e$, $R^f$, et $R^g$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, un groupe aryloxy en C6 à C20 substitué ou non substitué, un groupe hétéroaryle en C3 à C20 substitué ou non substitué, et au moins une paire de $R^{bb}$ et $R^{cc}$, $R^{dd}$ et $R^{ee}$, ou $R^{ff}$ et $R^{gg}$ sont liés l'un à l'autre pour former une structure cyclique ;
de préférence dans laquelle

(i) dans la formule chimique 2D, $Y^4$ est N ou $CR^k$, dans laquelle $R^k$ est un halogène, un groupe cyano, un groupe haloalkyle en C1 à C10 ou un groupe cyanoalkyle en C1 à C10, ou
dans la formule chimique 2D, $Y^3$ est N ou $CR^k$, dans laquelle $R^k$ est un halogène, un groupe cyano, un groupe haloalkyle en C1 à C10, ou un groupe cyanoalkyle en C1 à C10, et $X^2$ est -O-, - S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, - $(C(R^m)=C(R^n))-$, ou $-(C(R^p)=N)-$, dans laquelle $R^{a1}$, $R^{a2}$, $R^b$, $R^c$, $R^d$, $R^e$, $R^f$, $R^g$, $R^m$, $R^n$, et $R^p$ sont chacun indépendamment un halogène, un groupe haloalkyle en C1 à C20, ou un groupe cyanoalkyle en C1 à C20, et n1 de $-(CR^fR^g)_{n1}-$ est 1 ou 2 ; ou
(ii) le composé semi-conducteur de type p est représenté par la formule chimique 2D-1 ou la formule chimique 2D-2 :

[Formule chimique 2D-1]

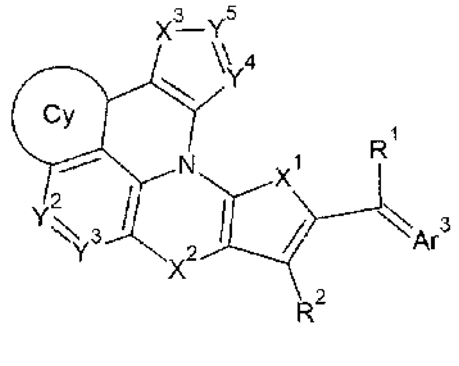

dans laquelle, dans la formule chimique 2D-1,
$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,

$X^3$ est le même que $X^3$ dans la formule chimique 2D,
$Y^2$ à $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci,

[Formule chimique 2D-2]

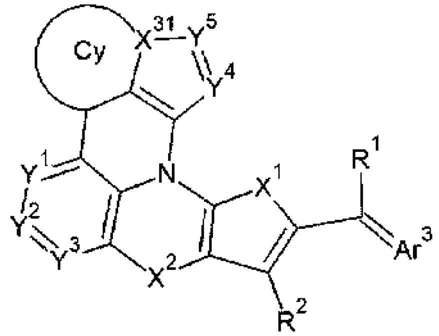

dans laquelle, dans la formule chimique 2D-2,
$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,
$X^{31}$ est N, B, $SiR^b$, $GeR^d$, $CR^f$, Si, Ge, ou C, dans laquelle $R^b$, $R^d$, et $R^f$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, ou un groupe aryloxy en C6 à C20 substitué ou non substitué,
$Y^1$ à $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et
Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci.

**6.** Composition selon la revendication 1, dans laquelle le composé semi-conducteur de type p de formule chimique 1 est représenté par la formule chimique 2E :

[Formule chimique 2E]

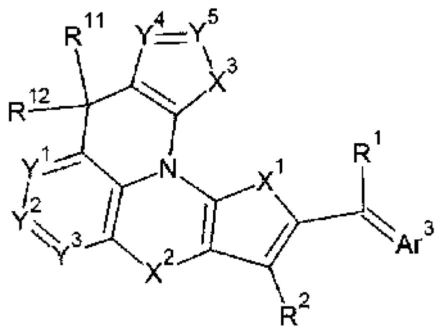

dans laquelle, dans la formule chimique 2E,

$X^1$, $X^2$, $Ar^3$, $R^1$, $R^2$, $R^{11}$, et $R^{12}$ sont les mêmes que $X^1$, $X^2$, $Ar^3$, $R^1$, $R^2$, $R^{11}$, et $R^{12}$, respectivement, dans la formule chimique 1, et

$X^3$ est -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, - $GeR^dR^e-$, $-GeR^{dd}R^{ee}-$, $-CR^fR^g-$, ou $-CR^{ff}R^{gg}-$, dans laquelle $R^{a1}$, $R^{a2}$, $R^b$, $R^c$, $R^d$, $R^e$, $R^f$, et $R^g$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, un groupe aryloxy en C6 à C20 substitué ou non substitué, ou un groupe hétéroaryle en C3 à C20 substitué ou non substitué, et au moins une paire de $R^{bb}$ et $R^{cc}$, $R^{dd}$ et $R^{ee}$, ou $R^{ff}$ et $R^{gg}$ sont liés l'un à l'autre pour former une structure cyclique, et

$Y^1$ à $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci ;

de préférence dans laquelle

(i) dans la formule chimique 2E, $X^3$ est -O-, -S-, -Se-, -Te-, -S(=O)-, $-S(=O)_2-$, $-NR^{a1}-$, - $BR^{a2}-$, $-SiR^bR^c-$, $-GeR^dR^e-$, ou $-CR^fR^g-$, dans laquelle $R^{a1}$, $R^{a2}$, $R^b$, $R^c$, $R^d$, $R^e$, $R^f$, et $R^g$ sont chacun indépendamment un halogène, un groupe haloalkyle en C1 à C20 ou un groupe cyanoalkyle en C1 à C20, ou

dans la formule chimique 2E, $Y^3$ est N ou $CR^k$, dans laquelle $R^k$ est un halogène, un groupe cyano, un groupe haloalkyle en C1 à C10, ou un groupe cyanoalkyle en C1 à C10, et $X^2$ est -O-, - S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, - $(C(R^m)=C(R^n))-$, ou $-(C(R^p)=N)-$, dans laquelle $R^{a1}$, $R^{a2}$, $R^b$, $R^c$, $R^d$, $R^e$, $R^f$, $R^g$, $R^m$, $R^n$, et $R^p$ sont chacun indépendamment un halogène, un groupe haloalkyle C1 à C20, ou un groupe cyanoalkyle en C1 à C20 et n1 de $-(CR^fR^g)_{n1}-$ est 1 ou 2 ; ou

(ii) le composé semi-conducteur de type p est représenté par la formule chimique 2E-1 ou la formule chimique 2E-2 :

[Formule chimique 2E-1]

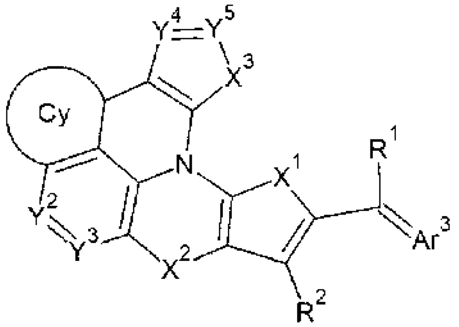

dans laquelle, dans la formule chimique 2E-1,

$X^1$, $X^2$, $Ar^3$, $R^1$, et $R^2$ sont les mêmes que $X^1$, $X^2$, $Ar^3$, $R^1$, et $R^2$, respectivement, dans la formule chimique 1, $X^3$ est le même que $X^3$ dans la formule chimique 2E,

$Y^2$ à $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et

Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci,

[Formule chimique 2E-2]

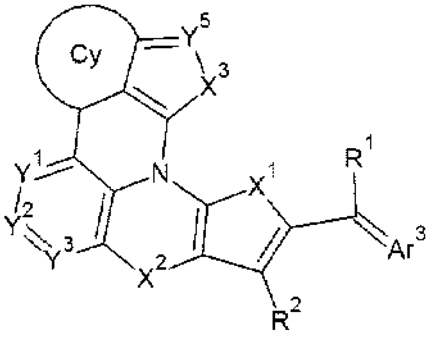

dans laquelle, dans la formule chimique 2E-2,

$X^1$, $X^2$, $Ar^3$, $R^1$, et $R^2$ sont les mêmes que $X^1$, $X^2$, $Ar^3$, $R^1$, et $R^2$, respectivement, dans la formule chimique 1,

$X^3$ est le même que $X^3$ dans la formule chimique 2E,

$Y^1$ à $Y^3$ et $Y^5$ sont chacun indépendamment N ou $CR^k$, dans laquelle $R^k$ est l'hydrogène, le deutérium, un halogène, un groupe cyano, un groupe nitro, un groupe hydroxyle, un groupe amine, un groupe alkyle en C1 à C10 substitué ou non substitué, ou un groupe alcoxy en C1 à C10 substitué ou non substitué ou des $R^k$ adjacents sont liés les uns aux autres pour former un groupe arène en C6 à C30 substitué ou non substitué, un groupe hétéroarène en C3 à C30 substitué ou non substitué, ou un cycle condensé de ceux-ci, et

Cy est un groupe arène en C6 à C30 substitué ou non substitué, un groupe hétéroarène en C3 à C30 substitué ou non substitué, un groupe cycloalcène en C5 à C30 substitué ou non substitué, un groupe hétérocycloalcène en C3 à C30 substitué ou non substitué, ou un cycle condensé de ceux-ci.

**7.** Composition selon l'une quelconque des revendications 1 à 6, dans laquelle dans $X^1$, $X^2$, et $-CR^{11}R^{12}-$ de la formule chimique 1, chaque structure cyclique de la structure cyclique de $X^1$, la structure cyclique de $X^2$ et la structure cyclique de $-CR^{11}R^{12}-$ est une structure spiro ou une structure cyclique fusionnée ;

de préférence dans laquelle la structure spiro comprend une fraction représentée par la formule chimique 3 :

[Formule chimique 3]

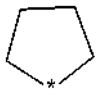
(1)

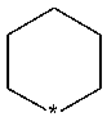
(2)

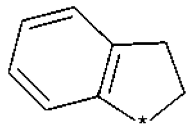
(3)

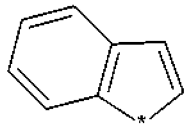
(4)

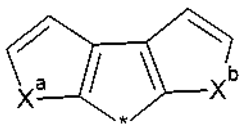
(5)

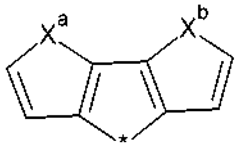
(6)

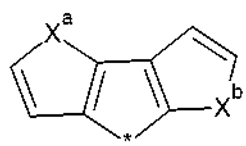
(7)

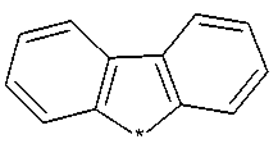
(8)

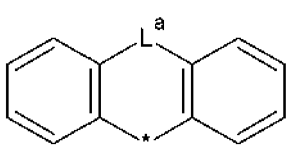
(9)

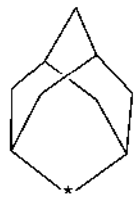
(10)

dans laquelle, dans la formule chimique 3,

$X^a$ et $X^b$ sont chacun indépendamment -O-, -S-, -Se-, -Te-, -S(=O)-, - $S(=O)_2$-, -$NR^{a1}$-, - $BR^{a2}$-, -$SiR^bR^c$-, -$SiR^{bb}R^{cc}$-, -$GeR^dR^e$-, ou -$GeR^{dd}R^{ee}$-, dans laquelle $R^{a1}$, $R^{a2}$, $R^b$, $R^c$, $R^d$, et $R^e$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, un groupe aryloxy en C6 à C20 substitué ou non substitué, ou un groupe hétéroaryle en C3 à C20 substitué ou non substitué, et au moins une paire de $R^{bb}$ et $R^{cc}$ ou $R^{dd}$ et $R^{ee}$ sont liés l'un à l'autre pour former une structure cyclique,

$L^a$ est -O-, -S-, -Se-, -Te-, -$NR^{a1}$-, -$BR^{a2}$-, -$SiR^bR^c$-, -$GeR^dR^e$-, -$(CR^fR^g)_{n1}$-, -$(C(R^p)=N)$-, ou une liaison simple, dans laquelle $R^{a1}$, $R^{a2}$, $R^b$, $R^c$, $R^d$, $R^e$, $R^f$, $R^g$, et $R^p$ sont chacun indépendamment l'hydrogène, le deutérium, un halogène, un groupe cyano, un groupe alkyle en C1 à C20 substitué ou non substitué, un groupe alcoxy en C1 à C20 substitué ou non substitué, un groupe aryle en C6 à C20 substitué ou non substitué, ou un groupe aryloxy en C6 à C20 substitué ou non substitué et n1 de -$(CR^fR^g)_{n1}$- est 1 ou 2 ,

l'hydrogène de chaque cycle est éventuellement remplacé par au moins un substituant choisi parmi le deutérium, un halogène, un groupe alkyle C1 à C20 substitué ou non substitué, un groupe alcoxy C1 à C20 substitué ou non substitué, un groupe aryle en C6 à C20 substitué ou non substitué, et un groupe aryloxy en C6 à C20 substitué ou non substitué, et

un ou plusieurs CH présents dans le cycle aromatique des fractions (3), (4), (5), (6), (7), (8) ou (9) sont éventuellement remplacés par N.

**8.** Composition selon la revendication 7, dans laquelle au moins un hydrogène de chaque cycle est remplacé par au moins un substituant choisi parmi le deutérium, un halogène, un groupe alkyle en C1 à C20 substitué ou non substitué, un groupe alcoxy en C1 à C20 substitué ou non substitué, un groupe aryle en C6 à C20 substitué ou non substitué, et un groupe aryloxy en C6 à C20 substitué ou non substitué.

**9.** Composition selon l'une quelconque des revendications 1 à 8, dans laquelle $Ar^3$ est un groupe cyclique représenté par la formule chimique 4 :

[Formule chimique 4]

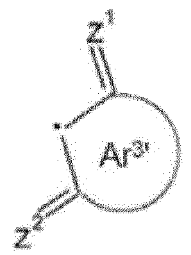

dans laquelle, dans la formule chimique 4,

$Ar^{3'}$ est un groupe aryle en C6 à C30 substitué ou non substitué ou un groupe hétéroaryle en C3 à C30 substitué ou non substitué,

$Z^1$ est O, S, Se ou Te, et

$Z^2$ est O, S, Se, Te, ou $CR^aR^b$, dans laquelle $R^a$ et $R^b$ sont chacun indépendamment l'hydrogène, un groupe alkyle en C1 à C10 substitué ou non substitué, un groupe cyano ou un groupe contenant un cyano, à condition que lorsque $Z^2$ est $CR^aR^b$, au moins l'un de $R^a$ ou $R^b$ soit un groupe cyano ou un groupe contenant un cyano ; ou dans laquelle, dans la formule chimique 1, $Ar^3$ est un groupe cyclique représenté par l'une des formules chimiques 5A à 5G :

[Formule chimique 5A]

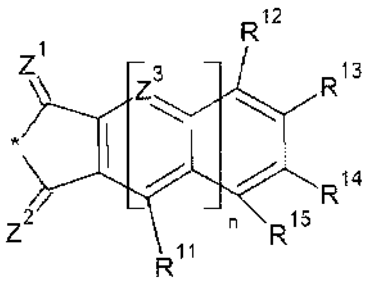

dans laquelle, dans la formule chimique 5A,
$Z^1$ est O, S, Se ou Te,
$Z^2$ est O, S, Se, Te ou $CR^aR^b$, dans laquelle $R^a$ et $R^b$ sont chacun indépendamment l'hydrogène, un groupe alkyle en C1 à C10 substitué ou non substitué, un groupe cyano ou un groupe contenant un cyano, à condition que lorsque $Z^2$ est $CR^aR^b$, au moins l'un de $R^a$ et $R^b$ soit un groupe cyano ou un groupe contenant un cyano,
$Z^3$ est N ou $CR^C$, dans laquelle $R^c$ est l'hydrogène, le deutérium ou un groupe alkyle en C1 à C10 substitué ou non substitué,
$R^{11}$, $R^{12}$, $R^{13}$, $R^{14}$ et $R^{15}$ sont chacun indépendamment l'hydrogène, le deutérium, un groupe alkyle en C1 à C30 substitué ou non substitué, un groupe aryle en C6 à C30 substitué ou non substitué, un groupe hétéroaryle en C4 à C30 substitué ou non substitué, un halogène, un groupe cyano (-CN), un groupe contenant un cyano, ou une combinaison de ceux-ci, dans laquelle $R^{12}$ et $R^{13}$ et $R^{14}$ et $R^{15}$ sont chacun indépendamment présents ou sont liés l'un à l'autre pour former un cycle aromatique fusionné,
n est à 0 ou 1, et
* est un point de liaison,

[Formule chimique 5B]

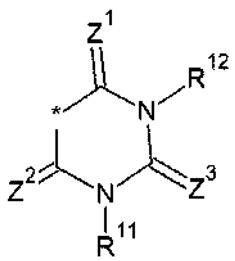

dans laquelle, dans la formule chimique 5B,
$Z^1$ est O, S, Se ou Te,
$Z^2$ est O, S, Se, Te ou $CR^aR^b$, dans laquelle $R^a$ et $R^b$ sont chacun indépendamment l'hydrogène, un groupe alkyle en C1 à C10 substitué ou non substitué, un groupe cyano ou un groupe contenant un cyano, à condition que lorsque $Z^2$ est $CR^aR^b$, au moins l'un de $R^a$ et $R^b$ soit un groupe cyano ou un groupe contenant un cyano,
$Z^3$ est O, S, Se, Te et $C(R^a)(CN)$, dans laquelle $R^a$ est l'hydrogène, un groupe cyano (-CN) ou un groupe alkyle en C1 à C10,
$R^{11}$ et $R^{12}$ sont chacun indépendamment l'hydrogène, le deutérium, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C4 à C30 substitué ou non substitué, un halogène, un groupe cyano (-CN) ou une combinaison de ceux-ci, et
* est un point de liaison,

[Formule chimique 5C]

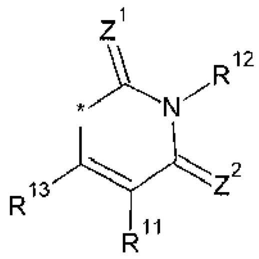

dans laquelle, dans la formule chimique 5C,

$Z^1$ est O, S, Se ou Te,

$Z^2$ est O, S, Se, Te ou $CR^aR^b$, dans laquelle $R^a$ et $R^b$ sont chacun indépendamment l'hydrogène, un groupe alkyle en C1 à C10 substitué ou non substitué, un groupe cyano ou un groupe contenant un cyano, à condition que lorsque $Z^2$ est $CR^aR^b$, au moins l'un de $R^a$ et $R^b$ soit un groupe cyano ou un groupe contenant un cyano,

$R^{11}$, $R^{12}$ et $R^{13}$ sont chacun indépendamment l'hydrogène, le deutérium, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C4 à C30 substitué ou non substitué, un halogène, un groupe cyano (-CN) ou une combinaison de ceux-ci, et

* est un point de liaison,

[Formule chimique 5D]

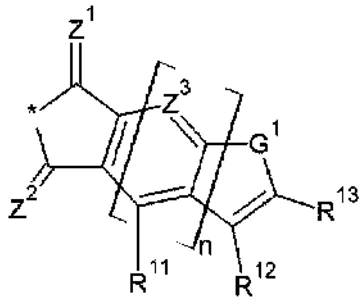

dans laquelle, dans la formule chimique 5D,

$Z^1$ est O, S, Se ou Te,

$Z^2$ est O, S, Se, Te ou $CR^aR^b$, dans laquelle $R^a$ et $R^b$ sont chacun indépendamment l'hydrogène, un groupe alkyle en C1 à C10 substitué ou non substitué, un groupe cyano ou un groupe contenant un cyano, à condition que lorsque $Z^2$ est $CR^aR^b$, au moins l'un de $R^a$ et $R^b$ soit un groupe cyano ou un groupe contenant un cyano,

$Z^3$ est N ou $CR^c$, dans lequel $R^c$ est l'hydrogène ou un groupe alkyle en C1 à C10 substitué ou non substitué,

$G^1$ est O, S, Se, Te, $SiR^xR^y$ ou $GeR^zR^w$, dans laquelle $R^x$, $R^y$, $R^z$ et $R^w$ sont chacun indépendamment l'hydrogène, le deutérium, un halogène, un groupe alkyle en C1 à C20 substitué ou non substitué, ou un groupe aryle en C6 à C20 substitué ou non substitué,

$R^{11}$, $R^{12}$ et $R^{13}$ sont chacun indépendamment l'hydrogène, le deutérium, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C4 à C30 substitué ou non substitué, un halogène, un groupe cyano, un groupe contenant un cyano, ou une quelconque combinaison de ceux-ci, dans lequel $R^{12}$ et $R^{13}$ sont chacun indépendamment présents ou sont liés l'un à l'autre pour former un noyau aromatique fusionné,

n est 0 ou 1, et

* est un point de liaison,

[Formule chimique 5E]

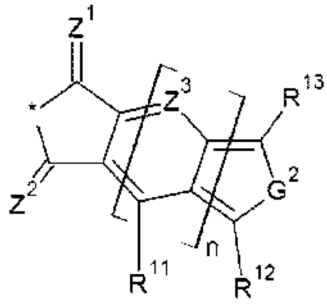

dans laquelle, dans la formule chimique 5E,

$Z^1$ est O, S, Se ou Te,

$Z^2$ est O, S, Se, Te ou $CR^aR^b$, dans laquelle $R^a$ et $R^b$ sont chacun indépendamment l'hydrogène, un groupe alkyle en C1 à C10 substitué ou non substitué, un groupe cyano ou un groupe contenant un cyano, à condition que lorsque $Z^2$ est $CR^aR^b$, au moins l'un de $R^a$ et $R^b$ soit un groupe cyano ou un groupe contenant un cyano,

$Z^3$ est N ou $CR^C$, dans lequel $R^c$ est l'hydrogène ou un groupe alkyle en C1 à C10 substitué ou non substitué,

$G^2$ est O, S, Se, Te, $SiR^xR^y$ ou $GeR^zR^w$, dans laquelle $R^x$, $R^y$, $R^z$ et $R^w$ sont chacun indépendamment l'hydrogène, le deutérium, un halogène, un groupe alkyle en C1 à C20 substitué ou non substitué, ou un groupe aryle en C6 à C20 substitué ou non substitué,

$R^{11}$, $R^{12}$ et $R^{13}$ sont chacun indépendamment l'hydrogène, le deutérium, un groupe alkyle en C1 à C30 substitué ou non substitué, un groupe alcoxy en C1 à C30 substitué ou non substitué, un groupe aryle en C6 à C30 substitué ou non substitué, un groupe hétéroaryle en C4 à C30 substitué ou non substitué, un halogène, un groupe cyano, un groupe contenant un cyano, ou une combinaison de ceux-ci, et

* est un point de liaison,

[Formule chimique 5F]

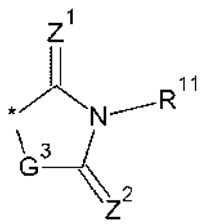

dans laquelle, dans la formule chimique 5F,

$Z^1$ est O, S, Se ou Te,

$Z^2$ est O, S, Se, Te ou $CR^aR^b$, dans laquelle $R^a$ et $R^b$ sont chacun indépendamment l'hydrogène, un groupe alkyle en C1 à C10 substitué ou non substitué, un groupe cyano ou un groupe contenant un cyano, à condition que lorsque $Z^2$ est $CR^aR^b$, au moins l'un de $R^a$ et $R^b$ soit un groupe cyano ou un groupe contenant un cyano,

$R^{11}$ est l'hydrogène, le deutérium, un groupe alkyle en C1 à C30 substitué ou non substitué, un groupe aryle en C6 à C30 substitué ou non substitué, un groupe hétéroaryle en C4 à C30 substitué ou non substitué, un halogène, un groupe cyano (-CN), un groupe contenant un cyano ou une quelconque combinaison de ceux-ci, et

$G^3$ est O, S, Se, Te, $SiR^xR^y$ ou $GeR^zR^w$, dans laquelle $R^x$, $R^y$, $R^z$ et $R^w$ sont chacun indépendamment l'hydrogène, le deutérium, un halogène, un groupe alkyle en C1 à C20 substitué ou non substitué, ou un groupe aryle en C6 à C20 substitué ou non substitué,

[Formule chimique 5G]

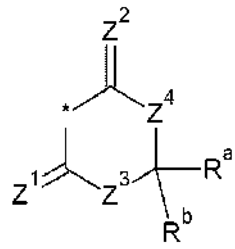

dans laquelle, dans la formule chimique 5G,
$Z^1$ est O, S, Se ou Te,
$R^a$ et $R^b$ sont chacun indépendamment un hydrogène, un groupe alkyle en C1 à C10 substitué ou non substitué, un groupe cyano ou un groupe contenant un cyano, et
$Z^2$ à $Z^4$ sont chacun indépendamment O, S, Se, Te ou $CR^cR^d$, dans laquelle $R^c$ et $R^d$ sont chacun indépendamment l'hydrogène, un groupe alkyle C1 à C10 substitué ou non substitué, un groupe cyano ou un groupe contenant un cyano, à condition que lorsque $Z^2$ est $CR^cR^d$, au moins l'un de $R^c$ ou $R^d$ soit un groupe cyano ou un groupe contenant un cyano.

**10.** Composition selon l'une quelconque des revendications 1 à 9, dans laquelle la composition a une longueur d'onde d'absorption maximale ($\lambda_{max}$) dans une plage de longueurs d'onde supérieure ou égale à 500 nm et inférieure ou égale à 600 nm dans un état de couche mince ; et/ou
dans laquelle la composition est configurée pour présenter une courbe d'absorption de lumière comportant une largeur totale à mi-hauteur (FWHM) dans un état de couche mince de 50 nm à 110 nm.

**11.** Dispositif photoélectrique (100, 200), comprenant :

une première électrode (10) et une seconde électrode (20) se faisant face l'une à l'autre, et
une couche active (30) entre la première électrode et la seconde électrode,
dans lequel la couche active comprend la composition selon l'une quelconque des revendications 1 à 10.

**12.** Capteur d'image (300, 400, 500, 600) comprenant le dispositif photoélectrique selon la revendication 11, de préférence :

dans lequel le dispositif photoélectrique est un dispositif photoélectrique vert configuré pour absorber sélectivement la lumière dans une région de longueur d'onde verte,
le capteur d'image comprend en outre un dispositif photoélectrique bleu (50B) configuré pour détecter sélectivement la lumière dans une région de longueur d'onde bleue, et un dispositif photoélectrique rouge (50R) configuré pour détecter sélectivement la lumière dans une région de longueur d'onde rouge,
dans lequel le dispositif photoélectrique vert, le dispositif photoélectrique bleu et le dispositif photoélectrique rouge sont empilés.

**13.** Capteur d'image selon la revendication 12, comprenant en outre :

un substrat semi-conducteur (310) intégré à une pluralité de premiers dispositifs de photodétection (50B) configurés pour détecter la lumière dans une région de longueur d'onde bleue et une pluralité de seconds dispositifs de photodétection (50R) configurés pour détecter la lumière dans une région de longueur d'onde rouge
dans lequel le dispositif photoélectrique se trouve sur le substrat semi-conducteur et est configuré pour détecter sélectivement la lumière dans une région de longueur d'onde verte.

**14.** Capteur d'image selon la revendication 13, comprenant en outre :

une couche de filtre de couleur (70, 72),
dans lequel la couche de filtre de couleur comprend un filtre bleu (70B, 72B) configuré pour sélectivement transmettre la lumière dans la région de longueur d'onde bleue et un filtre rouge (70R, 72R) configuré pour sélectivement transmettre la lumière dans la région de longueur d'onde rouge ; et/ou
dans lequel la pluralité de dispositifs de photodétection et la pluralité de seconds dispositifs de photodétection

sont empilés dans une direction verticale dans le substrat semi-conducteur.

**15.** Dispositif électronique comprenant le capteur d'image selon l'une quelconque des revendications 12 à 14.

FIG. 1

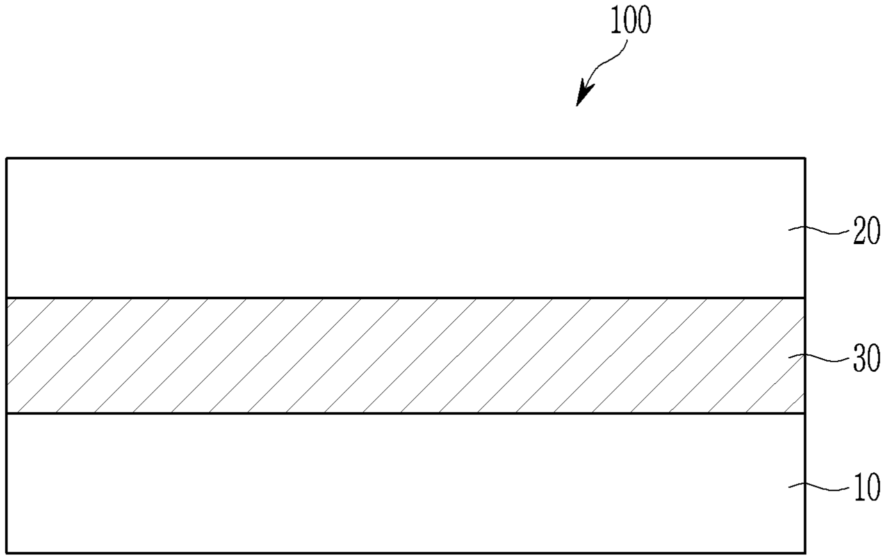

FIG. 2

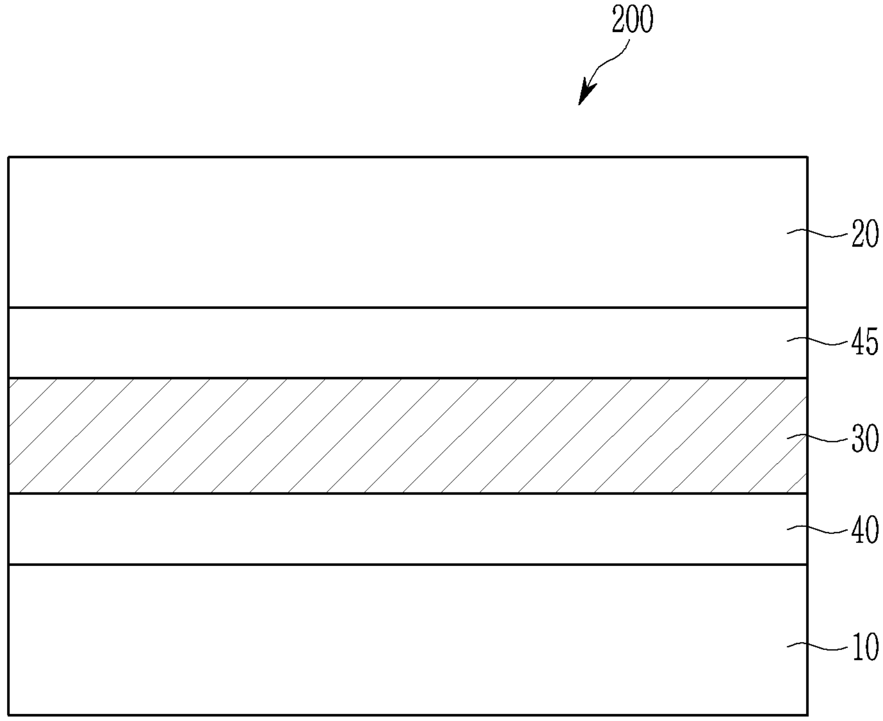

FIG. 3

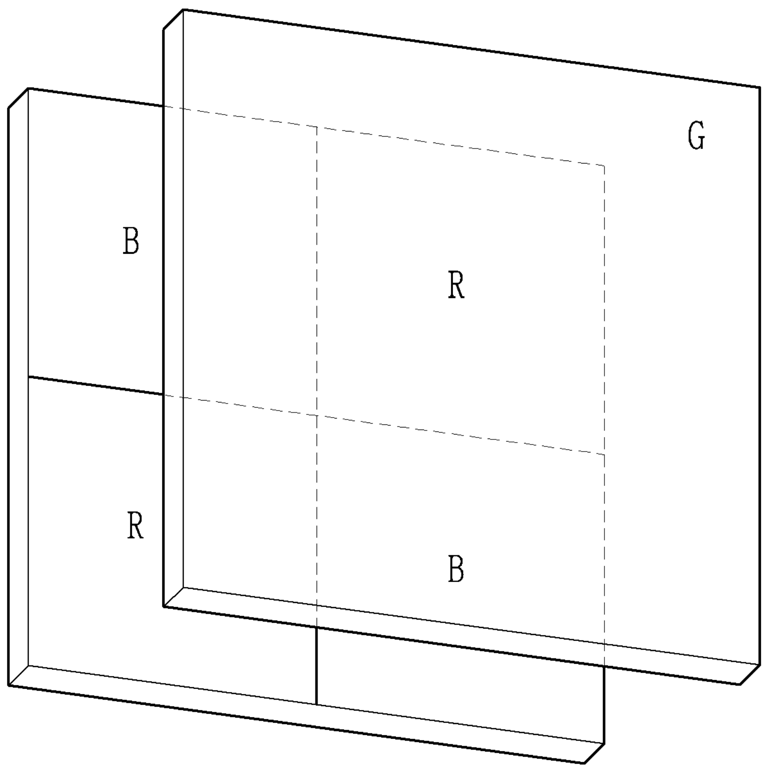

FIG. 4

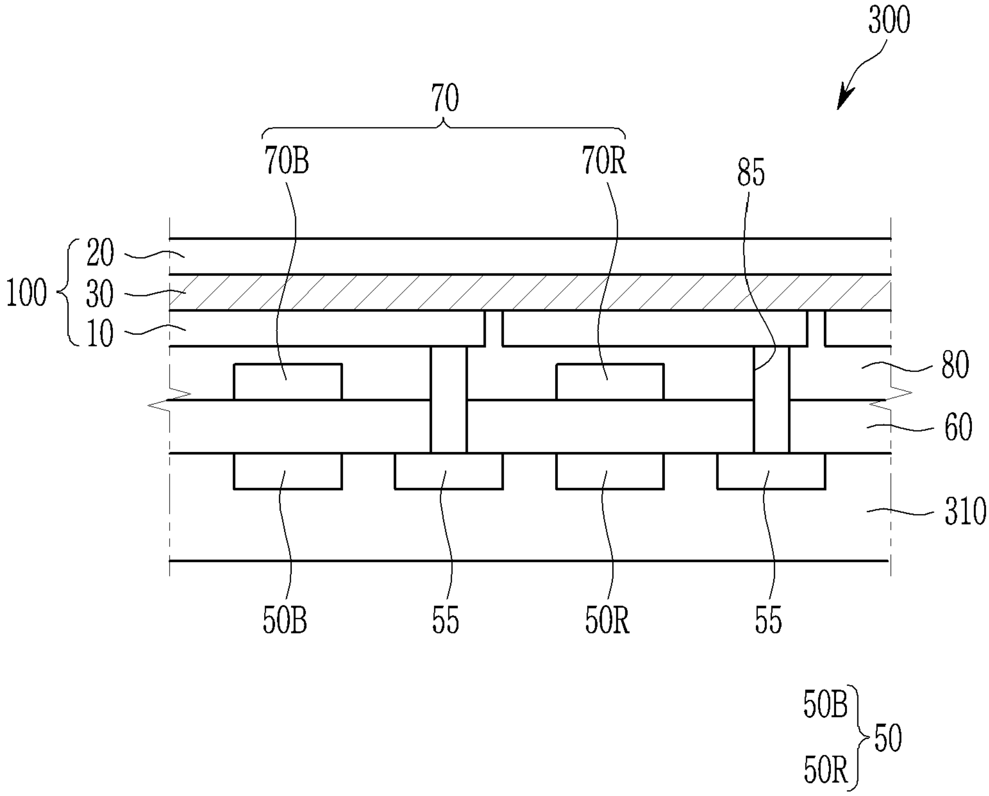

FIG. 5

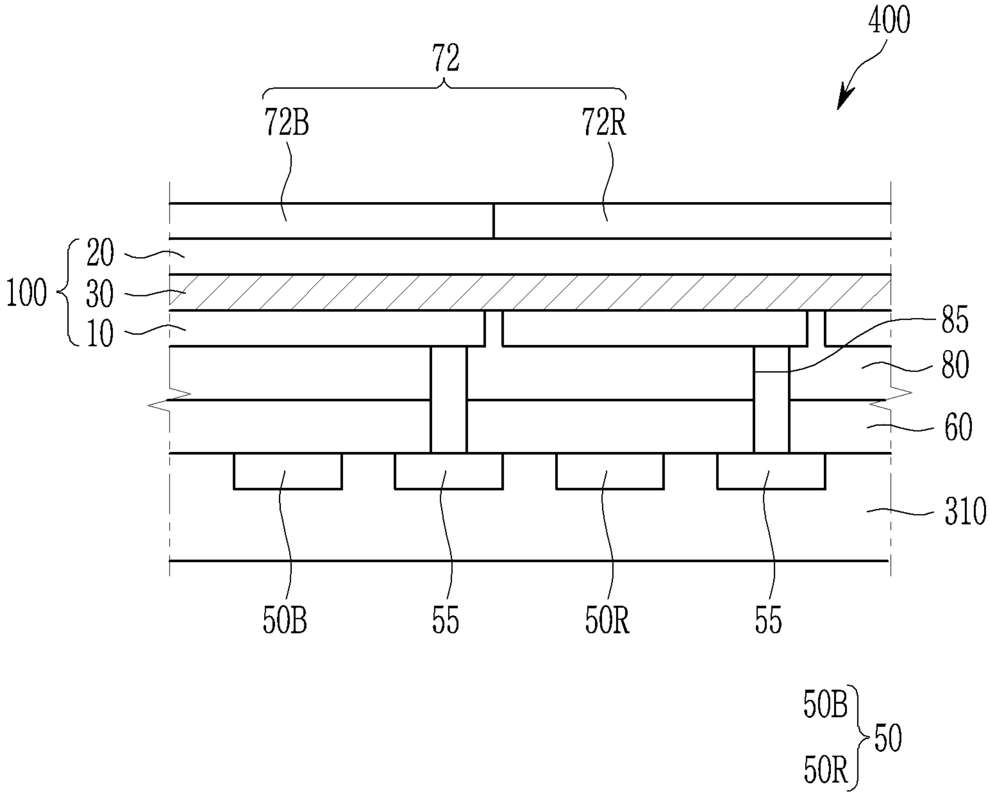

FIG. 6

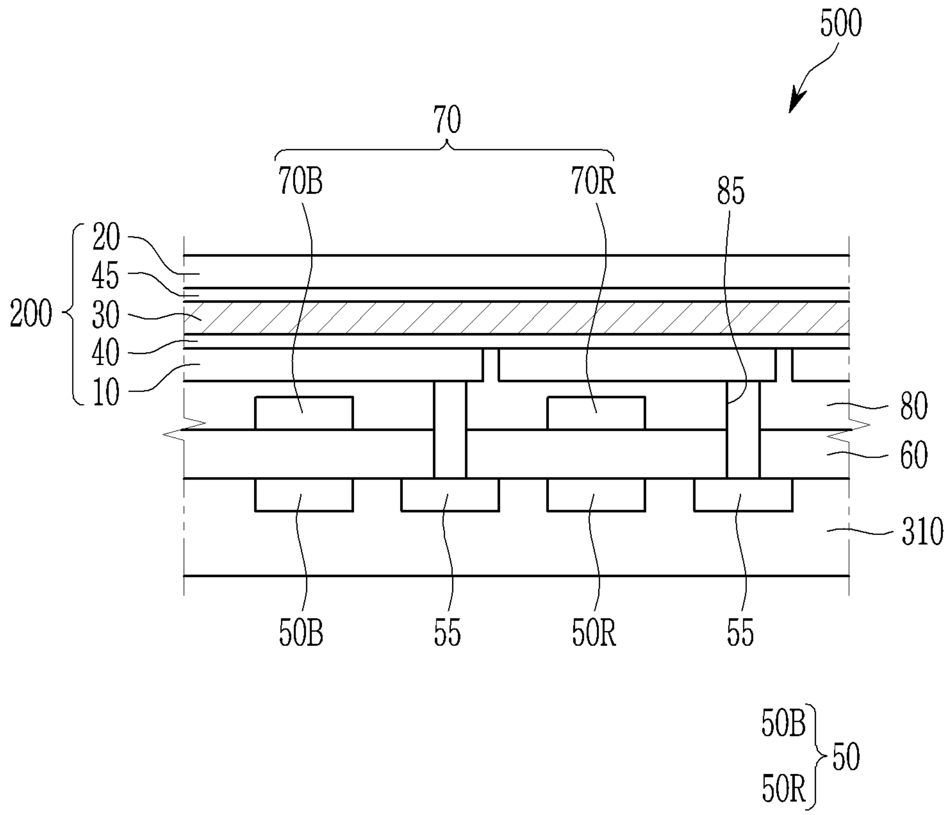
500
70
70B
70R
85
20
45
200
30
40
10
80
60
310
50B
55
50R
55
50B
50R
50

FIG. 7

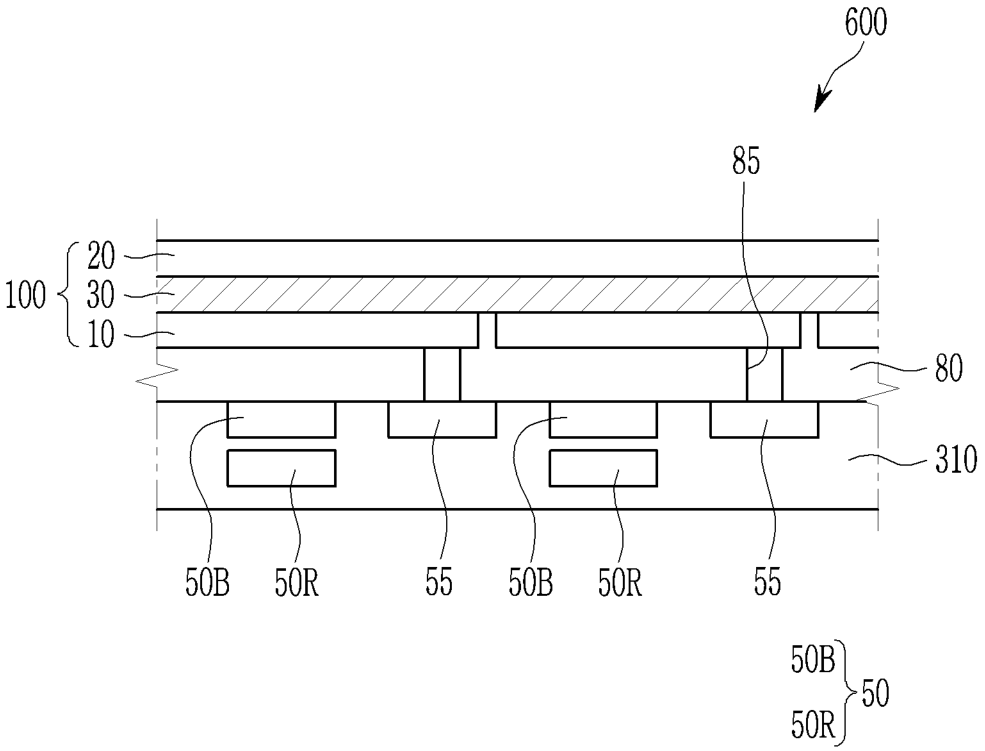

FIG. 8

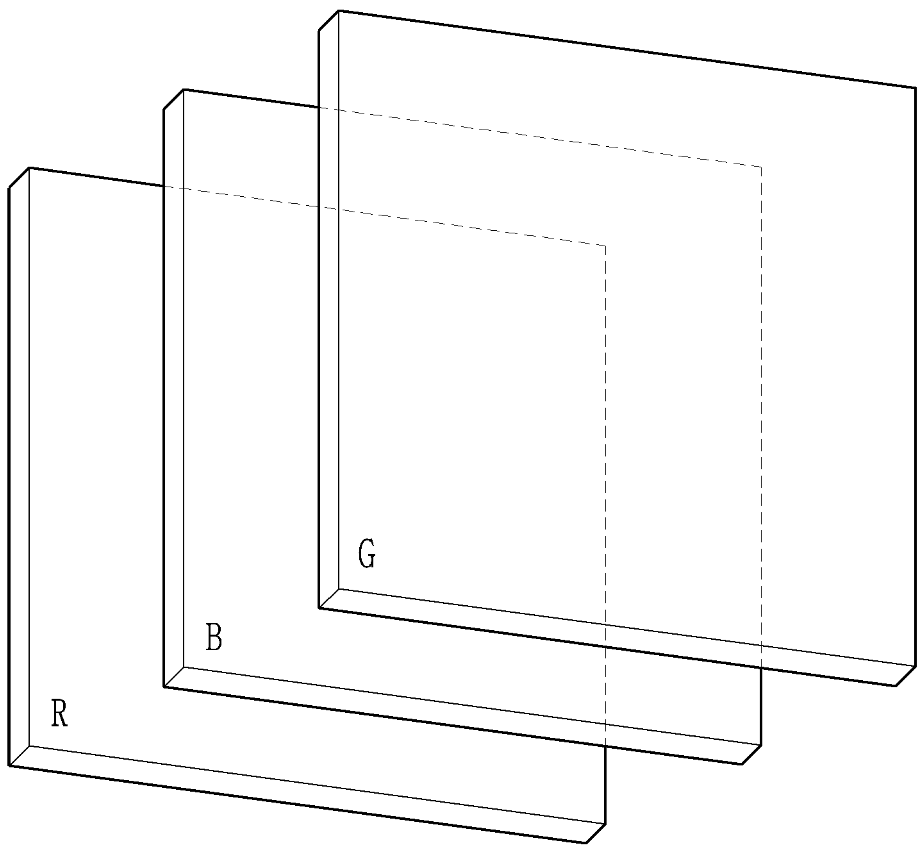

FIG. 9

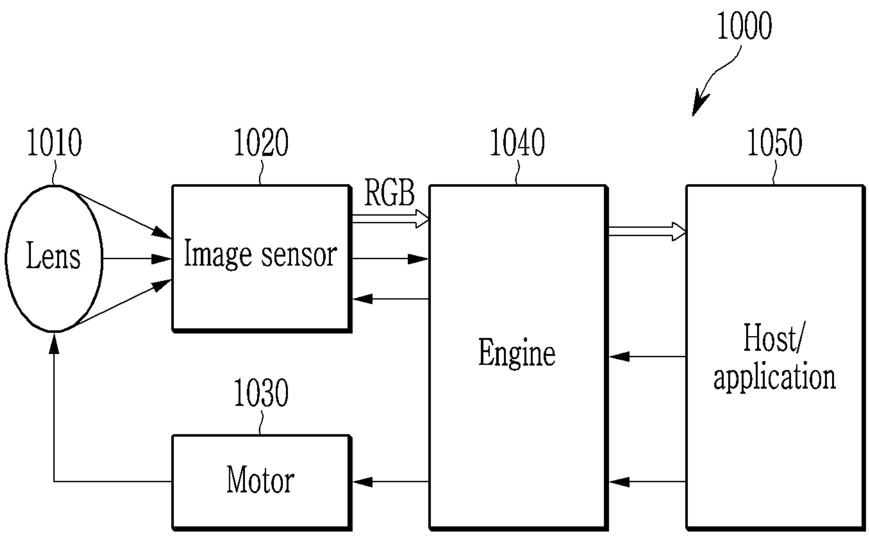
1000
1010
1020
1040
1050
Lens
Image sensor
RGB
Engine
Host/
application
1030
Motor

FIG. 10

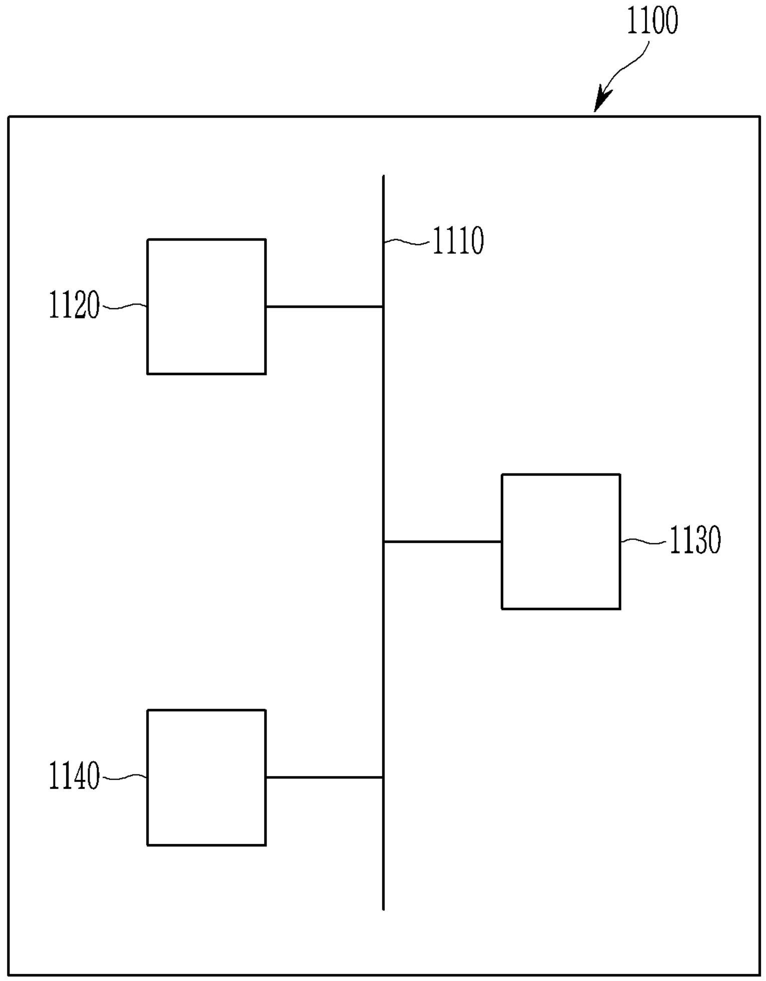

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3252051 A1
- EP 3243822 A1
- WO 2019230562 A1
- US 2016211465 A1


### Non-patent literature cited in the description


- **JUHA ALAKARHU.** Image Sensors and Image Quality in Mobile Phones. *2007 International Image Sensor Workshop (Ogunquit Maine, USA)*
- **TAKAHASHI, K. ; TARUTANI, S.** *Heterocycles,* 1996, vol. 43, 1927-1935
- *Angewante chem.,* 2007, vol. 46, 1627-1629